# EUROPEAN PATENT APPLICATION

(11) **EP 4 825 863 A1**
(43) Date of publication of application: **21.10.2026**
(21) Application number: 25731170.4
(22) Date of filing: 12.02.2025
(51) Int. Cl.: C07F 9/6558, C07D 501/34, A61K 31/661, A61K 31/546, A61P 31/04, C12Q 1/34, C12Q 1/04, G01N 21/64, A61K 41/00, A61K 49/00

(54) **DESIGN, PREPARATION AND APPLICATION OF TARGETED ACTIVATION ENRICHMENT TYPE NEAR-INFRARED PROBE AND PHOTOSENSITIZER**

(30) Priority: 12.12.2023 CN 202311703253; 29.11.2024 CN 202411740178
(71) Applicant: East China University of Science and Technology, Shanghai 200237 (CN)
(72) Inventor: XIE, Hexin, Shanghai 200237 (CN); LI, Yuyao, Shanghai 200237 (CN); CHEN, Fangfang, Shanghai 200237 (CN); PENG, Yan, Shanghai 200237 (CN); ZHU, Yifan, Shanghai 200237 (CN)
(74) Representative: Murgitroyd & Company
(86) International application number: PCT/CN2025/077038
(87) International publication number: WO 2025/124618

(57) **Abstract**

Provided are a design, preparation and application of a targeted activation enrichment type near-infrared probe and a photosensitizer. Specifically, provided are a novel molecular design, preparation and application method, so that, under the action of disease-related enzymes or chemical environments, fluorescence signals or photosensitive properties can be activated, and the probe or photosensitizer can effectively concentrate around disease-related cells and tissues. The compound is represented by formula I, and each variable is defined in the description.

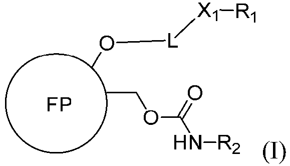

## Description

The present application claims priority to Chinese patent application No. 2023117032530, filed on December 12, 2023, and Chinese patent application No. 202411740178X, filed on November 29, 2024. The contents of the above Chinese patent application are incorporated herein by reference in their entireties.

### TECHNICAL FIELD

The present disclosure belongs to the fields of biomarkers and biomedicine, and specifically relates to the design, preparation, and application of targeted activatable enrichment-type near-infrared probes and photosensitizers.

### BACKGROUND

Abnormal expression of enzymes or disease-related microenvironmental factors (e.g., ROS) is closely associated with the occurrence and progression of many diseases. For example, levels of alkaline phosphatase (ALP) in the human body are closely associated with various diseases such as cancer and liver injury; γ-glutamyl transpeptidase (GGT) exhibits abnormal activity in diseases such as hepatotoxicity, asthma, diabetes, and cancer; N-acetyl-β-D-glucosaminidase (NAG) has been clinically proven to be a biomarker for kidney injury; and abnormal expression of carboxylesterase (CE) under physiological and pathological conditions is closely associated with the occurrence of liver cancer. The disease-related microenvironmental factor H₂O₂ is associated with diseases such as liver injury and kidney injury.

It is closely associated with kidney injury. Living organisms are extremely complex systems that undergo constant dynamic changes. Therefore, real-time *in vivo* monitoring of enzyme activity and ROS production is of great significance for the prevention, early diagnosis, and treatment of diseases.

To enable highly sensitive and real-time detection of enzyme activity and ROS, researchers have developed enzyme- and ROS-activatable fluorescence-enhanced probes based on the ability of enzymes and ROS to specifically catalyze the conversion of their substrates into products. Such probes typically consist of a fluorophore and enzyme- or ROS-responsive recognition moiety. In these probes, the fluorescence signal of the fluorophore remains in an "off" state due to the quenching effect of the recognition moiety. Upon specific hydrolysis of the recognition moiety by the target enzyme or ROS, the fluorescence is turned "on", resulting in enhanced fluorescence intensity and/or changes in fluorescence absorption and emission wavelengths, while simultaneously achieving catalytic amplification of the fluorescence signal. The sensitivity of enzyme- or ROS-activatable fluorescence-enhanced probes is typically evaluated in solution by measuring their response to enzymes, and the detection limit is determined through enzyme kinetics studies. However, unlike conventional detection solutions, which are relatively homogeneous, closed, and static, living organisms represent highly complex, dynamic, open, and heterogeneous environments. Following activation by enzymes or ROS, fluorescent signal molecules are readily affected by the dynamic physiological environment and may rapidly diffuse away from the detection site or be eliminated from the body, leading to a decrease in the signal-to-noise ratio and sensitivity for the detection of enzyme or ROS activity. Conventional fluorescence-enhanced probes lack signal retention capability. During imaging of enzymes or ROS in living organisms, especially for extracellular enzymes such as γ-glutamyl transpeptidase and alkaline phosphatase, the activated fluorescent signal molecules come into direct contact with bloodstream and are more likely to rapidly diffuse away from the enzyme detection site and be eliminated from the body through blood circulation. Consequently, the sensitivity of fluorescent probes for detecting enzymes or ROS in living organisms is reduced, making it difficult to achieve the desired fluorescence imaging performance. Therefore, the application of fluorescent probes in complex, dynamic, and open living organisms remains highly challenging. Accordingly, the development of near-infrared fluorescent probes capable of achieving substantial accumulation at the detection site is of great significance for improving the sensitivity for detecting enzyme activity or ROS *in vivo.*

The unique catalytic activity of enzymes is crucial not only for disease diagnosis but also for disease treatment.

In the field of oncology, photodynamic therapy (PDT) has become an important clinical approach for cancer treatment due to its minimally invasive nature, negligible drug resistance, and favorable therapeutic efficacy. PDT primarily relies on photosensitizers that generate highly cytotoxic reactive oxygen species (ROS) within tumor tissues upon irradiation with light of a specific wavelength, thereby inducing tumor cell death. Therefore, targeted delivery of photosensitizers to tumor tissues *in vivo* is one of the key factors for effective PDT. To achieve the objective, a series of activatable photosensitizers based on tumor-associated enzyme activity have been developed, enabling selective activation within tumor tissues. However, since living organisms are highly dynamic biological systems, activated photosensitizers are prone to rapid clearance from tumor tissues, thereby significantly reducing the therapeutic efficacy of PDT. Consequently, achieving effective accumulation of photosensitizers within tumor tissues is of great significance for enhancing both the efficacy and specificity of PDT.

In the field of antibacterial therapy, photodynamic therapy (PDT) is a treatment modality in which photosensitizers are activated by light of a specific wavelength to generate reactive oxygen species, thereby inducing the death of diseased tissues. Numerous studies have reported the application of photosensitizers in antibacterial therapy. However, similar to conventional probes, these molecules have difficulty retaining at the target site and are prone to diffusion during *in vivo* applications.

In summary, there is an urgent need in the art to develop a class of probes and photosensitizers capable of achieving accumulation at the target site.

### SUMMARY

The objective of the present disclosure is to provide the design, preparation, and application of a class of targeted activatable enrichment-type near-infrared probes and photosensitizers. Specifically, the present disclosure provides a novel molecular design, preparation, and application strategy that enables activation of fluorescence signals or photosensitizing properties under the action of disease-related enzymes or chemical environments, while allowing effective accumulation around disease-related cells and tissues. The compounds of the present disclosure may be conjugated with different specifically activatable groups according to different application requirements, thereby activating the molecule at the target site. For example, for oncology applications, groups that are specifically activated in the tumor environment may be introduced into the molecule; for applications targeting β-lactamase-expressing drug-resistant bacteria, groups that are specifically activated in the presence of β-lactamase may be introduced into the molecule.

In a first aspect of the present disclosure, provided is a compound or a salt thereof, wherein the compound is represented by formula I; 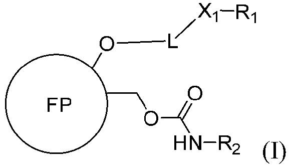 in formula I,
R₁ is a group capable of being specifically hydrolyzed by a biological macromolecular enzyme or a group capable of being specifically activated by a disease-related microenvironmental factor; and upon specific hydrolysis or activation, the bond between R₁ and X₁ is cleaved and R₁ is released;
X₁ is a linking bond, O, or NH;
L is a linking group capable of self-immolation after the release of R₁;
R₂ is C₁₋₆ alkyl or a water-solubilizing group;
the FP moiety is a fluorophore or a photosensitizing group.

In another preferred embodiment, the salt is a pharmaceutically acceptable salt.

In another preferred embodiment, the group capable of being specifically hydrolyzed by a biological macromolecular enzyme means that when R₁ is a group capable of being specifically hydrolyzed by a biological macromolecular enzyme, the biological macromolecular enzyme is capable of recognizing the group, and under the action of the biological macromolecular enzyme, X₁-R₁ is hydrolyzed, thereby cleaving the bond between X₁ and R₁.

In another preferred embodiment, the biological macromolecular enzyme is alkaline phosphatase, γ-glutamyl transpeptidase, aminopeptidase, Fapα, Hepsin, or β-lactamase, preferably alkaline phosphatase or β-lactamase.

In another preferred embodiment, the group capable of being specifically hydrolyzed by a biological macromolecular enzyme is: an enzyme-recognition group capable of being specifically hydrolyzed by alkaline phosphatase, an enzyme-recognition group capable of being specifically hydrolyzed by γ-glutamyl transpeptidase, an enzyme-recognition group capable of being specifically hydrolyzed by aminopeptidase, an enzyme-recognition group capable of being specifically hydrolyzed by Fapα, an enzyme-recognition group capable of being specifically hydrolyzed by Hepsin, or an enzyme-recognition group capable of being specifically hydrolyzed by β-lactamase.

In another preferred embodiment, the enzyme-recognition group capable of being specifically hydrolyzed by alkaline phosphatase is

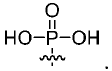

In another preferred embodiment, the enzyme-recognition group capable of being specifically hydrolyzed by γ-glutamyl transpeptidase is

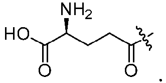

In another preferred embodiment, the enzyme-recognition group capable of being specifically hydrolyzed by aminopeptidase is

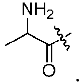

In another preferred embodiment, the enzyme-recognition group capable of being specifically hydrolyzed by Fapα is

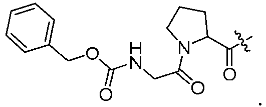

In another preferred embodiment, the enzyme-recognition group capable of being specifically hydrolyzed by Hepsin is

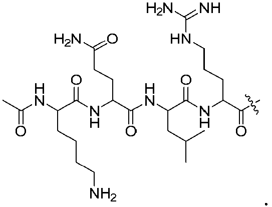

In another preferred embodiment, the enzyme-recognition group capable of being specifically hydrolyzed by β-lactamase is 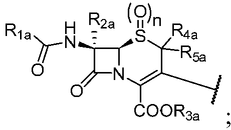 wherein R₁ₐ is selected from the group consisting of C₁₋₆ alkyl, phenyl, 5- or 6-membered heteroaryl, C₁₋₄ alkylene-phenyl, C₁₋₄ alkylene-(5- or 6-membered heteroaryl), and a water-solubilizing group, wherein the C₁₋₆ alkyl, phenyl, 5- or 6-membered heteroaryl, C₁₋₄ alkylene-phenyl, and C₁₋₄ alkylene-(5- or 6-membered heteroaryl) are optionally substituted by one or more halogens, C₁₋₆ alkyl, or C₁₋₆ haloalkyl; preferably, R₁ₐ is a water-solubilizing group;
R₂ₐ is selected from the group consisting of H and -OC₁₋₄ alkyl; preferably, R₂ₐ is selected from the group consisting of H and -OMe; more preferably, R₂ₐ is H;
R₃ₐ is selected from the group consisting of H, C₁₋₆ alkyl, and a monovalent cation *(e.g.,* Na⁺), wherein the C₁₋₆ alkyl is optionally substituted by one or more halogens, C₁₋₆ alkyl, or C₁₋₆ haloalkyl; preferably, R₃ₐ is selected from the group consisting of H, C₁₋₆ alkyl, and a monovalent cation; more preferably, R₃ₐ is H;
R₄ₐ and R₅ₐ are each independently selected from the group consisting of H and C₁₋₄ alkyl *(e.g.,* methyl or ethyl); alternatively, R₄ₐ and R₅ₐ, together with the carbon atom to which they are attached, form a C₃₋₄ cycloalkyl group; preferably, R₄ₐ and R₅ₐ are each independently selected from the group consisting of H and C₁₋₄ alkyl (e.g., methyl or ethyl); more preferably, both R₄ₐ and R₅ₐ are H;
n is 0, 1, or 2; preferably, n is 0.

In another preferred embodiment, the group capable of being specifically activated by a disease-related microenvironmental factor means that when R₁ is a group capable of being specifically activated by a disease-related microenvironmental factor, the bond between X₁ and R₁ can be cleaved under the action of the disease-related microenvironmental factor.

In another preferred embodiment, the disease-related microenvironmental factor is a β-lactamase-expressing drug-resistant bacterial infection or reactive oxygen species (ROS).

In another preferred embodiment, the reactive oxygen species is preferably a peroxide or a superoxide anion.

In another preferred embodiment, the group capable of being specifically activated by a peroxide is

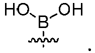

In another preferred embodiment, the group capable of being specifically activated by a superoxide anion is

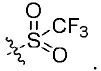

In another preferred embodiment, the group capable of being specifically activated by a disease-related microenvironmental factor is a group capable of being specifically activated by a peroxide or a group capable of being specifically activated by a superoxide anion.

In another preferred embodiment, the water-solubilizing group is -L₁-W₁-L₂-water-solubilizing moiety; wherein L₁ and L₂ are each independently absent or a divalent linking group, and W₁ is absent or a linking group formed via a click chemistry reaction.

In another preferred embodiment, L₁ and L₂ are each independently absent or a stable, non-cleavable divalent linking group under intracellular or *in vivo* conditions, preferably absent or C₁-C₄ alkylene.

In another preferred embodiment, W₁ is absent or

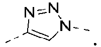

In another preferred embodiment, the water-solubilizing moiety is selected from the group consisting of a group containing a cation-anion pair, a group containing one or more carboxyl groups, and a group containing one or more sulfonic acid groups.

In another preferred embodiment, the group containing one or more carboxyl groups is a group containing 2 or 3 carboxyl groups, such as 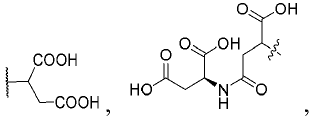 or

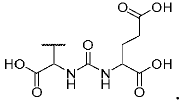

In another preferred embodiment, the group containing one or more sulfonic acid groups is a group containing 2 or 3 sulfonic acid groups, such as

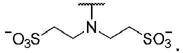

In another preferred embodiment, the group containing a cation-anion pair is 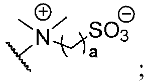 wherein a is 1, 2, 3, or 4, preferably 2 or 3.

In another preferred embodiment, the water-solubilizing group is 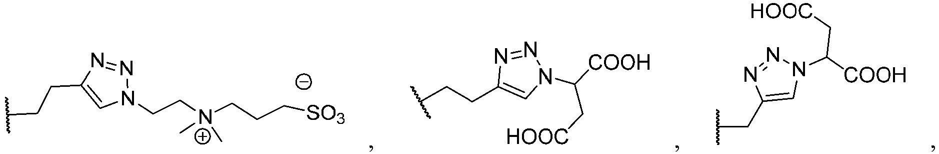 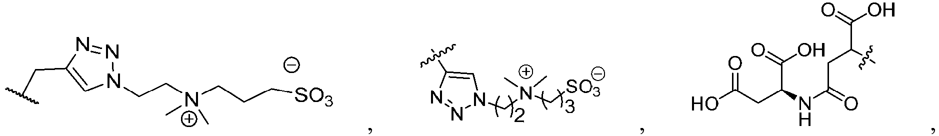 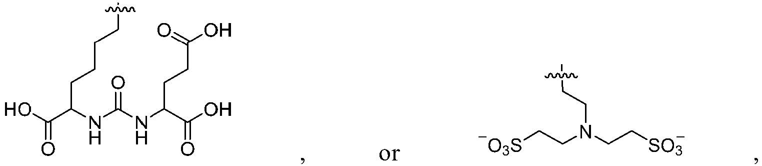 preferably

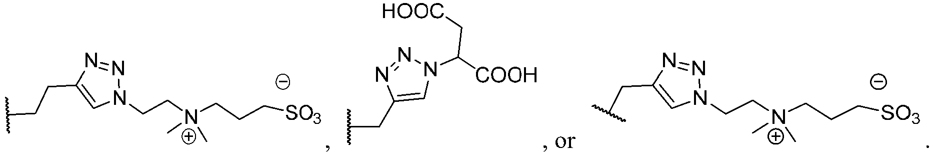

In another preferred embodiment, the water-solubilizing group is a water-solubilizing group capable of being linked to a carbamate structure, such that upon activation of the compound, the carbamate structure is cleaved and the water-solubilizing group is released.

In another preferred embodiment, R₁ is selected from the group consisting of: 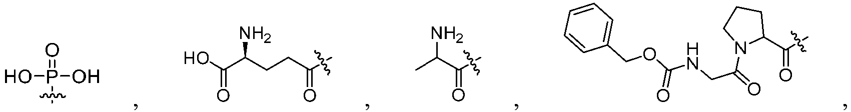 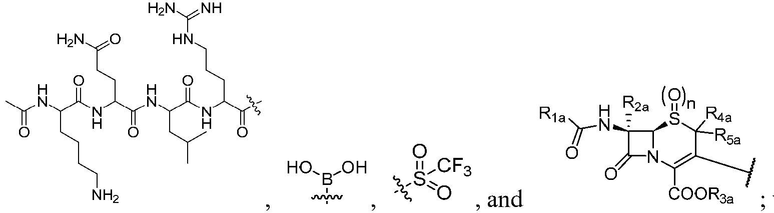 wherein R₁ₐ, R₂ₐ, R₃ₐ, R₄ₐ, R₅ₐ, and n are as defined above; preferably, R₁ is 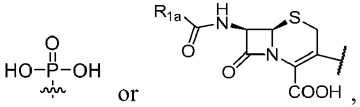 wherein R₁ₐ is a water-solubilizing group; more preferably, R₁ is

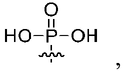

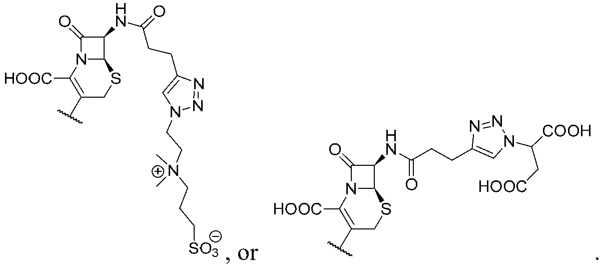

In another preferred embodiment, X₁ is a linking bond or O, preferably O.

In another preferred embodiment, L is selected from the group consisting of 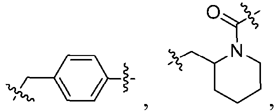 and C₁-C₄ alkylene; preferably, L is selected from the group consisting of 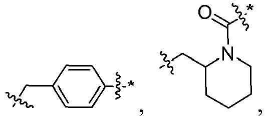 and C₁-C₄ alkylene; wherein * represents the position of attachment to X₁; more preferably, L is

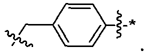

In another preferred embodiment, R₂ is C₁₋₆ alkyl or a water-solubilizing group; preferably, R₂ is ethyl, 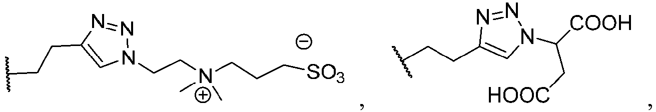 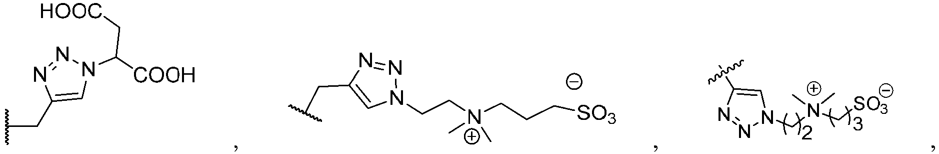 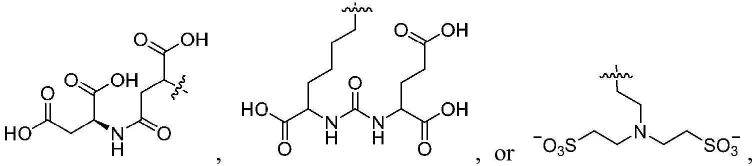 preferably ethyl,

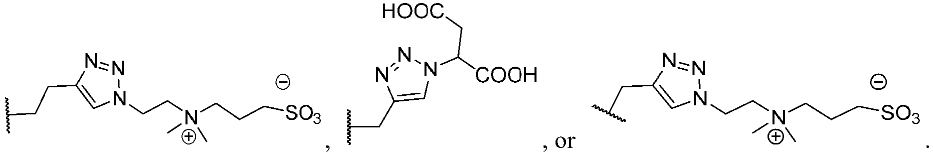

In another preferred embodiment, the FP moiety has a structure represented by formula II-1 or II-2: 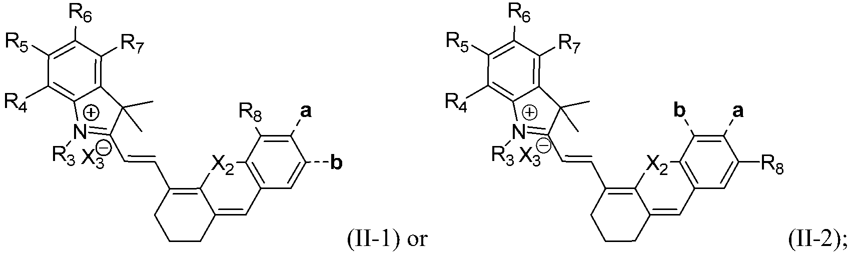 wherein
a represents the position of attachment to
b represents the position of attachment to 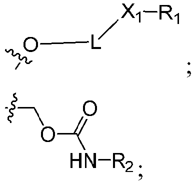
R₃ is -C₁₋₆ alkyl or -C₁₋₆ alkyl-R^{Fu}; wherein R^{Fu} is selected from the group consisting of 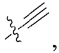 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl;
preferably, the FP moiety is

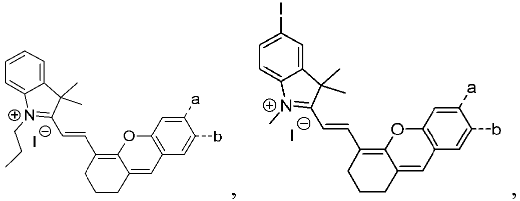

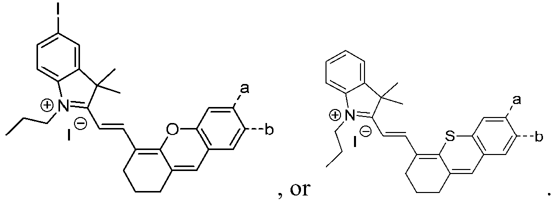

In another preferred embodiment, the compound is represented by formula I; 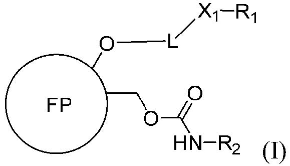 in formula I,
R₁ is a group capable of being specifically hydrolyzed by a biological macromolecular enzyme; and upon specific hydrolysis, the bond between R₁ and X₁ is cleaved and R₁ is released; the biological macromolecular enzyme is alkaline phosphatase or β-lactamase;
X₁ is a linking bond, O, or NH;
L is a linking group capable of self-immolation after the release of Ri;
R₂ is C₁₋₆ alkyl or a water-solubilizing group; the water-solubilizing group is -L₁-W₁-L₂-water-solubilizing moiety;
L₁ and L₂ are each independently C₁-C₄ alkylene;
W₁ is 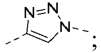
the water-solubilizing moiety is selected from the group consisting of 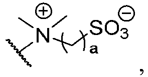 a group containing 2 or 3 carboxyl groups, and a group containing 2 or 3 sulfonic acid groups, wherein a is 1, 2, 3, or 4;
the FP moiety has a structure represented by formula II-1 or II-2: 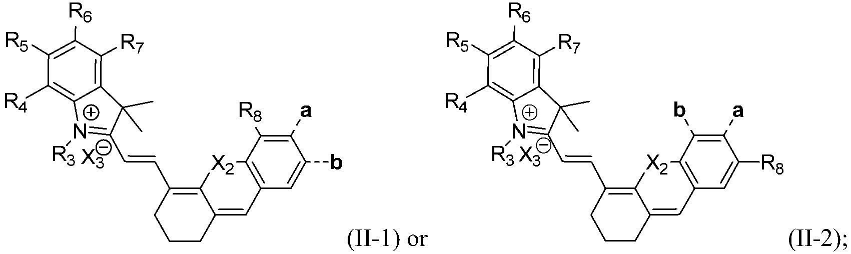
wherein
a represents the position of attachment to 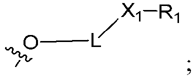
b represents the position of attachment to 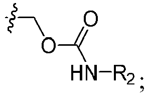
R₃ is -C₁₋₆ alkyl or -C₁₋₆ alkyl-R^{Fu}; wherein R^{Fu} is selected from the group consisting of 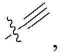 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl.

In another preferred embodiment, the compound is represented by formula I; 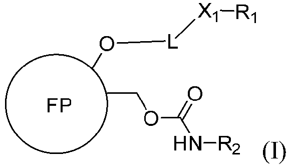 in formula I,
R₁ is 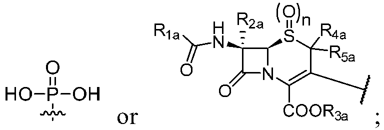 wherein R₁ₐ, R₂ₐ, R₃ₐ, R₄ₐ, R₅ₐ, and n are as defined in claim 3 or 4;
X₁ is a linking bond or O;
L is 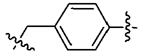 or C₁-C₄ alkylene;
R₂ is C₁₋₆ alkyl or a water-solubilizing group; the water-solubilizing group is -L₁-W₁-L₂-water-solubilizing moiety;
L₁ and L₂ are each independently C₁-C₄ alkylene;
W₁ is 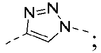
the water-solubilizing moiety is selected from the group consisting of 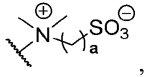 a group containing 2 or 3 carboxyl groups, and a group containing 2 or 3 sulfonic acid groups, wherein a is 1, 2, 3, or 4;
the FP moiety has a structure represented by formula II-1 or II-2: 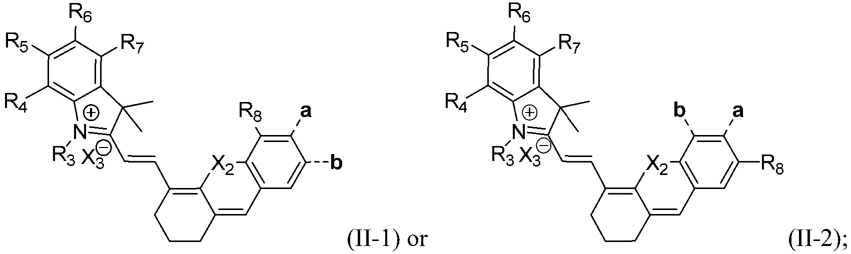
wherein
a represents the position of attachment to 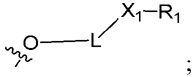
b represents the position of attachment to 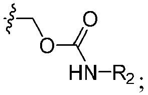
R₃ is -C₁₋₆ alkyl or -C₁₋₆ alkyl-R^{Fu}; wherein R^{Fu} is selected from the group consisting of 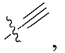 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl.

In another preferred embodiment, the compound is represented by formula I; 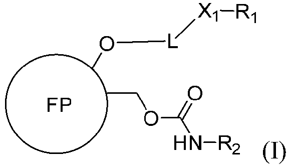 in formula I,
R₁ is a group capable of being specifically hydrolyzed by a biological macromolecular enzyme; and upon specific hydrolysis, the bond between R₁ and X₁ is cleaved and R₁ is released; the biological macromolecular enzyme is alkaline phosphatase;
X₁ is O or NH;
L is a linking group capable of self-immolation after the release of Ri;
R₂ is a water-solubilizing group; the water-solubilizing group is -L₁-W₁-L₂-water-solubilizing moiety;
L₁ and L₂ are each independently C₁-C₄ alkylene;
W₁ is 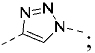
the water-solubilizing moiety is selected from the group consisting of 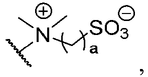 a group containing 2 or 3 carboxyl groups, and a group containing 2 or 3 sulfonic acid groups, wherein a is 1, 2, 3, or 4;
the FP moiety has a structure represented by formula II-1 or II-2: 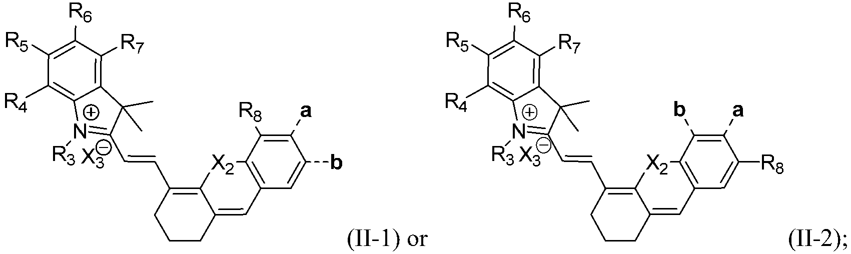
wherein
a represents the position of attachment to 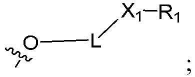
b represents the position of attachment to 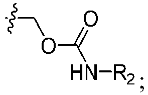
R₃ is -C₁₋₆ alkyl or -C₁₋₆ alkyl-R^{Fu}; wherein R^{Fu} is selected from the group consisting of 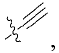 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl.

In another preferred embodiment, the compound is represented by formula I; 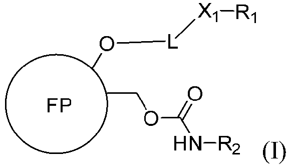 in formula I,
R₁ is 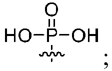
X₁ is O;
L is a linking group capable of self-immolation after the release of R₁;
R₂ is a water-solubilizing group; the water-solubilizing group is -L₁-W₁-L₂-water-solubilizing moiety;
L₁ and L₂ are each independently C₁-C₄ alkylene;
W₁ is 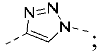
the water-solubilizing moiety is 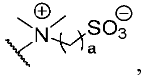 wherein a is 2 or 3;
the FP moiety has a structure represented by formula II-1: 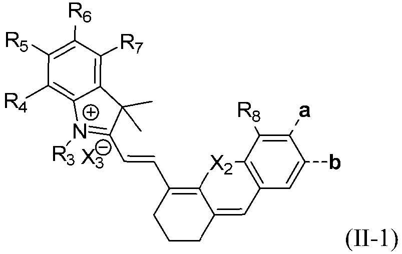
wherein
a represents the position of attachment to 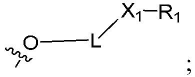
b represents the position of attachment to 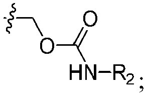
R₃ is -C₁₋₆ alkyl;
R₄, R₅, R₆, and R₇ are each independently H, Br, or I;
R₈ is H, F, Cl, or Br;
X₂ is O or S;
X₃ is I, Br, or Cl.

In another preferred embodiment, the compound is represented by formula I; 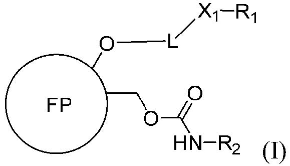 in formula I,
R₁ is 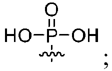
X₁ is O;
L is 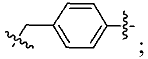
R₂ is a water-solubilizing group; the water-solubilizing group is -L₁-W₁-L₂-water-solubilizing moiety;
L₁ and L₂ are each independently C₁-C₄ alkylene;
W₁ is 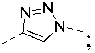
the water-solubilizing moiety is 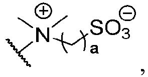 wherein a is 2 or 3;
the FP moiety is

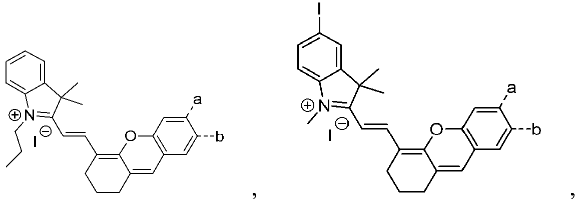

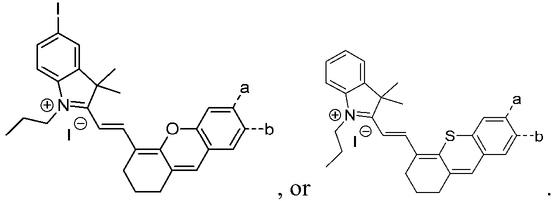

In another preferred embodiment, the compound is represented by formula I-A; 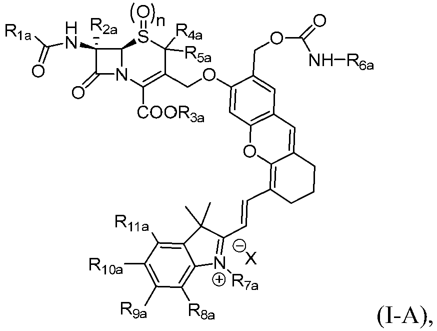 wherein
n is 0, 1, or 2;
R₁ₐ is selected from the group consisting of C₁₋₆ alkyl, phenyl, 5- or 6-membered heteroaryl, C₁₋₄ alkylene-phenyl, C₁₋₄ alkylene-(5- or 6-membered heteroaryl), and a water-solubilizing group;
R₂ₐ is selected from the group consisting of H and -OC₁₋₄ alkyl;
R₃ₐ is selected from the group consisting of H, C₁₋₆ alkyl, and a monovalent cation;
R₄ₐ and R₅ₐ are each independently selected from the group consisting of H and C₁₋₄ alkyl;
R₆ₐ is selected from the group consisting of C₁₋₆ alkyl and a water-solubilizing group;
R₇ₐ is C₁₋₆ alkyl;
R₈ₐ, R₉ₐ, R₁₀ₐ, and R₁₁ₐ are each independently selected from the group consisting of H, Br, and I;
x^{Θ} is a monovalent anion;
the water-solubilizing group is -L₁-W₁-L₂-water-solubilizing moiety;
L₁ and L₂ are each independently C₁-C₄ alkylene;
W₁ is 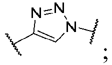
the water-solubilizing moiety is 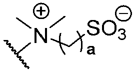 or a group containing 2 or 3 carboxyl groups, wherein a is 2 or 3.

In another preferred embodiment, the compound is represented by formula I-A; 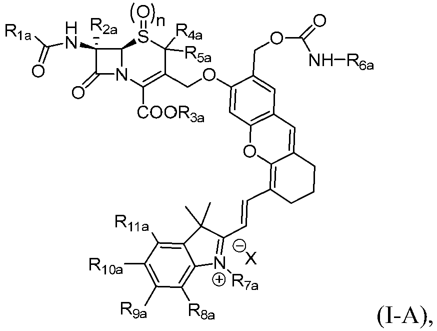 wherein
n is 0;
R₁ₐ is a water-solubilizing group;
R₂ₐ is selected from the group consisting of H and -OC₁₋₄ alkyl;
R₃ₐ is selected from the group consisting of H, C₁₋₆ alkyl, and a monovalent cation;
R₄ₐ and R₅ₐ are each independently selected from the group consisting of H and C₁₋₄ alkyl;
R₆ₐ is selected from the group consisting of C₁₋₆ alkyl and a water-solubilizing group;
R₇ₐ is C₁₋₆ alkyl;
R₈ₐ, R₉ₐ, R₁₀ₐ, and R₁₁ₐ are each independently selected from the group consisting of H, Br, and I;
x^{⊝} is a monovalent anion;
the water-solubilizing group is -L₁-W₁-L₂-water-solubilizing moiety;
L₁ and L₂ are each independently C₁-C₄ alkylene;
W₁ is 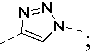
the water-solubilizing moiety is 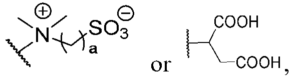 wherein a is 2 or 3.

In another preferred embodiment, the compound is represented by formula I-A; 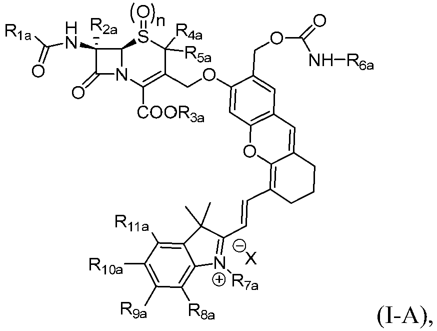 wherein
n is 0;
R₁ₐ is a water-solubilizing group;
R₂ₐ is H;
R₃ₐ is H;
both R₄ₐ and R₅ₐ are H;
R₆ₐ is selected from the group consisting of C₁₋₆ alkyl and a water-solubilizing group;
R₇ₐ is C₁₋₆ alkyl;
R₈ₐ, R₉ₐ, R₁₀ₐ, and R₁₁ₐ are each independently selected from the group consisting of H, Br, and I;
x^{⊝} is a monovalent anion;
the water-solubilizing group is -L₁-W₁-L₂-water-solubilizing moiety;
L₁ and L₂ are each independently C₁-C₄ alkylene;
W₁ is 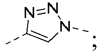
the water-solubilizing moiety is 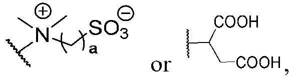 wherein a is 2 or 3.

In a first aspect of the present disclosure, provided is a compound or a salt thereof, wherein the compound is represented by formula I; 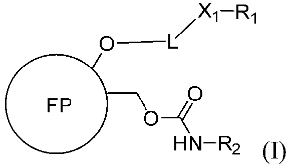 in formula I,
R₁ is a group capable of being specifically hydrolyzed by a biological macromolecular enzyme or a group capable of being specifically activated by a disease-related microenvironmental factor; and upon specific hydrolysis or activation, the bond between R₁ and X₁ is cleaved and R₁ is released;
X₁ is O or NH;
L is a linking group capable of self-immolation after the release of Ri;
R₂ is a water-solubilizing group;
the FP moiety is a fluorophore or a photosensitizing group.

In another preferred embodiment, the compound may be used as a probe or a photosensitizer; preferably, a far-infrared probe or a photosensitizer.

In another preferred embodiment, the FP moiety contains at least one aromatic ring, and 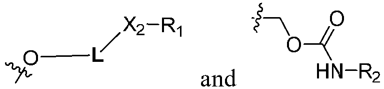 are respectively attached to adjacent ring atoms on the aromatic ring.

In another preferred embodiment, the aromatic ring is a benzene ring or a 5- or 6-membered heteroaromatic ring.

In another preferred embodiment, the aromatic ring is an aromatic ring fused to another ring.

In another preferred embodiment, the aromatic ring is a benzene ring.

In another preferred embodiment, the aromatic ring is a benzene ring in a fused ring system represented by the following formula: 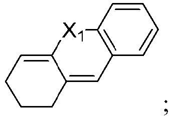 wherein X₁ is O or S.

In another preferred embodiment, the group capable of being specifically hydrolyzed by a biological macromolecular enzyme means that when R₁ is a group capable of being specifically hydrolyzed by a biological macromolecular enzyme, the biological macromolecular enzyme is capable of recognizing the group, and under the action of the biological macromolecular enzyme, X₁-R₁ is hydrolyzed, thereby cleaving the bond between X₁ and R₁.

In another preferred embodiment, the biological macromolecular enzyme comprises alkaline phosphatase, γ-glutamyl transpeptidase, aminopeptidase, Fapα, and Hepsin.

In another preferred embodiment, the enzyme-recognition group capable of being specifically hydrolyzed by alkaline phosphatase comprises:

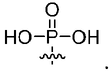

In another preferred embodiment, the enzyme-recognition group capable of being specifically hydrolyzed by γ-glutamyl transpeptidase comprises:

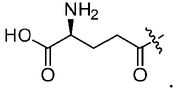

In another preferred embodiment, the enzyme-recognition group capable of being specifically hydrolyzed by aminopeptidase comprises:

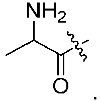

In another preferred embodiment, the enzyme-recognition group capable of being specifically hydrolyzed by Fapα comprises:

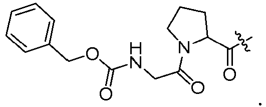

In another preferred embodiment, the enzyme-recognition group capable of being specifically hydrolyzed by Hepsin comprises:

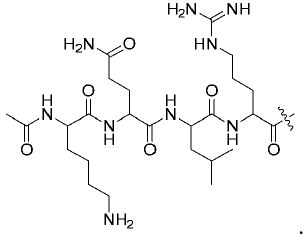

In another preferred embodiment, the enzyme-recognition group capable of being specifically hydrolyzed by a biological macromolecular enzyme is selected from the group consisting of: 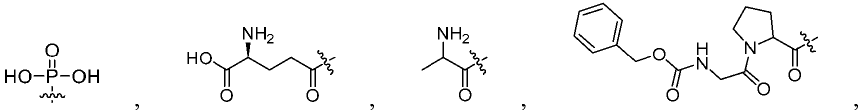 and

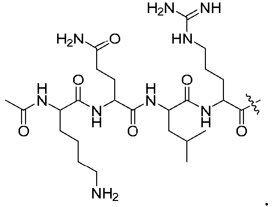

In another preferred embodiment, the group capable of being specifically activated by a disease-related microenvironmental factor means that when R₁ is a group capable of being specifically activated by a disease-related microenvironmental factor, the bond between X₁ and R₁ can be cleaved under the action of the disease-related microenvironmental factor.

In another preferred embodiment, the disease-related microenvironmental factor comprises reactive oxygen species (ROS).

In another preferred embodiment, the reactive oxygen species (ROS) comprises a peroxide (e.g., H₂O₂, ONOO⁻) and a superoxide anion ( ${O}_{2}^{⋅ -}$).

In another preferred embodiment, the group capable of being specifically activated by a peroxide comprises:

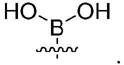

In another preferred embodiment, the group capable of being specifically activated by a superoxide anion comprises:

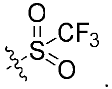

In another preferred embodiment, the group capable of being specifically activated by a disease-related microenvironmental factor is selected from the group consisting of:

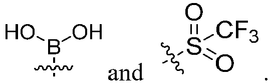

In another preferred embodiment, R₁ is selected from the group consisting of:

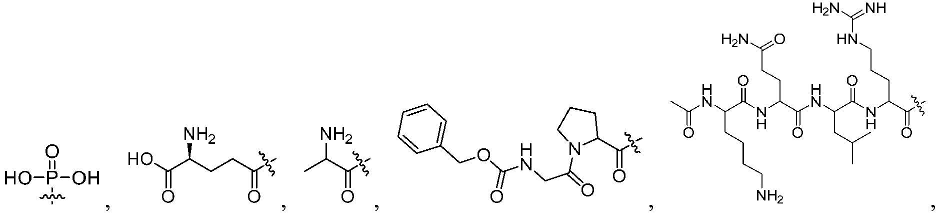

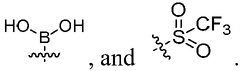

In another preferred embodiment, L is selected from the group consisting of

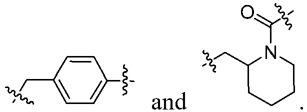

In another preferred embodiment, L is selected from the group consisting of 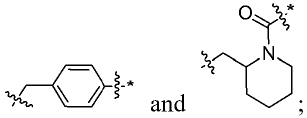 wherein * represents the position of attachment to X₁.

In another preferred embodiment, X₁ is O and L is

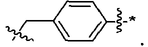

In another preferred embodiment, X₁ is NH and L is

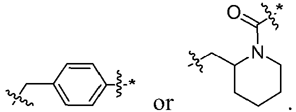

In another preferred embodiment, R₂ is a water-solubilizing group linked to a carbamate structure, such that upon activation of the compound, the carbamate structure is cleaved and the water-solubilizing group is released.

In another preferred embodiment, the water-solubilizing group comprises: a cation-anion pair (*e.g.,* 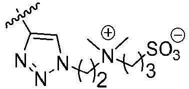 ), a group containing at least three carboxyl functional groups (*e.g*., 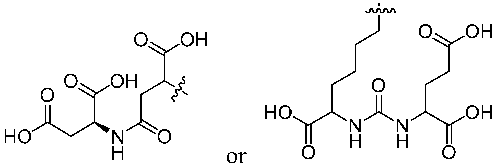 ), and a group containing at least two sulfonic acid functional groups (SO₃⁻) (*e.g.,* 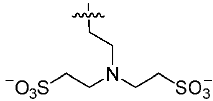 ).

In another preferred embodiment, R₂ is selected from the group consisting of: 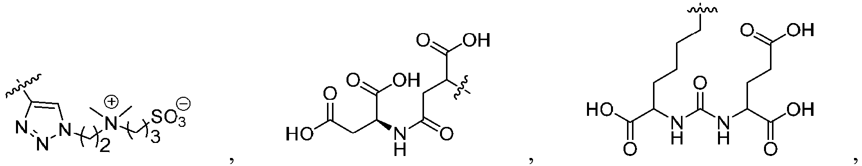 and

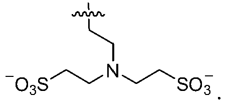

In another preferred embodiment, the FP moiety has a structure represented by formula II-1 or II-2: 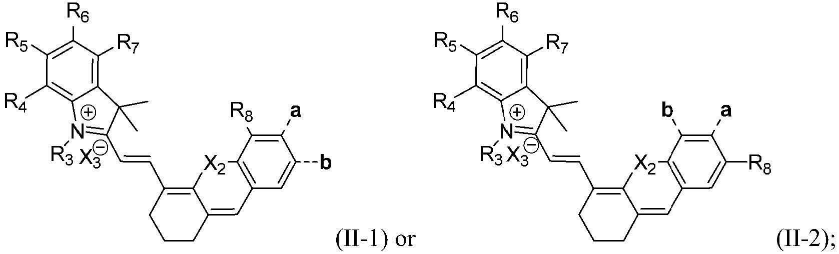 wherein
a represents the position of attachment to 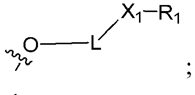
b represents the position of attachment to 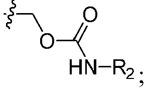
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 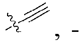 COOH, -N₃, -NH₂, and -N⁺(C₁₋₆ alkyl)₃);
R₄, R₅, R₆, and R₇ are each independently H or a heavy atom I or Br;
R₈ is H, F, Cl, or Br;
X₂ is O or S;
X₃ is I, Br, or Cl.

In another preferred embodiment, the FP moiety has a structure represented by formula III-1 or III-2: 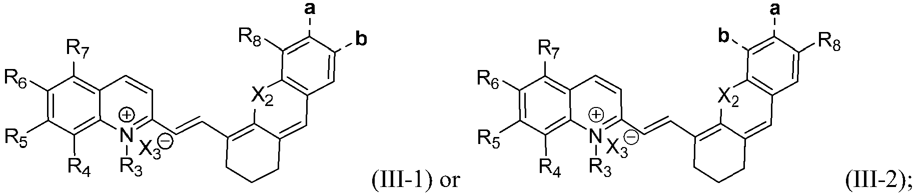 wherein
a represents the position of attachment to 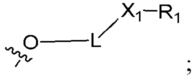
b represents the position of attachment to 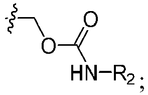
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 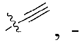 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br;
X₂ is O or S;
X₃ is I, Br, or Cl;

In another preferred embodiment, the FP moiety has a structure represented by formula IV-1 or IV-2: 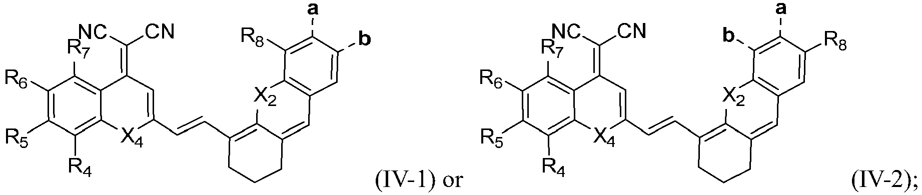 wherein
a represents the position of attachment to 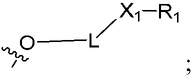
b represents the position of attachment to 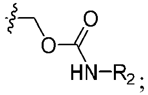
X₂ is O or S;
X₄ is O or NR₃;
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 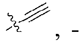 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br.

In another preferred embodiment, the FP moiety has a structure represented by formula V-1 or V-2: 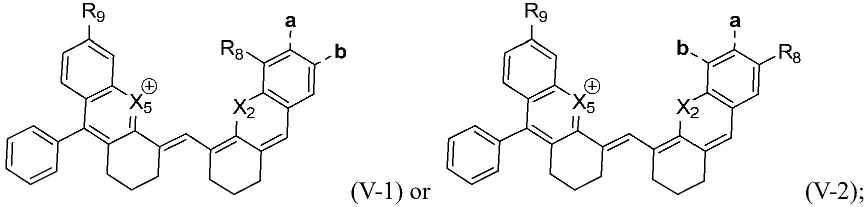 wherein
a represents the position of attachment to 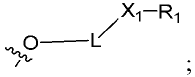
b represents the position of attachment to 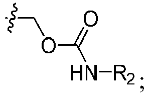
X₂ is O or S;
X₅ is O or S;
R₉ is NEt₂, NPh₂, or NCH₃Ph;
R₈ is H, F, Cl, or Br.

In another preferred embodiment, the FP moiety has a structure represented by formula VI-1 or VI-2: 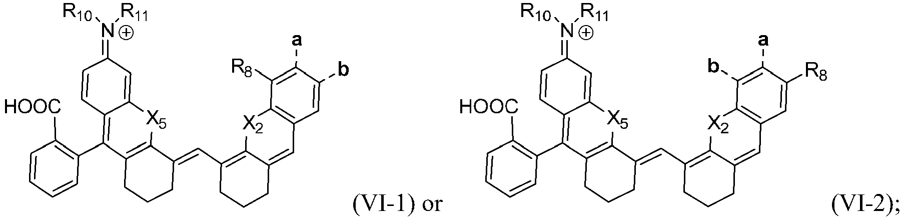 wherein
a represents the position of attachment to 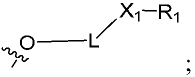
b represents the position of attachment to 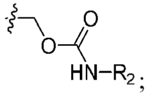
X₂ is O or S;
X₅ is O or S;
R₁₀ and R₁₁ are each independently C₁₋₆ alkyl *(e.g.,* Et) or Ph;
R₈ is H, F, Cl, or Br.

In another preferred embodiment, the water-solubilizing group is a water-solubilizing group capable of being linked to a carbamate structure, such that upon activation of the compound, the carbamate structure is cleaved and the water-solubilizing group is released.

In another preferred embodiment, the exemplary compound is selected from Table 1.

**Table 1**

| | |
|---|---|
| **ALPIN-6** | 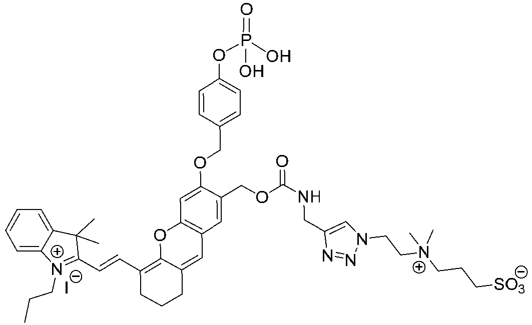 |
| **ALPIN-6I** | 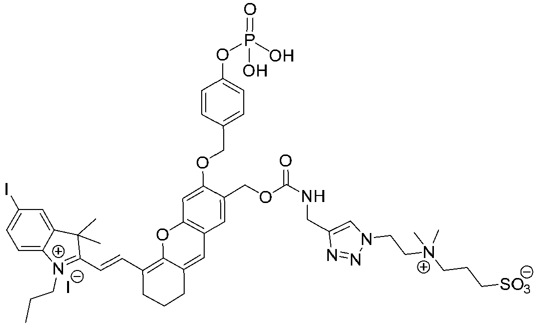 |
| **ALPIN-7** | 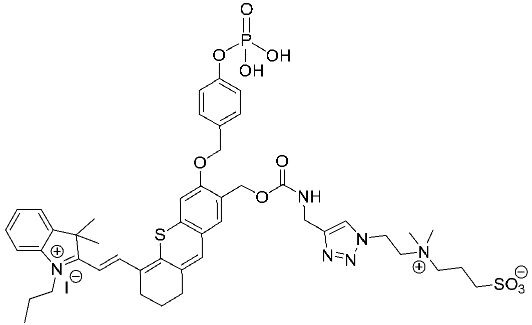 |

In a second aspect of the present disclosure, provided is a probe, wherein the probe is a compound represented by formula I; wherein X₁, X₂, R₁, and L are as defined in the first aspect; and the FP moiety is defined as follows.

In another preferred embodiment, the FP moiety has a structure represented by formula II-1 or II-2; wherein R₄, R₅, R₆, and R₇ are all H; a, b, R₃, R₈, X₂, and X₃ are as defined in the first aspect.

In another preferred embodiment, the FP moiety has a structure represented by formula III-1 or III-2; wherein R₄, R₅, R₆, and R₇ are all H; a, b, R₃, R₈, X₂, and X₃ are as defined in the first aspect.

In another preferred embodiment, the FP moiety has a structure represented by formula IV-1 or IV-2; wherein R₄, R₅, R₆, and R₇ are all H; a, b, R₈, X₂, and X₄ are as defined in the first aspect.

In another preferred embodiment, the FP moiety has a structure represented by formula V-1 or V-2; wherein a, b, R₈, R₉, X₂, and X₅ are as defined in the first aspect.

In another preferred embodiment, the FP moiety has a structure represented by formula VI-1 or VI-2; wherein a, b, R₈, R₁₀, R₁₁, X₂, and X₅ are as defined in the first aspect. In a third aspect of the present disclosure, provided is a photosensitizer, wherein the photosensitizer is a compound represented by formula I; wherein X₁, X₂, R₁, and L are as defined in the first aspect; and the FP moiety is defined as follows.

In another preferred embodiment, the FP moiety has a structure represented by formula II-1 or II-2; wherein R₄, R₅, R₆, and R₇ are each independently H or a heavy atom, and at least one of R₄, R₅, R₆, or R₇ is a heavy atom; a, b, R₃, R₈, X₂, and X₃ are as defined in the first aspect.

In another preferred embodiment, the FP moiety has a structure represented by formula III-1 or III-2; wherein R₄, R₅, R₆, and R₇ are each independently H or a heavy atom, and at least one of R₄, R₅, R₆, or R₇ is a heavy atom; a, b, R₃, R₈, X₂, and X₃ are as defined in the first aspect.

In another preferred embodiment, the FP moiety has a structure represented by formula IV-1 or IV-2; wherein R₄, R₅, R₆, and R₇ are each independently H or a heavy atom, and at least one of R₄, R₅, R₆, or R₇ is a heavy atom; a, b, R₈, X₂, and X₄ are as defined in the first aspect.

In another preferred embodiment, one, two, or three of R₄, R₅, R₆, and R₇ are heavy atoms.

In another preferred embodiment, the heavy atoms are each independently I or Br.

In another preferred embodiment, the photosensitizer is a near-infrared photosensitizer.

In a fourth aspect of the present disclosure, provided is a composition, wherein the composition comprises:
(1) the compound according to the first aspect; and
(2) a pharmaceutically or diagnostically acceptable carrier.

In another preferred embodiment, the composition is a probe composition.

In another preferred embodiment, the composition is a photosensitizer composition.

In a fifth aspect of the present disclosure, provided is a probe composition, wherein the probe composition comprises:
(1) the compound according to the second aspect; and
(2) a pharmaceutically or diagnostically acceptable carrier.

In a sixth aspect of the present disclosure, provided is a photosensitizer composition, wherein the photosensitizer composition comprises:
(1) the compound according to the third aspect; and
(2) a pharmaceutically or diagnostically acceptable carrier.

In a seventh aspect of the present disclosure, provided is a use of the compound according to the first aspect or a pharmaceutically or diagnostically acceptable salt thereof, or the probe according to the second aspect, or the composition according to the fourth aspect, or the probe composition according to the fifth aspect, wherein the use is selected from the group consisting of the following or any combination thereof:
(a) for the detection of an enzyme (preferably, the detection of an enzyme is performed *in vitro* and is non-therapeutic and non-diagnostic);
(b) for the detection of a disease-related microenvironmental factor *(e.g.,* ROS) (preferably, the detection of a disease-related microenvironmental factor *(e.g.,* ROS) is performed *in vitro* and is non-therapeutic and non-diagnostic);
(c) for the manufacture of a detection reagent for the detection of an enzyme or a disease-related microenvironmental factor *(e.g.,* ROS);
(d) for the imaging of cells (preferably, the imaging of cells is performed *in vitro* and is non-therapeutic and non-diagnostic);
(e) for the manufacture of a detection reagent for imaging analysis of cells;
(f) for the imaging of tumor tissues or cells (preferably, the imaging of cells is performed *in vitro* and is non-therapeutic and non-diagnostic);
(g) for the manufacture of a detection reagent for tumor tissues or cells;

In another preferred embodiment, the enzyme comprises alkaline phosphatase, γ-glutamyl transpeptidase, aminopeptidase, Fapα, and Hepsin.

In another preferred embodiment, the detection of an enzyme comprises detection of expression levels of the enzyme and/or detection of activity of the enzyme.

In another preferred embodiment, the detection reagent is capable of imaging tumor tissues or cells in a living subject or detecting the expression level or activity of a cell enzyme.

In an eighth aspect of the present disclosure, provided is a use of the compound according to the first aspect or a pharmaceutically or diagnostically acceptable salt thereof, or the photosensitizer according to the third aspect, or the composition according to the fourth aspect, or the photosensitizer composition according to the sixth aspect, wherein the use is selected from the group consisting of the following or any combination thereof:
(h) for the photodynamic therapy of tumors;
(i) for the manufacture of a photosensitizing therapeutic agent for the photodynamic therapy of tumors.

In a ninth aspect of the present disclosure, provided is a detection kit,
wherein the kit comprises the compound according to the first aspect or a pharmaceutically or diagnostically acceptable salt thereof, or the composition according to the fourth aspect.

In a tenth aspect of the present disclosure, provided is a method of imaging cells or tissues, wherein the method comprises the steps of: incubating cells or tissues to be imaged in the presence of the compound according to the first aspect, and obtaining an image of the cells or tissues using an instrument (e.g., a fluorescence microscope).

In another preferred embodiment, the method is performed *in vitro* and is non-therapeutic.

In an eleventh aspect of the present disclosure, provided is a method of *in vivo* imaging, wherein the method comprises the steps of:
(i) administering to a subject in need thereof the compound according to the first aspect or a pharmaceutically or diagnostically acceptable salt thereof, or the composition according to the fourth aspect; and
(ii) performing imaging using an instrument.

In another preferred embodiment, the subject is a human or a non-human mammal (preferably including non-human primates or rodents such as rats or mice).

In another preferred embodiment, the *in vivo* imaging is real-time imaging or static imaging; preferably, real-time imaging.

It should be understood that, within the scope of the present disclosure, the various technical features described above and the various technical features specifically described below (e.g., in the examples) may be combined with each other to form new or preferred technical solutions. For the sake of brevity, such combinations are not exhaustively described herein.

The inventors have conducted extensive and in-depth research over a prolonged period. To improve the diagnostic and therapeutic performance of probes and photosensitizers in disease applications, the inventors, based on a design strategy involving quinone methide, have for the first time developed a class of near-infrared probes and photosensitizers with functions of activation and enrichment at the detection site. Specifically, upon a single triggering event by a target enzyme or a factor present in a target microenvironment, the initially inactive probes such as near-infrared probes or photosensitizers can undergo activation. The probes or photosensitizers of the present disclosure are capable of: 1) transforming from highly hydrophilic and poorly cell membrane-permeable molecules into highly lipophilic and cell membrane-permeable molecules, thereby reducing their clearance rate and enhancing their retention efficiency at the target site; 2) forming a quinone methide intermediate that reacts with macromolecules such as proteins or intracellular nucleophiles to form a covalent bond, thereby anchoring the fluorophore or photosensitizer; 3) activating near-infrared fluorescence or photoacoustic signals or activating photosensitizing activity under near-infrared light irradiation. The approach greatly enhances the enrichment of signal molecules at the detection site, thereby improving the detection sensitivity of near-infrared fluorescent probes and the photodynamic therapeutic efficacy of photosensitizers. Based on these findings, the inventors have completed the present disclosure.

### Near-infrared probes and photosensitizers and applications thereof

The present disclosure provides a variety of near-infrared probes and photosensitizers (also referred to herein as the compounds of the present disclosure) with functions of activation and enrichment at the detection site, as well as preparation methods therefor and applications thereof in *in vivo* imaging and therapy. The near-infrared probes or photosensitizers provided by the present disclosure possess good water solubility. Their fluorescence signals or photosensitizing properties remain in an "off" state at non-target sites. However, upon a single triggering event by a target enzyme or a disease-related microenvironmental factor (*e.g.,* ROS), these probes or photosensitizers will be capable of: 1) transforming from highly hydrophilic and poorly cell membrane-permeable molecules into highly lipophilic and cell membrane-permeable molecules, thereby reducing their clearance rate and enhancing their retention efficiency at the target site; 2) forming a quinone methide intermediate that reacts with macromolecules such as proteins or intracellular nucleophiles to form a covalent bond, thereby anchoring the fluorophore or photosensitizer; 3) activating near-infrared fluorescence or photoacoustic signals or activating photosensitizing activity under near-infrared light irradiation. These near-infrared probes or photosensitizers consist of a recognition group for an enzyme or disease-related microenvironmental factor *(e.g.,* ROS), a near-infrared fluorescent or photoacoustic or a photosensitizing group, a leaving group, and a hydrophilic group attached to the leaving group. Since the near-infrared fluorescent probes or photosensitizers provided by the present disclosure possess good hydrophilicity, they can be freely delivered to tumor tissues *in vivo,* and under the selective action of a target enzyme or disease-related microenvironmental factor *(e.g.,* ROS), the recognition group can be released, which in turn promotes the release of the leaving group bearing a highly hydrophilic functional group, thereby generating a highly lipophilic and highly reactive quinone methide (QM) intermediate. The process not only reduces the clearance rate of the near-infrared fluorescent probes or photosensitizers of the present disclosure from tumor tissues, but also effectively improves cell membrane permeability to facilitate cellular uptake. Furthermore, nucleophilic residues on proteins within tumor cells can react with the quinone methide intermediate to form a covalent bond, thereby achieving effective accumulation within tumors while simultaneously activating near-infrared fluorescence or photoacoustic signals or photosensitizing activity.

In a specific embodiment, the present disclosure provides a class of targeted activatable enrichment-type near-infrared probes and photosensitizers (*i.e.,* the compounds of the present disclosure or salts thereof, also referred to as compounds having a cleavable hydrophilic group), the structure of which is represented by general formula (I): 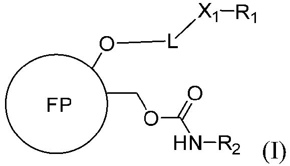 in formula I,
R₁ is a group capable of being specifically hydrolyzed by a biological macromolecular enzyme or a group capable of being specifically activated by a disease-related microenvironmental factor; and upon specific hydrolysis or activation, the bond between R₁ and X₂ is cleaved and R₁ is released;
X₁ is O or NH;
L is a linking group capable of self-immolation after the release of Ri;
R₂ is a water-solubilizing group linked to a carbamate structure, such that upon activation of the compound, the carbamate structure is cleaved and the water-solubilizing group is released;
the FP moiety is a fluorophore or a photosensitizing group.

In another preferred embodiment, after the compound is specifically hydrolyzed or specifically activated, the bond between X₁ and R is cleaved. L undergoes self-immolation as a result of the electron transfer caused by cleavage of the bond between X₁ and R₁ and the release of R₁. Subsequently, 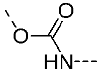 is cleaved as a result of the electron transfer caused by the release of L, thereby causing the release of R₂.

In another embodiment, R₁ is selected from the group consisting of: 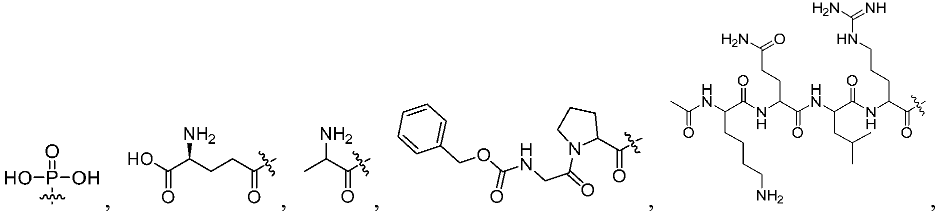 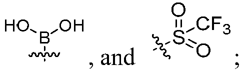 and/or
L is selected from the group consisting of 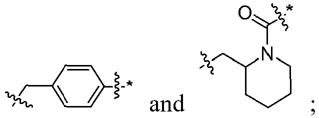 wherein * represents the position of attachment to X₂; and/or
R₂ is selected from the group consisting of: 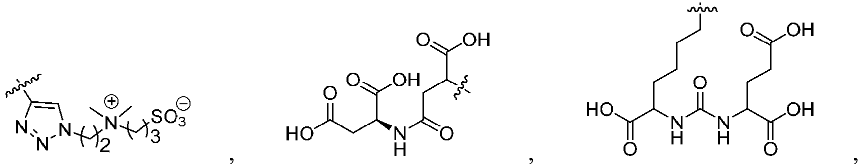 and

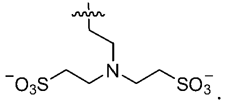

In another embodiment, the FP moiety has a structure represented by formula II: 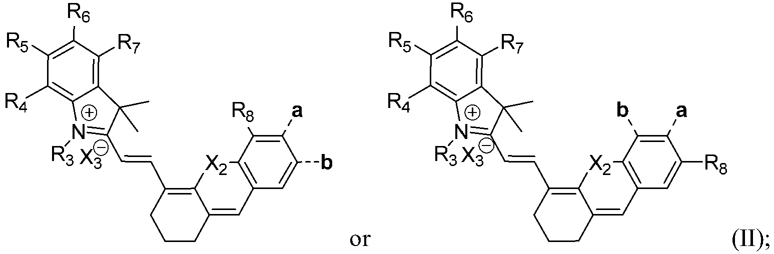 wherein
a represents the position of attachment to 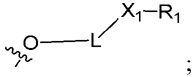
b represents the position of attachment to 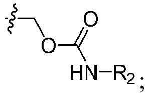
R₃ is -C₁₋₆ alkyl or -C₁₋₆ alkyl-R^{Fu}; wherein R^{Fu} is a modifiable functional group or a positively charged group; R^{Fu} is selected from the group consisting of 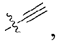 -COOH, -N₃, - NH₂, and -N⁺(C₁₋₆ alkyl)₃. R₄, R₅, R₆, and R₇ are each independently H or a heavy atom I or Br. R₈ is H, F, Cl, or Br.
X₂ is O or S.
X₃ is I, Br, or Cl.

In another embodiment, the FP moiety has a structure represented by the following formula: 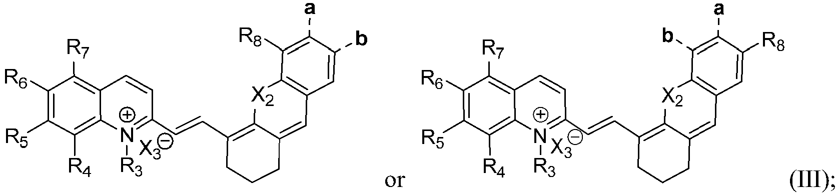 wherein
a represents the position of attachment to 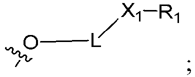
b represents the position of attachment to 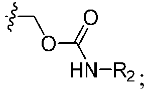
R₃ is -C₁₋₆ alkyl or -C₁₋₆ alkyl-R^{Fu}; wherein R^{Fu} is a modifiable functional group or a positively charged group; R^{Fu} is selected from the group consisting of 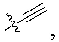 -COOH, -N₃, - NH₂, and -N⁺(C₁₋₆ alkyl)₃. R₄, R₅, R₆, and R₇ are each independently H or a heavy atom I or Br. R₈ is H, F, Cl, or Br.
X₂ is O or S.
X₃ is I, Br, or Cl.

In another embodiment, the FP moiety has a structure represented by the following formula: 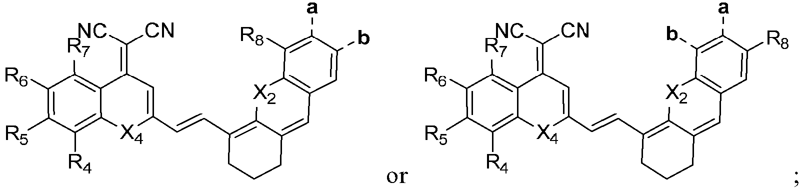 wherein
a represents the position of attachment to 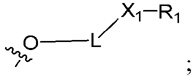
b represents the position of attachment to 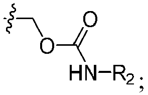
X₂ is O or S.
X₄ is O or NR₃;
R₃ is -C₁₋₆ alkyl or -C₁₋₆ alkyl-R^{Fu}; wherein R^{Fu} is a modifiable functional group or a positively charged group; preferably, R^{Fu} is selected from the group consisting of 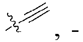 COOH, -N₃, -NH₂, and -N⁺(C₁₋₆ alkyl)₃.
R₄, R₅, R₆, and R₇ are each independently H or a heavy atom such as I or Br.
R₈ is H, F, Cl, or Br.

In another embodiment, the FP moiety has a structure represented by the following formula: 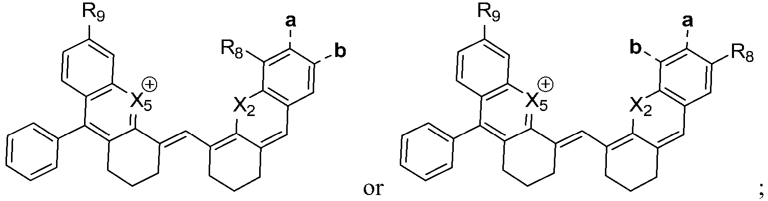 wherein
a represents the position of attachment to 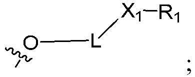
b represents the position of attachment to 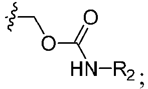
X₂ is O or S.
X₅ is O or S.
R₉ is NEt₂, NPh₂, or NCH₃Ph.
R₈ is H, F, Cl, or Br.

In another embodiment, the FP moiety has a structure represented by the following formula: 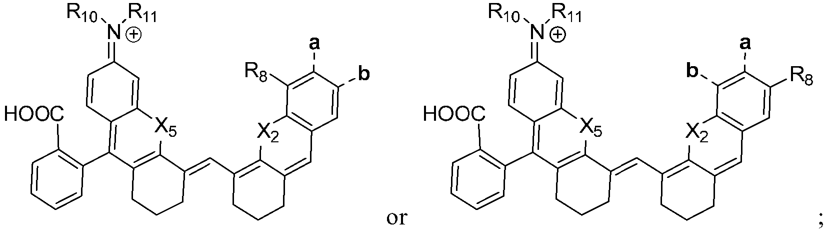 wherein
a represents the position of attachment to 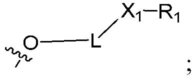
b represents the position of attachment to 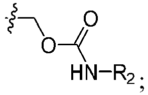
X₂ is O or S.
X₅ is O or S.
R₁₀ and R₁₁ are each independently Et or Ph.
R₈ is H, F, Cl, or Br.

In another embodiment, the FP moiety, X₁, X₂, R₁, and L are as defined in the first aspect.

In another embodiment, the FP moiety, X₁, X₂, R₁, and L are as defined in the first aspect, and are each independently the corresponding groups of the specific compounds listed in Table A.

In another embodiment, the FP moiety, R₁, R₂, R₃, R₄, R₅, R₆, R₇, R₈, R₉, R₁₀, R₁₁, X₁, X₂, X₃, X₄, X₅, and L are each independently the corresponding groups of the specific compounds listed in Table A.

In another embodiment, the near-infrared probe or photosensitizer is selected from Table A.

In another embodiment, provided is a near-infrared fluorescent probe represented by the following structure, namely compound ALPIN-6.

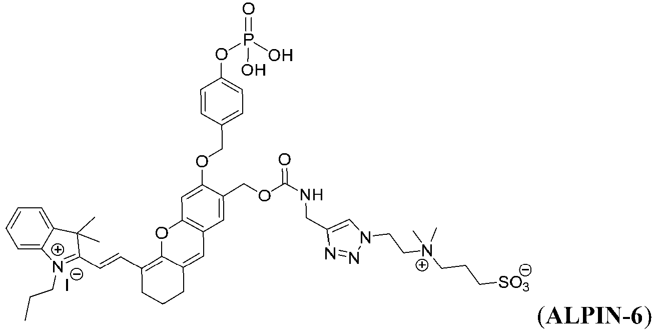

In another embodiment, provided is a near-infrared photosensitizer represented by the following structure, namely compound ALPIN-61.

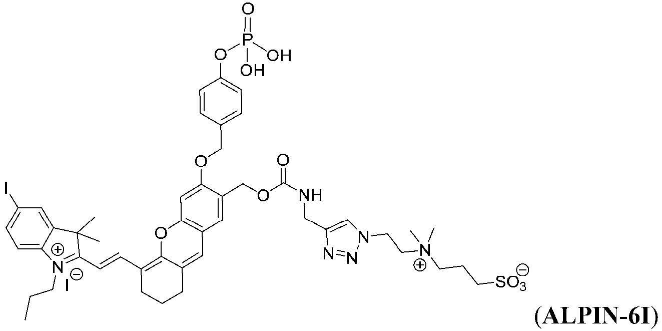

In another embodiment, provided is a near-infrared fluorescent and photoacoustic probe represented by the following structure, namely compound ALPIN-7.

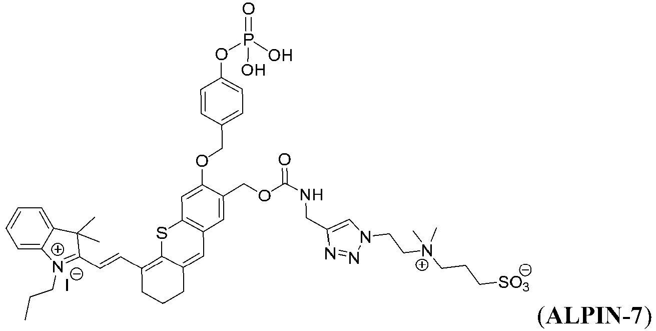

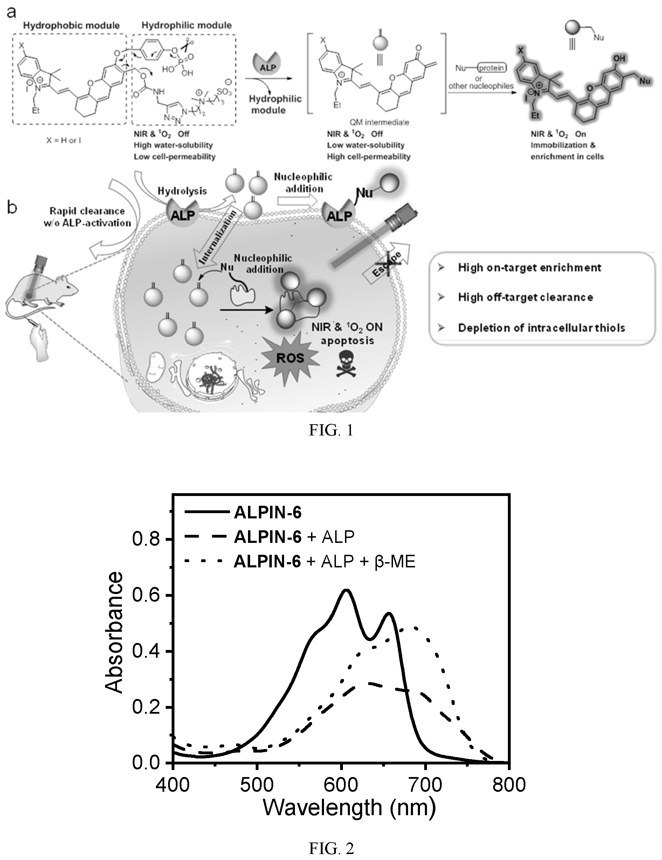

In another embodiment, the present disclosure provides near-infrared probes and photosensitizers with functions of activation and enrichment at the detection site. After the probes or photosensitizers target a desired target, the enzyme or disease-related microenvironmental factor hydrolyzes the recognition group *(i.e.,* the bond between X₂ and R₁ is cleaved, while the linking group L simultaneously undergoes self-immolation), thereby causing the release of both the leaving group (carbamate structure) and the water-solubilizing group (R₂) from the initially inactive near-infrared probes or photosensitizers. The process triggers the following responses: 1) transformation from highly hydrophilic and poorly cell membrane-permeable molecules into highly lipophilic and cell membrane-permeable molecules, thereby reducing their clearance rate and enhancing their retention efficiency at the target site; 2) formation of a quinone methide intermediate that reacts with macromolecules such as proteins or intracellular nucleophiles to form a covalent bond, thereby anchoring the fluorophore or photosensitizer; 3) activation of near-infrared fluorescence or photoacoustic signals or activation of photosensitizing activity under near-infrared light irradiation. The mechanism of action is shown in FIG. 1, in which an alkaline phosphatase detection probe is used as an example.

In another embodiment, the present disclosure provides near-infrared probes (e.g., compounds ALPIN-6 and ALPIN-7) for real-time imaging of alkaline phosphatase (ALP) activity both *in vitro* and *in vivo.*

In another embodiment, the present disclosure further provides a near-infrared photosensitizer (e.g., ALPIN-6I) for use as an ALP-activated photosensitizer in photodynamic therapy of tumors.

Experimental results have demonstrated that the near-infrared fluorescent probes or photosensitizers provided by the present disclosure (particularly the preferred embodiments thereof) possess functions of activation and enrichment at the detection site. The near-infrared fluorescent probes are capable of effectively achieving visualization of relevant enzymes both *in vitro* and *in vivo,* greatly enhancing the accumulation efficiency of probes, and improving the sensitivity and specificity of imaging. Furthermore, the photosensitizers can enable selective killing of tumor cells overexpressing the target enzyme under co-incubation conditions, while leaving other cells unaffected. More importantly, treatment of tumor-bearing mice via intravenous injection of the photosensitizer can completely eliminate tumors overexpressing the target enzyme in all tested mice, with no tumor recurrence during their recovery and no detectable toxicity to other tissues observed.

In another aspect of the present disclosure, provided is a compound or a salt thereof, wherein the compound is represented by formula I-A; 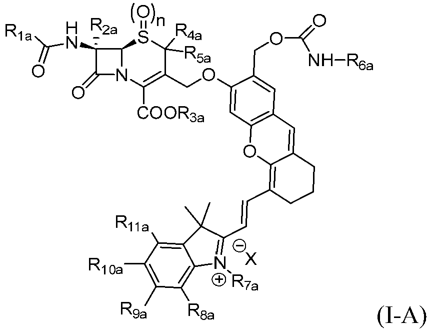 wherein
n is 0, 1, or 2;
R₁ₐ is selected from the group consisting of substituted or unsubstituted C₁₋₆ alkyl, substituted or unsubstituted phenyl, substituted or unsubstituted 5- or 6-membered heteroaryl, substituted or unsubstituted C₁₋₄ alkylene-phenyl, substituted or unsubstituted C₁₋₄ alkylene-5- or 6-membered heteroaryl, and a group containing a water-solubilizing moiety;
R₂ₐ is selected from the group consisting of H and -OC₁₋₄ alkyl;
R₃ₐ is selected from the group consisting of H, substituted or unsubstituted C₁₋₆ alkyl, and a monovalent cation (e.g., Na⁺);
R₄ₐ and R₅ₐ are each independently selected from the group consisting of H and C₁₋₄ alkyl *(e.g.,* methyl or ethyl); alternatively, R₄ₐ and R₅ₐ, together with the carbon atom to which they are attached, form a C₃₋₄ cycloalkyl group;
R₆ₐ is selected from the group consisting of substituted or unsubstituted C₁₋₆ alkyl and a group containing a water-solubilizing moiety;
R₇ₐ is substituted or unsubstituted C₁₋₆ alkyl;
R₈ₐ, R₉ₐ, R₁₀ₐ, and R₁₁ₐ are each independently selected from the group consisting of H and a heavy atom;
x^{⊝} is a monovalent anion;

The term "substituted" means that one or more hydrogen atoms on a group are replaced by a substituent selected from the group consisting of halogen, C₁-C₆ alkyl, and C₁-C₆ haloalkyl.

In another preferred embodiment, the salt refers to a diagnostically or pharmaceutically acceptable salt.

In another preferred embodiment, the group containing a water-solubilizing moiety is -L₁-W₁-L₂-water-solubilizing moiety; wherein L₁ and L₂ are each independently absent or a divalent linking group, and W₁ is absent or a linking group formed via a click chemistry reaction.

In another preferred embodiment, L₁ and L₂ are each independently absent or a stable, non-cleavable divalent linking group under intracellular or *in vivo* conditions.

In another preferred embodiment, L₁ and L₂ are each independently absent or C₁-C₄ alkylene.

In another preferred embodiment, the linking group formed via a click chemistry reaction is

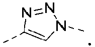

In another preferred embodiment, W₁ is absent or

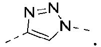

In another preferred embodiment, the water-solubilizing moiety is selected from the group consisting of a group containing a cation-anion pair, a group containing one or more carboxyl groups, and a group containing one or more sulfonic acid groups.

In another preferred embodiment, the group containing one or more carboxyl groups is a group containing 2 or 3 carboxyl groups.

In another preferred embodiment, the group containing one or more sulfonic acid groups is a group containing 2 or 3 sulfonic acid groups.

In another preferred embodiment, the moiety containing a cation-anion pair comprises 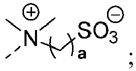 wherein a is 1, 2, 3, or 4.

In another preferred embodiment, the group containing one or more carboxyl groups comprises

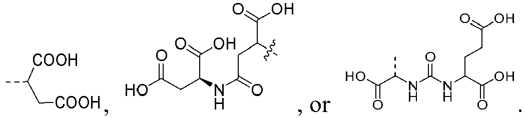

In another preferred embodiment, the group containing one or more sulfonic acid groups comprises

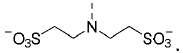

In another preferred embodiment, a is 2 or 3.

In another preferred embodiment, the group containing a water-solubilizing moiety is selected from the group consisting of:

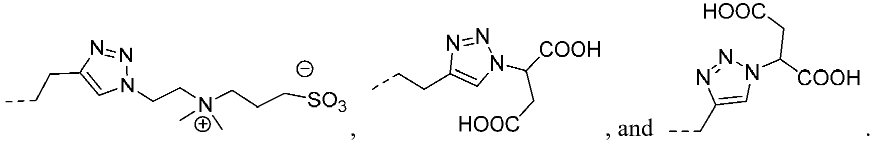

In another preferred embodiment, at least one of R₁ₐ or R₆ₐ is a group containing a water-solubilizing moiety.

In another preferred embodiment, R₁ₐ is a group containing a water-solubilizing moiety.

In another preferred embodiment, R₆ₐ is a group containing a water-solubilizing moiety.

In another preferred embodiment, both R₁ₐ and R₆ₐ are groups containing a water-solubilizing moiety.

In another preferred embodiment, the heavy atoms are each independently I or Br.

In another preferred embodiment, n is 0.

In another preferred embodiment, R₂ₐ is selected from the group consisting of H and -OMe.

In another preferred embodiment, R₂ₐ is H.

In another preferred embodiment, R₃ₐ is selected from the group consisting of H, C₁₋₆ alkyl, and a monovalent cation (e.g., Na⁺).

In another preferred embodiment, R₃ₐ is H.

In another preferred embodiment, R₄ₐ and R₅ₐ are each independently selected from the group consisting of H and C₁₋₄ alkyl.

In another preferred embodiment, R₄ₐ and R₅ₐ are each independently selected from the group consisting of H, methyl, and ethyl.

In another preferred embodiment, R₇ₐ is C₁₋₆ alkyl.

In another preferred embodiment, R₇ₐ is ethyl.

In another preferred embodiment, x^{Θ} is selected from the group consisting of I⁻, Br⁻, and Cl⁻.

In another preferred embodiment, x^{Θ} is I⁻.

In another preferred embodiment, the compound or a salt thereof is used as a probe (a near-infrared probe or a near-infrared fluorescent probe).

In another preferred embodiment, when the compound or a salt thereof is used as a probe, R₈ₐ, R₉ₐ, R₁₀ₐ, and R₁₁ₐ are all H.

In another preferred embodiment, the compound or a salt thereof is used as a photosensitizer.

In another preferred embodiment, when the compound or a salt thereof is used as a photosensitizer, at least one of R₈, R₉, R₁₀, or R₁₁ is a heavy atom, and the remaining groups are H.

In another preferred embodiment, one, two, or three of R₅ₐ, R₉ₐ, R₁₀ₐ, and R₁₁ₐ are heavy atoms.

In another preferred embodiment, the heavy atoms are each independently I or Br.

In another preferred embodiment, n, R₁ₐ, R₂ₐ, R₃ₐ, R₄ₐ, R₅ₐ, R₆ₐ, R₇ₐ, R₈ₐ, R₉ₐ, R₁₀ₐ, and R₁₁ₐ are each independently the corresponding groups of the specific compounds described in the examples of the present disclosure.

In another preferred embodiment, the compound is selected from the group consisting of:

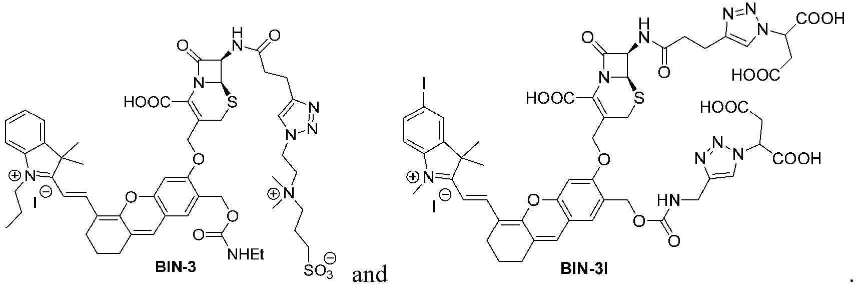

In another preferred embodiment, when the compound or a salt thereof is used as a probe, the compound is **BIN-3.**

In another preferred embodiment, when the compound or a salt thereof is used as a photosensitizer, the compound is **BIN-31.**

In a second aspect of the present disclosure, provided is a probe, wherein the probe is a compound represented by formula I or a salt thereof; wherein n, R₁ₐ, R₂ₐ, R₃ₐ, R₄ₐ, R₅ₐ, R₆ₐ, and R₇ₐ are as defined in the first aspect; and R₈ₐ, R₉ₐ, R₁₀ₐ, and R₁₁ₐ are all H.

In another preferred embodiment, the salt is a pharmaceutically or diagnostically acceptable salt.

In another preferred embodiment, the probe is **BIN-3.**

In a third aspect of the present disclosure, provided is a photosensitizer, wherein the photosensitizer is a compound represented by formula I or a salt thereof; wherein n, R₁ₐ, R₂ₐ, R₃ₐ, R₄ₐ, R₅ₐ, R₆ₐ, and R₇ₐ are as defined in the first aspect; and at least one of R₈ₐ, R₉ₐ, R₁₀ₐ, or R₁₁ₐ is a heavy atom, while the remaining groups are each independently selected from H and a heavy atom.

In another preferred embodiment, the salt is a pharmaceutically or diagnostically acceptable salt.

In another preferred embodiment, one, two, or three of R₅ₐ, R₉ₐ, R₁₀ₐ, and R₁₁ₐ are heavy atoms.

In another preferred embodiment, the heavy atoms are each independently I or Br.

In another preferred embodiment, the photosensitizer is a near-infrared photosensitizer.

In another preferred embodiment, the photosensitizer is **BIN-3I.**

In a fourth aspect of the present disclosure, provided is a composition, wherein the composition comprises:
(1) the compound according to the first aspect or a salt thereof; and
(2) a pharmaceutically or diagnostically acceptable carrier.

In another preferred embodiment, the salt is a pharmaceutically or diagnostically acceptable salt.

In another preferred embodiment, the composition is a probe composition.

In another preferred embodiment, the composition is a photosensitizer composition.

In a fifth aspect of the present disclosure, provided is a probe composition, wherein the probe composition comprises:
(1) the probe according to the second aspect; and
(2) a pharmaceutically or diagnostically acceptable carrier.

In a sixth aspect of the present disclosure, provided is a photosensitizer composition, wherein the photosensitizer composition comprises:
(1) the photosensitizer according to the third aspect; and
(2) a pharmaceutically or diagnostically acceptable carrier.

In a seventh aspect of the present disclosure, provided is a use of the compound according to the first aspect or a salt thereof, or the probe according to the second aspect, or the composition according to the fourth aspect, or the probe composition according to the fifth aspect, wherein the use is selected from the group consisting of the following or any combination thereof:
(a) for the detection of β-lactamase (preferably, the detection of β-lactamase is performed *in vitro* and is non-therapeutic and non-diagnostic);
(b) for the detection or imaging of drug-resistant bacteria (preferably, the detection or imaging of bacteria is performed *in vitro* and is non-therapeutic and non-diagnostic);
(c) for the manufacture of a detection reagent for the detection or imaging of drug-resistant bacteria.

In another preferred embodiment, the drug-resistant bacteria are β-lactamase-expressing drug-resistant bacteria.

In another preferred embodiment, the detection or imaging of bacteria comprises determining whether the bacteria being detected express β-lactamase and the level of β-lactamase expressed.

In another preferred embodiment, the detection reagent is capable of detecting drug-resistant bacteria in a living subject.

In an eighth aspect of the present disclosure, provided is a use of the compound according to the first aspect or a salt thereof, or the photosensitizer according to the third aspect, or the composition according to the fourth aspect, or the photosensitizer composition according to the sixth aspect, wherein the use is selected from the group consisting of the following or any combination thereof:
(a) for the killing of drug-resistant bacteria (preferably, the killing of drug-resistant bacteria is performed *in vitro* and is non-therapeutic);
(b) for the manufacture of a medicament for the killing of drug-resistant bacteria or for the treatment or prevention of diseases caused by infections with drug-resistant bacteria.

In a ninth aspect of the present disclosure, provided is a detection kit,
wherein the kit comprises the compound according to the first aspect or a salt thereof, or the composition according to the fourth aspect.

In a tenth aspect of the present disclosure, provided is a method of imaging drug-resistant bacteria, wherein the method comprises the steps of: incubating the compound according to the first aspect or a salt thereof with a subject, and obtaining an image of the drug-resistant bacteria using an instrument (e.g., a fluorescence microscope).

In another preferred embodiment, the method is performed *in vitro* and is non-therapeutic.

In another preferred embodiment, the subject comprises bacteria, cells, tissues, and culture media (e.g., a culture dish in which bacteria have been cultured).

### β-lactamase-activatable probes or photosensitizers

β-lactamase exhibits high efficiency in hydrolyzing antibiotics during the catalytic process. Bacterial expression of β-lactamase not only leads to antibiotic resistance, but also provides a unique opportunity for the detection and even selective killing of drug-resistant bacteria *in vivo.* Since the development of the first β-lactamase fluorescent probe by the laboratory of Roger Y. Tsien in 1998, a large number of β-lactamase fluorescent substrates have been reported to date. In *in vitro* solution-based assays, most of these probes exhibit high sensitivity and selectivity for the detection of β-lactamase activity. However, monitoring bacterial infections in living organisms using fluorescent probes remains challenging. The challenge arises primarily because β-lactamase is an enzyme mainly localized in the bacterial periplasmic space or secreted extracellularly, while the *in vivo* environment of living organisms is highly complex and variable.

For *in vivo* imaging with fluorescent probes, it is not only necessary to achieve enhanced fluorescence intensity as in *in vitro* studies, but also particularly crucial that the activated fluorophore be effectively retained or even enriched at the target site. In addition, the excitation and emission of near-infrared (NIR) fluorescence are essential for *in vivo* imaging, as the NIR spectral region minimizes interference from autofluorescence *in vivo* and enables deeper tissue penetration. The same strategy is applied to photosensitizers capable of photodynamic therapy. By introducing iodine atoms into near-infrared dyes, the heavy-atom effect enhances the generation of ¹O₂. Upon near-infrared light irradiation, increased production of reactive oxygen species is achieved, thereby enabling both signal retention and inhibition or even killing of drug-resistant bacteria, ultimately realizing a theranostic platform.

In this study, the conventional design of β-lactamase-activatable fluorescent probes is combined with a quinone methide (QM)-based self-anchoring strategy and modifications to hydrophilic and lipophilic properties, thereby developing novel β-lactamase-responsive near-infrared fluorescent probes and photosensitizers for fluorescence imaging and treatment of bacterial infections in mice. In addition to exhibiting enhanced fluorescence emission upon selective activation by β-lactamase, as do conventional fluorescent probes and photosensitizers, these novel fluorescent probes and photosensitizers can effectively accumulate on β-lactamase-expressing bacteria through changes in hydrophilicity and through the formation of covalent bonds with proteins located on or around the bacteria. These characteristics provide unique advantages for *in vivo* imaging and photodynamic therapy, enabling the selective monitoring and killing of drug-resistant bacteria.

### Labeled compounds and photosensitizers of the present disclosure

One objective of the present disclosure is to provide a **β-lactamase-activatable** enrichment strategy for the detection and treatment of drug-resistant bacteria. Specifically, the present disclosure utilizes a β-lactam structure as the recognition group for fluorescent probes and photosensitizers, and combines a hydrophilicity-switchable and charge-switchable quinone methide system with a multi-responsive design strategy to develop near-infrared fluorescent probes and photosensitizers for the detection and killing of β-lactamase-expressing drug-resistant bacteria.

Another object of the present disclosure is to provide a synthetic method.

Yet another object of the present disclosure is to provide a specific application of the probes and photosensitizers, wherein enzymatic activation enables selective labeling of drug-resistant bacteria and facilitates enrichment-based detection and killing of drug-resistant bacteria *in vivo.*

In one aspect of the present disclosure, provided is a compound represented by formula I-A, wherein formula I-A represents a β-lactamase-responsive near-infrared probe and a β-lactamase-responsive near-infrared photosensitizer;

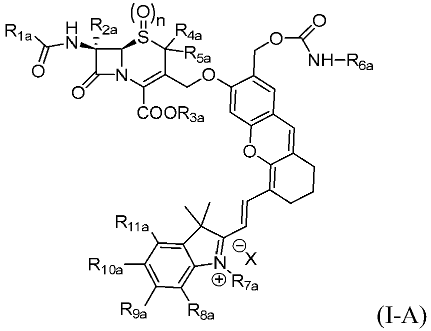

wherein each group is as defined in the first aspect.

In some embodiments, n is 0, 1, or 2; and/or

R₁ₐ is selected from the group consisting of substituted or unsubstituted C₁₋₆ alkyl, substituted or unsubstituted phenyl, substituted or unsubstituted 5- or 6-membered heteroaryl, substituted or unsubstituted C₁₋₄ alkylene-phenyl, substituted or unsubstituted C₁₋₄ alkylene-5- or 6-membered heteroaryl, and water-solubilizing groups such as zwitterionic groups 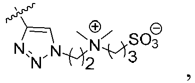 dicarboxylate groups 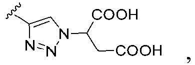 tricarboxylate groups 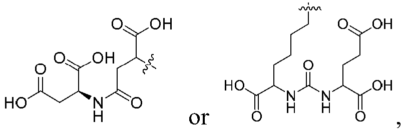 and disulfonate groups 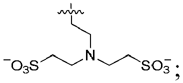 and/or
R₂ₐ is selected from the group consisting of H and OMe; and/or
R₃ₐ is selected from the group consisting of H, substituted or unsubstituted C₁₋₆ alkyl, and a monovalent cation such as Na⁺; and/or
R₄ₐ and R₅ₐ are each independently selected from the group consisting of H, methyl, and ethyl; alternatively, both R₄ₐ and R₅ₐ are 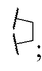 and/or
R₆ₐ is selected from the group consisting of water-solubilizing groups linked to a carbamate structure, such as zwitterionic groups 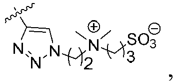 dicarboxylate groups 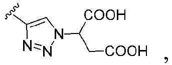 tricarboxylate groups 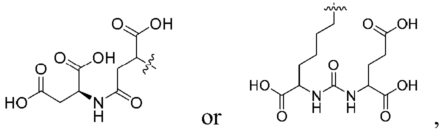 and disulfonate groups 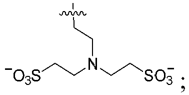 wherein upon enzymatic activation, the carbamate structure is cleaved and the water-solubilizing group is released; and/or
R₇ₐ is substituted or unsubstituted C₁₋₆ alkyl; and/or
R₈ₐ, R₉ₐ, R₁₀ₐ, and R₁₁ₐ are H (for near-infrared fluorescent probes) or I or Br (for photosensitizers), wherein the introduction of I or Br as heavy atoms enhances the efficiency of the photosensitizer in generating ROS; and/or
X is I, Br, Cl, *etc.*

In a second aspect of the present disclosure, provided are the following compounds and methods of synthesizing the same.

### Probe structure (II)

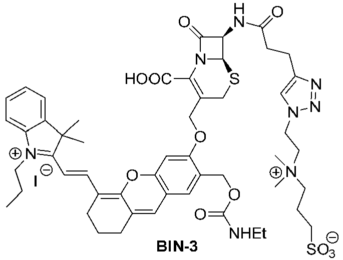

### Photosensitizer structure (III)

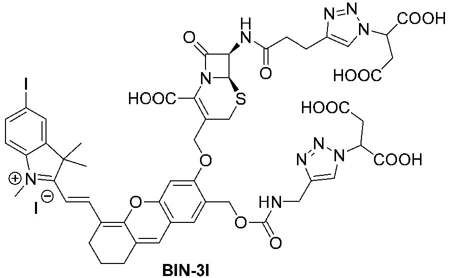

In a third aspect of the present disclosure, provided are methods of evaluating the reagents **(II**) and **(III)** according to the second aspect, demonstrating their use for highly sensitive and selective detection and killing of β-lactamase-expressing drug-resistant bacteria both *in vitro* and *in vivo.*

The present disclosure has developed a β-lactamase-activatable near-infrared enrichment-type probe and photosensitizer. Experimental analyses, including UV absorption spectroscopy, fluorescence spectroscopy, HPLC analysis, SDS-PAGE analysis, and near-infrared fluorescence scanning before and after enzymatic hydrolysis *in vitro,* have demonstrated that the β-lactamase-responsive probe and photosensitizer can be selectively activated by β-lactamase, release fluorescence signals, and form covalent conjugates with nucleophiles.

Subsequently, fluorescence imaging and probe-enrichment studies are performed on β-lactamase-expressing drug-resistant bacteria. Experimental results have demonstrated that the probe and photosensitizer can selectively label and accumulate on drug-resistant bacteria. Finally, a series of *in vivo* imaging experiments have demonstrated that the probe can be retained or even enriched at the site of drug-resistant bacterial infection within the highly dynamic environment of living organisms, and enables rapid and real-time monitoring of the therapeutic efficacy of inhibitors or antibiotics against bacterial infections *in vivo* based on fluorescence intensity.

### Terminology

As used herein, unless otherwise specified, the terms or abbreviations have the general meanings well known to those skilled in the art. For example, Ph represents phenyl.

Unless otherwise indicated, the term "alkyl", either alone or as part of another substituent (*e.g*., the alkyl moiety of an alkoxy group), refers to a linear or branched hydrocarbon group having the specified number of carbon atoms (*i.e*., C₁₋₆ indicates 1 to 6 carbon atoms). Examples of alkyl groups include: methyl (Me), ethyl (Et), *n*-propyl, isopropyl, *n*-butyl, *tert*-butyl, isobutyl, *sec*-butyl, *n*-pentyl, *n*-hexyl, *etc*. In the present disclosure, preferred alkyl groups are those containing 1 to 4 carbon atoms, *i.e*., C₁₋₄ alkyl.

### The main advantages of the present disclosure include one or more of the following:

(a) The compounds of the present disclosure may be conjugated with different specifically activatable groups according to different application requirements, thereby activating the molecule at the target site. For example, for oncology applications, groups that are specifically activated in the tumor environment may be introduced into the molecule; for applications targeting β-lactamase-expressing drug-resistant bacteria, groups that are specifically activated in the presence of β-lactamase may be introduced into the molecule.
(b) The probes (*e.g*., ALPIN-6 and ALPIN-7) and photosensitizers (*e.g*., ALPIN-6I) of the present disclosure possess a "one-trigger, three-response" capability, meaning that upon a single triggering event by an enzyme or disease-related microenvironmental factor, the following three responses are triggered: 1) transformation from highly hydrophilic and poorly cell membrane-permeable molecules into highly lipophilic and cell membrane-permeable molecules, thereby reducing their clearance rate and enhancing their retention efficiency at the target site; 2) formation of a quinone methide intermediate that reacts with macromolecules such as proteins or intracellular nucleophiles to form a covalent bond, thereby anchoring the fluorophore or photosensitizer; 3) activation of near-infrared fluorescence or photoacoustic signals or activation of photosensitizing activity under near-infrared light irradiation.
(c) The probes (*e.g*., ALPIN-6 and ALPIN-7) and photosensitizers (*e.g*., ALPIN-6I) of the present disclosure possess functions of activation and enrichment at the detection site.
(d) Upon selective hydrolysis by β-lactamase, the probes or photosensitizers of the present disclosure can not only enhance fluorescence intensity, as do most conventional β-lactamase-responsive probes and photosensitizers, but also significantly accumulate on β-lactamase-expressing bacteria.
(e) The probes and photosensitizers of the present disclosure exhibit good selectivity. Experimental results have demonstrated highly selective fluorescent labeling of β-lactamase-positive bacteria without cross-labeling even in the presence of other susceptible bacteria, thereby enabling wash-free and real-time visualization of drug-resistant bacteria.
(f) The probes or photosensitizers of the present disclosure can selectively accumulate at the infection site. Consequently, the probes of the present disclosure enable sensitive and non-invasive visualization of β-lactamase-expressing bacterial infections *in vivo* with low signal-to-noise ratio, while the photosensitizers of the present disclosure enable precise killing of drug-resistant bacteria.

### BRIEF DESCRIPTION OF THE DRAWINGS

FIG. 1 shows the mechanism of action of the probes or photosensitizers of the present disclosure.
FIG. 2 shows the verification of the solution-phase enzymatic response of the near-infrared fluorescent probe ALPIN-6.
FIG. 3 shows the verification of the solution-phase enzymatic response of the near-infrared fluorescent probe ALPIN-6.
FIG. 4 shows the verification of the solution-phase enzymatic response of the near-infrared fluorescent probe ALPIN-6.
FIG. 5 shows the verification of the solution-phase enzymatic response of the near-infrared fluorescent probe ALPIN-6.
FIG. 6 shows the verification of the solution-phase enzymatic response of the near-infrared fluorescent probe ALPIN-6.
FIG. 7 shows a comparison of imaging results obtained using near-infrared fluorescent probes ALPIN-6, ALPIN-5, and ALPIN-4 in HeLa cells overexpressing alkaline phosphatase (ALP), as well as a comparison of imaging results between HeLa cells and HEK293 cells weakly expressing ALP.
FIG. 8 shows real-time imaging of HeLa cells using the near-infrared fluorescent probe ALPIN-6.
FIG. 9 shows specific imaging of mixed HeLa and HEK293 cells using the near-infrared fluorescent probe ALPIN-6.
FIG. 10 shows real-time imaging of HeLa tumor-bearing mice using the near-infrared fluorescent probe ALPIN-6.
FIG. 11 shows imaging of HeLa tumor-bearing mice using the near-infrared fluorescent probe ALPIN-6.
FIG. 12 shows real-time imaging of HeLa tumor-bearing mice using the near-infrared fluorescent probe ALPIN-6.
FIG. 13 shows the distribution of the near-infrared fluorescent probe ALPIN-6 in HeLa tumor-bearing mice.
FIG. 14 shows quantification of the accumulation of the near-infrared photosensitizer ALPIN-6I in HeLa cells.
FIG. 15 shows ROS production by the photosensitizer ALPIN-6I in HeLa cells.
FIG. 16 shows the killing of tumor cells by the photosensitizer ALPIN-6I.
FIG. 17 shows the selective killing of tumor cells by the photosensitizer ALPIN-6I in mixed cell populations.
FIG. 18 shows real-time imaging of HeLa tumor-bearing mice using the near-infrared photosensitizer ALPIN-6I.
FIG. 19 shows photodynamic therapy of HeLa tumor-bearing mice using the near-infrared photosensitizer ALPIN-6I.
FIG. 20 shows photodynamic therapy of HeLa tumor-bearing mice using the near-infrared photosensitizer ALPIN-6I.
FIG. 21 shows photodynamic therapy of HeLa tumor-bearing mice using the near-infrared photosensitizer ALPIN-6I.
FIG. 22 shows a comparison of imaging results obtained using the near-infrared fluorescent and photoacoustic probes ALPIN-7 and ALPIN-8 in HeLa cells overexpressing alkaline phosphatase (ALP), as well as a comparison of imaging results between HeLa cells and HEK293 cells weakly expressing ALP.
FIG. 23 shows real-time imaging of HeLa cells using the near-infrared fluorescent and photoacoustic probe ALPIN-7.
FIG. 24 shows real-time imaging of HeLa tumor-bearing mice using the near-infrared fluorescent and photoacoustic probe ALPIN-7.
FIG. 25 shows the fluorescence intensity of the near-infrared fluorescent and photoacoustic probe ALPIN-7 in HeLa tumor-bearing mice.
FIG. 26 shows the fluorescence distribution of the near-infrared fluorescent and photoacoustic probe ALPIN-7 in HeLa tumor-bearing mice.
FIG. 27 shows UV absorption and fluorescence spectra of **BIN-3** before and after incubation with β-lactamase TEM-1 according to an example of the present disclosure.
FIG. 28 shows UV absorption and fluorescence spectra of **BIN-2** before and after incubation with β-lactamase TEM-1 according to an example of the present disclosure.
FIG. 29 shows UV absorption and fluorescence spectra of **BIN-3** before and after incubation with β-lactamase TEM-1 according to an example of the present disclosure.
FIG. 30 shows UV absorption and fluorescence spectra of **BIN-2** after incubation with β-lactamase TEM-1 according to an example of the present disclosure.
FIG. 31 shows UV absorption and fluorescence spectra of **BIN-3I** after incubation with β-lactamase TEM-1 according to an example of the present disclosure.
FIG. 32 shows UV absorption and fluorescence spectra of **BIN-3I** before and after incubation with β-lactamase TEM-1 according to an example of the present disclosure.
FIG. 33 shows UV absorption and fluorescence spectra of **BIN-3I** before and after incubation with β-lactamase TEM-1 according to an example of the present disclosure.
FIG. 34 shows UV absorption and fluorescence spectra of **BIN-2I** before and after incubation with β-lactamase TEM-1 according to an example of the present disclosure.
FIG. 35 is a histogram of fluorescence intensity of **BIN-3** and **BIN-2** (5 µM) in the presence of different enzymes and proteins according to an example of the present disclosure. 1: TEM-1 (10 nM); 2: TEM-1 (10 nM) + inhibitor (100 µM); 3: lysozyme (2 U/mL); 4: β-glucosidase (5 µM); 5: lipase (2 U/mL); 6: bovine serum albumin (10 µM); 7: glutathione (2 µM); 8: lysine (1 mM); 9: cysteine (1 mM); 10: tryptophan (1 mM); λₑₓ/λₑₘ = 687/714 nm.
FIG. 36 shows SDS-PAGE and dual-color near-infrared fluorescence imaging after incubation of probes **BIN-3** and **BIN-2,** and photosensitizers **BIN-3I** and **BIN-2I** (10 µM) with TEM-1 and nucleophile BSA according to an example of the present disclosure.
FIG. 37 shows SDS-PAGE and dual-color near-infrared fluorescence imaging after incubation of probes **BIN-3** and **BIN-2,** and photosensitizers **BIN-3I** and **BIN-2I** (10 µM) with TEM-1 and nucleophile BSA according to an example of the present disclosure.
FIG. 38 shows fluorescence imaging of bla-positive and bla-negative bacteria using the primary probe **BIN-3** and the control probe **BIN-2.**
FIG. 39 shows fluorescence imaging of bla-negative bacteria using the primary probe **BIN-3** and the control probe **BIN-2** before and after washing.
FIG. 40 shows selective imaging of mixed bacterial populations using the primary probe **BIN-3** and the control probe **BIN-2.**
FIG. 41 shows enrichment studies of **BIN-3** in bla-positive bacteria according to an example of the present disclosure.
FIG. 42 shows enrichment studies of **BIN-2** in bla-positive bacteria according to an example of the present disclosure.
FIG. 43 shows hematological analysis, blood biochemistry data, and H&E-stained tissue sections of mice administered **BIN-3** and **BIN-2** according to an example of the present disclosure.
FIG. 44 shows representative whole-body fluorescence images of mice after intravenous injection of the probe, or after intramuscular injection of an inhibitor (100 mM, 50 µL) prior to probe injection.
FIG. 45 shows the temporal changes in fluorescence intensity of the right thigh.
FIG. 46 shows the temporal changes in fluorescence intensity and signal-to-noise ratio of the right thigh relative to whole-body fluorescence signals.
FIG. 47 shows photographs of the infected left and right thighs of mice at 10 hours after probe injection.
FIG. 48 shows fluorescence images of the infected thighs and major organs at 10 hours after probe injection.
FIG. 49 shows quantification of fluorescence intensity in the infected thighs and major organs at 10 hours after probe injection.
FIG. 50 shows fluorescence images of mice with thigh infections containing different numbers of bla-positive *Escherichia coli* following intravenous injection of the probe, in which the black circles indicate the regions used for whole-body signal quantification, and the inset shows fluorescence images acquired at λ_{ex/em} = 660/710 nm.
FIG. 51 shows quantification of fluorescence intensity in the infected right thigh.
FIG. 52 shows signal-to-noise ratios of the thigh inoculated with different bacterial loads relative to whole-body fluorescence signals.
FIG. 53 shows whole-body fluorescence images acquired using the mCherry channel (λ_{ex/em} = 580/620 nm) and the near-infrared channel (λ_{ex/em} = 660/710 nm) at 10 hours after injection of the primary probe.
FIG. 54 shows photographs of infected thighs, with intact skin or after skin removal, at 10 hours after probe injection.
FIG. 55 shows fluorescence images of the posterior thigh acquired using the mCherry and near-infrared channels at 10 hours after injection of the primary probe.

### DETAILED DESCRIPTION OF THE PREFERRED EMBODIMENT

The present disclosure is further described below in conjunction with specific examples. It should be understood that these examples are provided merely to illustrate the present disclosure and are not intended to limit the scope of the present disclosure. Experimental methods for which specific conditions are not indicated in the following examples are generally performed according to conventional conditions or according to conditions recommended by the manufacturer. Unless otherwise specified, percentages and parts are by weight.

The following describes specific embodiments of the preparation methods of the near-infrared fluorescent probes and photosensitizers of the present disclosure with functions of activation and enrichment at the detection site. Note: In the implementation of the present disclosure, ¹H-NMR and ¹³C-NMR spectra were recorded on a Bruker 400 MHz instrument using deuterated chloroform (CDCl₃) or deuterated dimethyl sulfoxide (*d₆*-DMSO) as the solvent and tetramethylsilane (TMS) as the internal standard; all solvents were of chromatographic grade, analytical grade, or chemically pure grade.

### Preparation Example 1: Synthesis of near-infrared probes

**In this example, the preparation process of a near-infrared probe containing** a **cleavable water-solubilizing group is described, using the preparation method of a near-infrared fluorescent probe (ALPIN-6) as an example.**

**The structure of ALPIN-6 is as follows:**

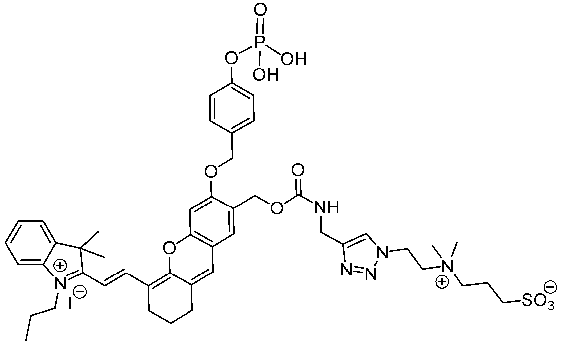

**The preparation route of ALPIN-6 is as follows:**

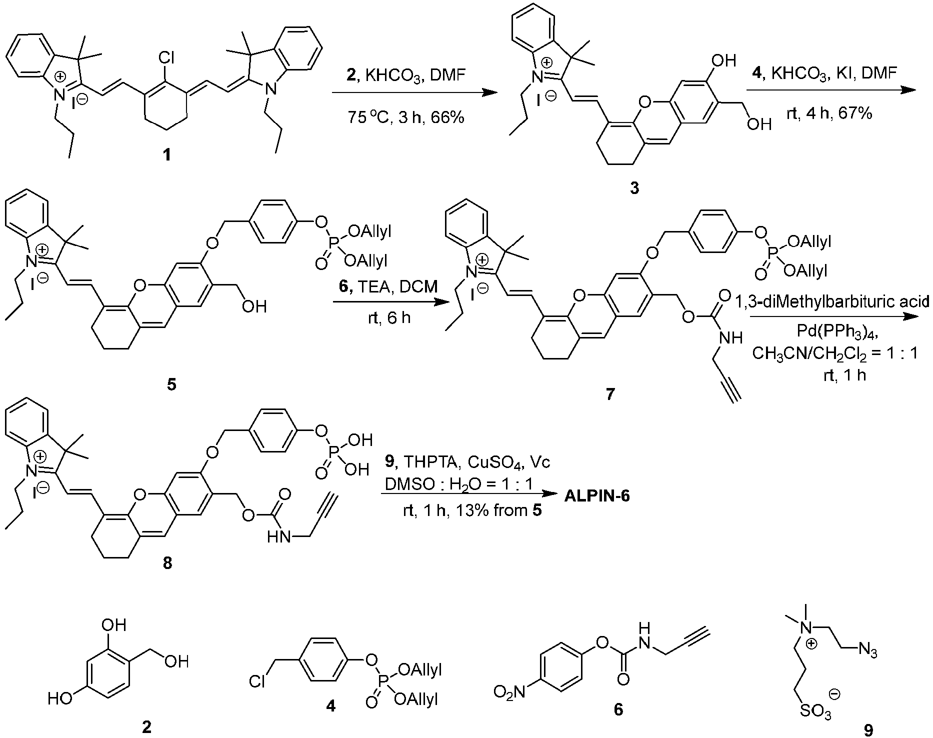

### a. Preparation of Compound 3

Compound **1** (524.2 mg, 0.78 mmol), Compound **2** (948.0 mg, 6.77 mmol), and KHCO₃ (255.1 mg, 2.55 mmol) were added to a 25 mL Schlenk tube, which was evacuated and purged with N₂ three times. Under a N₂ atmosphere, ultra-dry DMF (4 mL) was added, and the mixture was heated to 75°C and stirred for 4 hours. After completion of the reaction, the reaction mixture was cooled to room temperature, diluted with ethyl acetate (10 mL), and washed three times with water (10 mL). The organic phase was separated using a separatory funnel, dried over anhydrous sodium sulfate, and filtered under reduced pressure. The residue was mixed with silica gel and purified by column chromatography. The product was eluted with DCM:CH₃OH = 5:1 and concentrated under reduced pressure using a rotary evaporator to obtain a blue solid (293.3 mg) with a yield of 66%. Spectral data: ¹H NMR (400 MHz, CD₃OD) δ 8.74 (d, *J* = 14.8 Hz, 1H), 7.63 (d, *J* = 7.6 Hz, 1H), 7.56 - 7.45 (m, 4H), 7.46 - 7.38 (m, 1H), 6.88 (s, 1H), 6.44 (d, *J* = 15.2 Hz, 1H), 4.69 (s, 2H), 4.29 (t, *J* = 7.4 Hz, 2H), 2.78 (t, *J* = 5.8 Hz, 2H), 2.72 (t, *J* = 5.8 Hz, 2H), 2.00 - 1.88 (m, 4H), 1.82 (s, 6H), 1.08 (t, *J* = 7.4 Hz, 3H). ¹³C NMR (101 MHz, CD₃OD) δ 176.92, 162.43, 159.58, 153.96, 144.99, 141.80, 141.70, 135.36, 128.79, 128.09, 126.76, 126.58, 126.32, 122.33, 114.83, 114.19, 112.17, 102.28, 100.79, 58.57, 50.26, 45.93, 28.57, 27.08, 23.67, 20.73, 20.31, 10.23. HRMS (ESI) m/z calcd for C₃₁H₃₂NO₃ (M-I)⁺ 442.2382, found 442.2377.

### b. Preparation of Compound 5

Dye **3** (50.5 mg, 0.089 mmol), KI (142.8 mg, 0.860 mmol), KHCO₃ (49.4 mg, 0.493 mmol), and 18-crown-6 (20.3 mg, 0.077 mmol) were added to a 10 mL reaction tube, which was evacuated and purged with N₂ three times. Under a N₂ atmosphere, ultra-dry DMF (1.0 mL) was added, followed by Compound **4** (48.4 mg, 0.159 mmol), and the mixture was stirred at room temperature for 4 hours. After completion of the reaction, the reaction mixture was diluted with DCM (10.0 mL) and washed with water (5.0 mL). The organic phase was separated using a separatory funnel, dried over anhydrous sodium sulfate, and filtered under reduced pressure. The residue was mixed with silica gel and purified by column chromatography. The product was eluted with DCM:CH₃OH = 10:1 and concentrated under reduced pressure using a rotary evaporator to obtain a blue solid compound (50.1 mg) with a yield of 67%. Spectral data: ¹H NMR (400 MHz, CDCl₃) ¹H NMR (600 MHz, CD₃OD) δ 8.74 (d, *J* = 15.0 Hz, 1H), 7.68 (d, *J* = 7.2 Hz, 1H), 7.58 (s, 1H), 7.53 - 7.51 (m, 2H), 7.48 - 7.44 (m, 4H), 7.27 (d, *J* = 7.8 Hz, 2H), 7.12 (s, 1H), 6.48 (d, *J* = 14.4 Hz, 1H), 6.00 - 5.78 (m, 2H), 5.30 (s, 2H), 5.28 (d, *J* = 18.0 Hz, 2H), 5.08 (d, *J* = 10.2 Hz, 2H), 4.70 (s, 2H), 4.44 - 4.47 (m, 2H), 4.31 (t, *J* = 7.2 Hz, 4H), 2.81- 2.78 (m 2H), 2.72 - 2.70 (m, 2H), 1.96 - 1.92 (m, 4H), 1.84 (s, 6H), 1.08 (t, *J* = 7.2 Hz, 3H). ¹³C NMR (151 MHz, CD₃OD) δ 177.32, 162.31, 159.46, 153.89, 153.05 (d, *J* = 6.7 Hz), 145.40, 141.93, 141.56, 134.90, 134.33 (d, *J* = 7.8 Hz), 130.95, 129.65 (d, *J* = 2.5 Hz), 128.76, 128.43, 127.29, 126.74, 126.00, 124.85, 122.50, 120.19 (d, *J* = 5.1 Hz), 115.44, 115.06, 114.53, 112.06, 102.64, 99.28, 82.38, 70.35, 70.28, 69.59, 66.38 (d, *J* = 5.5 Hz), 58.27, 50.47, 48.46, 43.36, 28.62, 27.20, 26.06, 23.92, 20.18, 15.15. HRMS (ESI) m/z calcd for C₄₄H₄₇NO₇P (M-I)⁺ 732.3090, found 732.3089.

### c. Preparation of Compound ALPIN-6

Compound **5** (33.5 mg, 0.040 mmol) was added to a 10 mL reaction flask, followed by ultra-dry DCM (0.5 mL), Compound **6** (16.7 mg, 0.076 mmol), and triethylamine (20 µL, 0.144 mmol), and the mixture was stirred at room temperature for 6 hours. The reaction mixture was diluted with DCM, washed with water, dried over anhydrous sodium sulfate, and filtered under reduced pressure. The filtrate was concentrated under reduced pressure using a rotary evaporator to obtain Compound **7,** which was used directly in the next step without further purification.

Compound **7** (22.8 mg), 1,3-dimethylbarbituric acid (7.8 mg, 0.05 mmol), and Pd(PPh₃)₄ (5.8 mg, 0.005 mmol) were added to a 10 mL Schlenk tube, which was evacuated and purged with N₂ three times, followed by addition of MeCN (0.5 mL) and DCM (0.5 mL). After stirring at room temperature for 1 hour, the reaction mixture was concentrated under reduced pressure using a rotary evaporator, followed by addition of Compound **9** (10.2 mg, 0.043 mmol), CuSO₄ (3.5 mg, 0.022 mmol), ascorbic acid (17.5 mg, 0.099 mmol), DMSO (0.5 mL), and H₂O (0.5 mL). The mixture was stirred at room temperature for 1 hour, followed by purification by preparative HPLC to obtain the target compound (2.1 mg) with a three-step yield of 13%.

Spectral data: ¹H NMR (600 MHz, *d*₆-DMSO) δ 8.57 (d, *J* = 15.0 Hz, 1H), 8.22 (s, 1H), 8.11 (s, 1H), 7.91- 7.87 (m, 1H), 7.64 - 7.62 (m, 2H), 7.60 (s, 1H), 7.57 - 7.50 (m, 4H), 7.24 - 7.22 (m, 2H), 6.59 (d, *J* = 15.0 Hz, 1H), 5.34 (s, 2H), 5.10 (s, 2H), 4.92 (t, *J* = 6.9 Hz, 2H), 4.40 (t, *J* = 6.9 Hz, 2H), 4.27 (d, *J* = 6.0 Hz, 2H), 3.85 (t, *J* = 6.9 Hz, 2H), 3.07 (s, 6H), 2.73 - 2.68 (m, 4H), 2.44 (t, *J* = 6.9 Hz, 2H), 1.99 - 1.94 (m, 2H), 1.86 - 1.82 (m, 6H), 1.79 (s, 6H), 0.99 (t, *J* = 7.2 Hz, 3H). HRMS (ESI) m/z calcd for C₄₇H₅₈N₆O₁₁PS (M-I)⁺ 945.3616, found 945.3624.

### Preparation Example 2: Synthesis of near-infrared photosensitizers

**In this example, the preparation process of near-infrared photosensitizers is described, using the preparation method of a near-infrared photosensitizer (ALPIN-6I) as an example.**

**The structure of ALPIN-6I is as follows:**

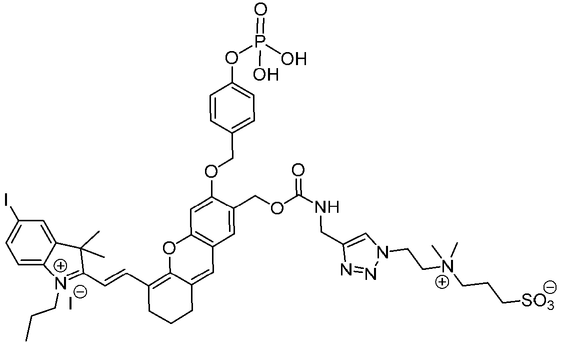

**The preparation route of ALPIN-6I is as follows:**

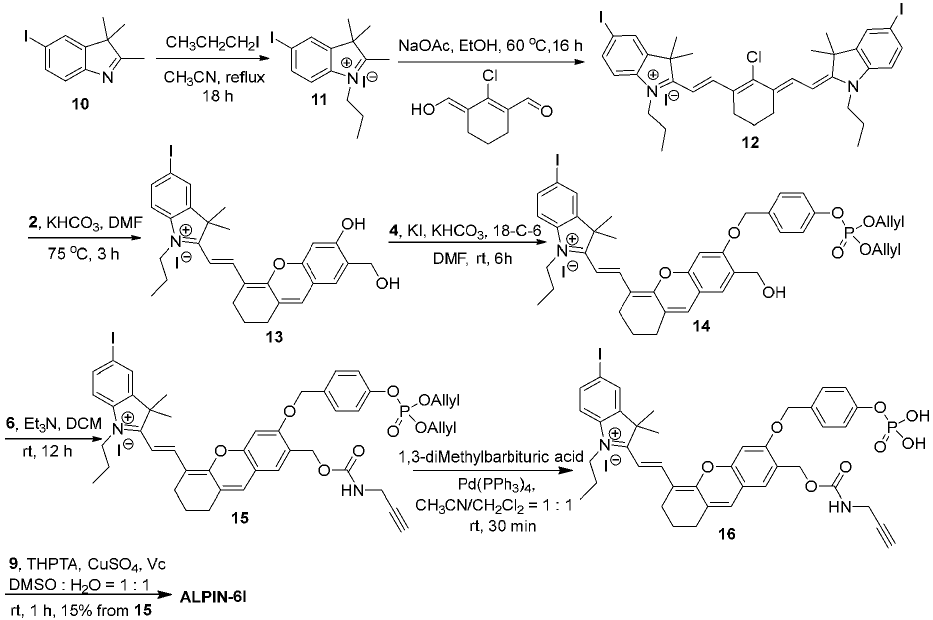

### a. Preparation of Compound 11

Compound **10** (5-iodo-2,3,3-trimethyl-3H-indole, 5.0 g, 17.537 mmol) was added to a 100 mL reaction flask, followed by ultra-dry ACN (30 mL) and 1-iodopropane (8.5 mL, 87.683 mmol), and the mixture was heated under reflux for 18 hours. The reaction mixture was concentrated under reduced pressure using a rotary evaporator to remove the acetonitrile, followed by addition of acetone, then heated to 60°C, and cooled to precipitate a solid, which was filtered under reduced pressure to obtain a pink solid (7.489 g) with a yield of 94%. Spectral data: ¹H NMR (600 MHz, *d*₆-DMSO) δ 8.32 (d, *J* = 1.2 Hz, 1H), 8.00 (dd, *J* = 8.4, 0.6 Hz, 1H), 7.82 (d, *J* = 8.4 Hz, 1H), 4.41 (t, *J* = 7.8 Hz, 2H), 2.83 (s, 3H), 1.89 - 1.80 (m, 2H), 1.54 (s, 6H), 0.98 (t, *J* = 7.2 Hz, 3H). HRMS (ESI) m/z calcd for C₁₄H₁₉IN (M-I)⁺ 328.0557, found 328.0563.

### b. Preparation of Compound 12

Anhydrous sodium acetate (81.3 mg, 0.991 mmol), 2-chloro-3-(hydroxymethylene)-1-cyclohexene-1-carbaldehyde (805.0 mg, 4.664 mmol), and Compound **11** (4.443 g, 9.762 mmol) were added to a reaction flask, which was purged with N₂ three times, followed by addition of ethanol (8 mL), and the mixture was stirred at 60°C for 16 hours. The residue was mixed with silica gel and purified by column chromatography to obtain a green solid (2.806 g) with a yield of 66%. Spectral data: ¹H NMR (400 MHz, CDCl₃) δ 8.32 (d, *J* = 13.6 Hz, 2H), 7.70 (d, *J* = 8.0 Hz, 2H), 7.65 (s, 2H), 7.00 (d, *J* = 8.0 Hz, 2H), 6.26 (d, *J* = 13.6 Hz, 2H), 4.44 - 3.93 (m, 4H), 2.94 - 2.60 (m, 4H), 2.07 - 1.83 (m, 6H), 1.71 (s, 12H), 1.06 (t, *J* = 6.8 Hz, 6H). HRMS (ESI) m/z calcd for C₃₆H₄₂ClI₂N₂ (M-I)⁺ 791.1120, found 791.1134.

### c. Preparation of Compound 13

Compound **12** (1.973 g, 2.147 mmol), Compound **2** (1.572 g, 11.217 mmol), and KHCO₃ (676.1 mg, 6.753 mmol) were added to a reaction flask, which was purged with N₂ three times, followed by addition of DMF (5 mL), and the mixture was stirred at 75°C for 3 hours. The reaction mixture was diluted with ethyl acetate, washed three times with water, and dried over anhydrous sodium sulfate. The residue was mixed with silica gel and purified by column chromatography to obtain a blue compound (730.0 mg) with a yield of 49%. Spectral data: ¹H NMR (600 MHz, *d*₆-DMSO) δ 7.85 (d, *J* = 13.2 Hz, 1H), 7.74 (d, *J* = 1.2 Hz, 1H), 7.69 (s, 1H), 7.57 (dd, *J* = 8.4, 1.2 Hz, 1H), 7.37 (s, 1H), 6.92 (d, *J* = 8.4 Hz, 1H), 6.19 (s, 1H), 5.76 (s, 1H), 4.40 (s, 2H), 3.84 (t, *J* = 7.2 Hz, 2H), 2.72 - 2.56 (m, 4H), 1.84 - 1.76 (m, 2H), 1.72 - 1.64 (m, 2H), 1.61 (s, 6H), 0.93 (t, *J* = 7.2 Hz, 3H). ¹³C NMR (151 MHz, *d*₆-DMSO) δ 162.73, 157.81, 157.32, 143.42, 141.79, 139.32, 138.75, 136.41, 130.43, 129.54, 124.76, 116.76, 116.43, 110.76, 101.43, 94.65, 84.15, 58.89, 54.82, 46.65, 28.00, 27.06, 23.91, 20.83, 19.43, 11.08. HRMS (ESI) m/z calcd for C₂₉H₃₁INO₃ (M-I)⁺ 568.1343, found 568.1350.

### d. Preparation of Compound 14

Compound **13** (82.3 mg, 0.118 mmol), Compound **4** (79.0 mg, 0.240 mmol), KI (205.3 mg, 1.237 mmol), KHCO₃ (63.6 mg, 0.635 mmol), and 18-crown-6 (31.7 mg, 0.120 mmol) were added to a reaction flask, which was purged with N₂ three times, followed by addition of DMF (1 mL), and the mixture was stirred at room temperature for 6 hours. The reaction mixture was diluted with DCM, washed three times with water, and dried over anhydrous sodium sulfate. The residue was mixed with silica gel and purified by column chromatography to obtain a blue compound (57.1 mg) with a yield of 50%. Spectral data: ¹H NMR (600 MHz, CDCl₃) δ 8.59 (d, *J* = 14.4 Hz, 1H), 7.80 (d, *J* = 1.2 Hz, 1H), 7.74 (dd, *J* = 8.4, 1.2 Hz, 1H), 7.65 (s, 1H), 7.41 (d, *J* = 7.8 Hz, 2H), 7.38 (s, 1H), 7.24 (d, *J* = 8.4 Hz, 2H), 7.05 - 6.96 (m, 2H), 6.20 (d, *J* = 14.4 Hz, 1H), 5.99 - 5.89 (m, 2H), 5.37 (dd, *J* = 17.4, 1.2 Hz, 2H), 5.27 (dd, *J* = 10.2, 0.6 Hz, 2H), 5.17 (s, 2H), 4.76 (s, 2H), 4.69 - 4.60 (m, 4H), 4.11 (t, *J* = 7.2 Hz, 2H), 2.77 - 2.69 (m, 2H), 2.67 - 2.59 (m, 2H), 1.95 - 1.86 (m, 4H), 1.78 (s, 6H), 1.04 (t, *J* = 7.2 Hz, 3H). ¹³C NMR (151 MHz, CDCl₃) δ 175.11, 163.56, 159.97, 154.33, 150.46 (d, *J* = 6.8 Hz), 144.88, 143.55, 141.59, 137.92, 137.12, 132.69, 131.95 (d, *J* = 7.6 Hz), 131.82, 130.92, 128.69, 127.19, 126.57, 120.28 (d, *J* = 4.8 Hz), 118.86, 115.89, 115.45, 113.32, 101.65, 99.15, 90.34, 70.16, 68.98 (d, *J* = 5.6 Hz), 59.51, 50.05, 46.37, 28.96, 28.22, 24.20, 20.87, 20.21, 11.45. HRMS (ESI) m/z calcd for C₄₂H₄₆INO₇P (M-I)⁺ 834.2051, found 834.2056.

### e. Preparation of Compound 15

Compound **14** (57.1 mg, 0.059 mmol) and Compound **6** (31.6 mg, 0.144 mmol) were added to a reaction flask, followed by DCM and Et₃N (29 µL, 0.205 mmol), and the mixture was stirred at room temperature for 12 hours. The reaction mixture was diluted with DCM, washed three times with water, and dried over anhydrous sodium sulfate. The residue was mixed with silica gel and purified by column chromatography to obtain a blue compound (19.6 mg) with a yield of 48%. Spectral data: ¹H NMR (600 MHz, *d*₆-DMSO) δ 8.54 (d, *J* = 12.0 Hz, 1H), 8.24 (s, 1H), 7.89 (d, *J* = 12.0 Hz, 1H), 7.79 (t, *J* = 6.0 Hz, 1H), 7.60 (d, *J* = 6.0 Hz, 2H), 7.58 - 7.55 (m, 2H), 7.52 (d, *J* = 6.0 Hz, 1H), 7.32 (d, *J* = 6.0 Hz, 2H), 7.19 (s, 1H), 6.54 (d, *J* = 12.0 Hz, 1H), 6.01 - 5.87 (m, 2H), 5.41 (s, 2H), 5.34 (d, *J* = 18.0 Hz, 2H), 5.22 (d, *J* = 12.0 Hz, 2H), 5.12 (s, 2H), 4.70 - 4.58 (m, 4H), 4.35 (t, *J* = 6.0 Hz, 2H), 3.81 (d, *J* = 6.0 Hz, 2H), 3.13 (s, 1H), 2.78 - 2.65 (m, 4H), 1.88 - 1.79 (m, 4H), 1.78 (s, 6H), 0.97 (t, *J* = 6.0 Hz, 3H). HRMS (ESI) m/z calcd for C₄₆H₄₉IN₂O₈P (M-I)⁺ 915.2266, found 915.2270.

### f. Preparation of Compound ALPIN-6I

Compound **15** (26.6 mg, 0.029 mmol), 1,3-dimethylbarbituric acid (7.8 mg, 0.05 mmol), and Pd(PPh₃)₄ (4.5 mg, 0.0039 mmol) were added to a 10 mL Schlenk tube, which was evacuated and purged with N₂ three times, followed by addition of MeCN (0.5 mL) and DCM (0.5 mL). After stirring at room temperature for 30 minutes, the reaction mixture was concentrated under reduced pressure using a rotary evaporator, followed by addition of Compound **9** (10.2 mg, 0.043 mmol), CuSO₄ (2.1 mg, 0.013 mmol), ascorbic acid (22.1 mg, 0.125 mmol), DMSO (0.5 mL), and H₂O (0.5 mL). The mixture was stirred at room temperature for 1 hour, followed by purification by preparative HPLC to obtain the target compound (4.7 mg) with a three-step yield of 15%. Spectral data: ¹H NMR (600 MHz, *d*₆-DMSO) δ 8.55 (d, *J* = 14.7 Hz, 1H), 8.22 (s, 1H), 8.12 (s, 1H), 7.91 - 7.86 (m, 1H), 7.65 - 7.61 (m, 2H), 7.60 (s, 1H), 7.57 - 7.49 (m, 4H), 7.24 - 7.22 (m, 2H), 6.53 (d, *J* = 14.8 Hz, 1H), 5.36 (s, 2H), 5.10 (s, 2H), 4.93 (t, *J* = 6.9 Hz, 2H), 4.34 (t, *J* = 6.8 Hz, 2H), 4.28 (d, *J* = 5.7 Hz, 2H), 3.85 (t, *J* = 6.9 Hz, 2H), 3.07 (s, 6H), 2.73 - 2.68 (m, 4H), 2.45 (t, *J* = 6.8 Hz, 2H), 2.01 - 1.93 (m, 2H), 1.86 - 1.79 (m, 6H), 1.78 (s, 6H), 0.96 (t, *J* = 7.4 Hz, 3H). HRMS (ESI) m/z calcd for C₄₇H₅₇N₆O₁₁PSI (M-I)⁺ 1071.2583, found 1071.2585.

### Preparation Example 3: Preparation method of alkaline phosphatase-responsive near-infrared fluorescent and photoacoustic probe (ALPIN-7) with functions of activation and enrichment at the detection site

**The structure of ALPIN-7 is as follows:**

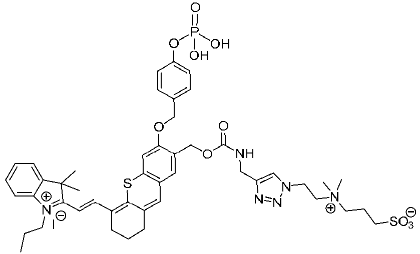

**The preparation route of ALPIN-7 is as follows:**

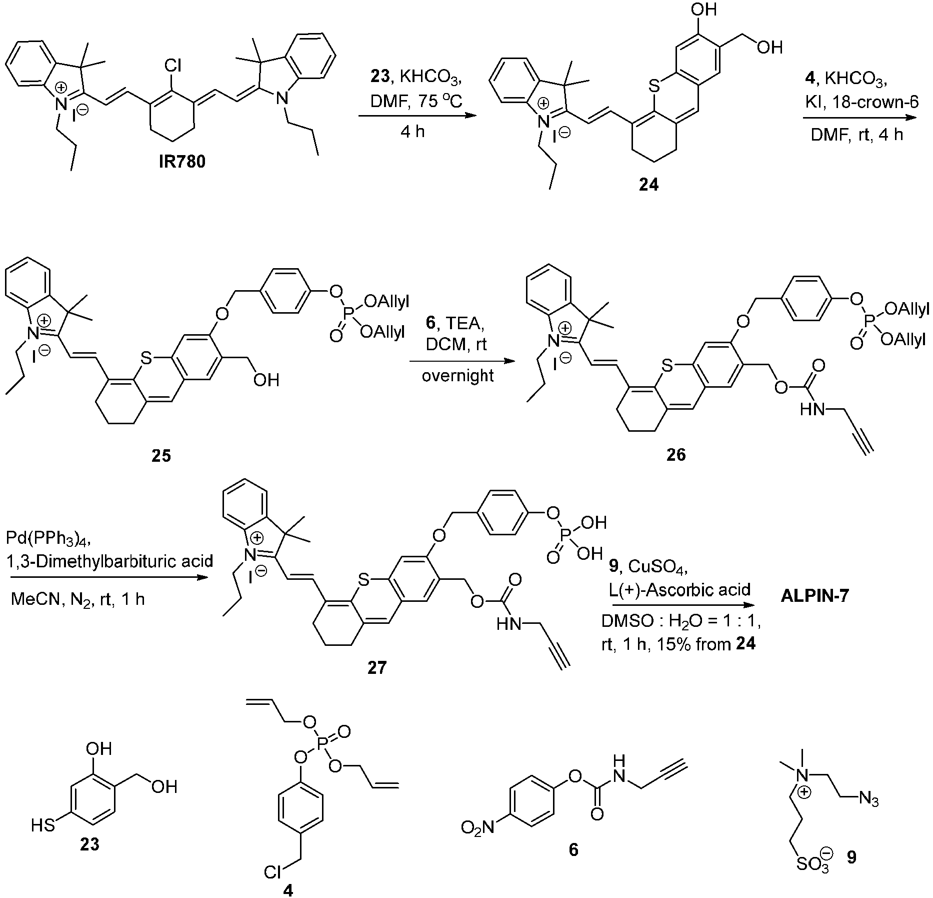

### a. Preparation of Compound 23

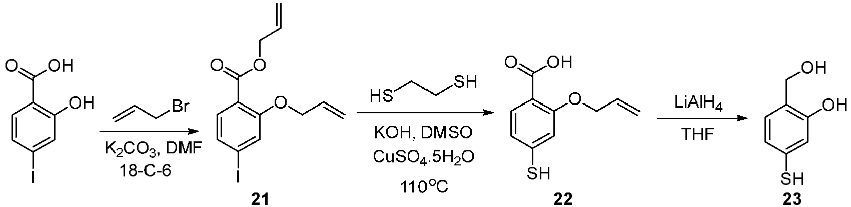

Preparation of Compound **21:** 2-Hydroxy-4-iodobenzoic acid (1.24 g, 4.7 mmol), K₂CO₃(1.98 g, 14.1 mmol), 18-C-6 (127.6 mg, 0.47 mmol), and ultra-dry DMF (5 mL) were added to a 25 mL Schlenk tube, followed by allyl bromide (1.2 mL, 14.1 mmol), and the mixture was stirred at room temperature overnight. After completion of the reaction, the reaction mixture was diluted with ethyl acetate (20 mL) and washed three times with water (20 mL). The organic phase was separated using a separatory funnel, dried over anhydrous sodium sulfate, and filtered under reduced pressure. The residue was mixed with silica gel and purified by column chromatography, followed by concentration under reduced pressure using a rotary evaporator to obtain a white solid (1.256 g) with a yield of 88%. Spectral data: ¹H NMR (400 MHz, CDCl₃) δ 7.53 (d, *J* = 8.2 Hz, 1H), 7.34 (dd, *J* = 8.2, 1.6 Hz, 1H), 7.30 (d, *J* = 1.2 Hz, H), 6.09 - 5.96 (m, 2H), 5.54 (dd, *J* = 17.2, 1.6 Hz, 1H), 5.41 (dd, *J* = 15.6, 1.6 Hz, 1H), 5.32 (dd, *J* = 10.8, 1.6Hz, 1H), 5.27 (dd, *J* = 10.8, 1.6 Hz, 1H), 4.79 (d, *J* = 5.6, 2H), 4.60 (d, *J* = 4.8, 2H).

Preparation of Compound **22:** Compound **21** (1.83 g, 5.32 mmol), 1,2-ethanedithiol (890 µL, 10.64 mmol), CuSO₄·5H₂O (66.4 mg, 0.266 mmol), and KOH (1.5 g, 26.6 mmol) were added to a 25 mL Schlenk tube, which was purged with N₂ three times, followed by addition of DMSO (4 mL) and H₂O (1 mL), and the mixture was stirred at 110°C overnight. After completion of the reaction, the reaction mixture was cooled to room temperature, acidified with 1 M HCl aqueous solution, diluted with ethyl acetate (20 mL), and washed three times with water (20 mL). The organic phase was separated using a separatory funnel, dried over anhydrous sodium sulfate, and filtered under reduced pressure. The residue was mixed with silica gel and purified by column chromatography, followed by concentration under reduced pressure using a rotary evaporator to obtain a white solid (752.6 mg) with a yield of 67%. Spectral data: ¹H NMR (400 MHz, CDCl₃) δ 8.03 (d, *J* = 8.4 Hz, 1H), 6.98 (dd, *J* = 8.4, 1.6 Hz, 1H), 6.89 (d, *J* = 1.6 Hz, 1H), 6.14 - 6.04 (m, 1H), 5.45 - 5.52 (m, 2H), 4.77 (d, *J* = 5.6 Hz, 2H), 3.69 (s, 1H).

Preparation of Compound **23:** Compound **22** (435.2 mg, 2.1 mmol) was added to a 10 mL Schlenk tube, which was purged with N₂ three times, followed by addition of dry THF (4.2 mL). A solution of lithium aluminum hydride in THF (6.3 mL) was then added dropwise under a nitrogen atmosphere, and the mixture was stirred at room temperature overnight. After completion of the reaction, the reaction mixture was slowly added dropwise with water in an ice bath, acidified with 1 M HCl aqueous solution, diluted with ethyl acetate (20 mL), and washed three times with water (20 mL). The organic phase was separated using a separatory funnel, dried over anhydrous sodium sulfate, and filtered under reduced pressure. The residue was concentrated under reduced pressure using a rotary evaporator to obtain the target compound, which was used directly in the next step.

### b. Preparation of Compound 24

IR780 (108.2 mg, 0.16 mmol), Compound **23** (117.1 mg, 0.75 mmol), and KHCO₃ (48.3 mg, 0.48 mmol) were added to a 10 mL Schlenk tube, which was purged with N₂ three times, followed by addition of dry DMF (0.5 mL), and the mixture was stirred at 75°C for 2 hours. After completion of the reaction, the reaction mixture was diluted with ethyl acetate (20 mL) and washed three times with water (20 mL). The organic phase was separated using a separatory funnel, dried over anhydrous sodium sulfate, and filtered under reduced pressure. The residue was mixed with silica gel and purified by column chromatography, followed by concentration under reduced pressure using a rotary evaporator to obtain a blue compound (54.2 mg) with a yield of 62%. Spectral data: ¹H NMR (400 MHz, CDCl₃) δ 8.11 (d, *J* = 13.6 Hz, 1H), 7.58 (s, 1H), 7.52 (d, *J* = 7.6 Hz, 1H), 7.43 - 7.39 (m, 2H), 7.30 - 7.25 (m, 2H), 6.86 (s, 1H), 6.19 (d, *J* = 13.6 Hz, 1H), 4.65 (s, 2H), 4.09 (t, *J* = 7.2 Hz, 2H), 2.75 (t, *J* = 5.2 Hz, 2H), 2.63 (t, *J* = 5.6 Hz, 2H), 1.92 - 1.61 (m, 4H), 1.73 (s, 6H), 1.03 (t, *J* = 7.6 Hz, 3H). ¹³C NMR (101 MHz, CDCl₃) δ 175.83, 158.76, 146.42, 144.86, 144.47, 143.50 (d, *J* = 3.2 Hz), 142.27, 138.62, 134.31, 133.85, 132.40, 128.70, 128.44, 126.03, 124.54, 115.07, 114.57, 103.69, 63.24, 52.94, 49.10, 35.81, 31.57, 29.91, 24.62, 24.28, 14.23.

### c. Preparation of Compound 26

Dye **24** (54.2 mg, 0.076 mmol), KI (31.2 mg, 0.186 mmol), KHCO₃ (19.7 mg, 0.197 mmol), and 18-crown-6 (3.4 mg, 0.013 mmol) were added to a 10 mL reaction tube, which was evacuated and purged with N₂ three times. Under a N₂ atmosphere, ultra-dry DMF (1.0 mL) was added, followed by Compound **4** (87.4 mg, 0.279 mmol), and the mixture was stirred at room temperature for 4 hours. After completion of the reaction, the reaction mixture was diluted with DCM (10.0 mL) and washed with water (5.0 mL). The organic phase was separated using a separatory funnel, dried over anhydrous sodium sulfate, and filtered under reduced pressure. The residue was mixed with silica gel and purified by flash column chromatography, followed by rotary evaporation until dryness to obtain Compound **25** as a blue solid. Compound **25** was added to a 10 mL reaction tube, followed by DCM (1 mL), Compound **6** (42.3 mg, 0.19 mmol), and TEA (37 µL, 0.27 mmol), and the mixture was stirred at room temperature overnight. After completion of the reaction, the reaction mixture was diluted with DCM (10.0 mL) and washed with water (5.0 mL). The organic phase was separated using a separatory funnel, dried over anhydrous sodium sulfate, and filtered under reduced pressure. The residue was mixed with silica gel and purified by column chromatography, followed by rotary evaporation until dryness to obtain a blue solid compound (40.7 mg) with a two-step yield of 57%. Spectral data: ¹H NMR (600 MHz, CDCl₃) δ 9.34 (d, *J* = 14.4 Hz, 1H), 8.55 - 8.53 (m, 2H), 8.49 (t, *J* = 7.2 Hz, 1H), 8.44 - 8.41 (m, 3H), 8.34 (d, *J* = 7.8 Hz, 1H), 8.28 - 8.26 (m, 2H), 8.22 (s, 2H), 7.55 (d, *J* = 14.4 Hz, 1H), 7.00 - 6.93(m, 2H), 6.40 (dd, *J* = 17.4, 1.2 Hz, 2H), 6.29 (dd, *J* = 10.8, 1.2 Hz, 2H), 6.21 (d, *J* = 17.4 Hz, 4H), 5.72 - 5.62 (m, 4H), 5.31 (t, *J* = 7.2 Hz, 2H), 5.01 (s, 3H), 3.77 (t, *J* = 5.6 Hz, 2H), 3.67 (t, *J* = 5.6 Hz, 2H), 3.27 (s, 1H), 2.98 - 2.95 (m, 2H), 2.81 (s, 6H), 2.28 (s, 2H), 2.07 (t, *J* = 7.2 Hz, 3H). ¹³C NMR (151 MHz, CDCl₃) δ 177.29, 160.47, 160.23, 157.51, 145.23, 142.07, 141.41, 136.40, 135.76, 133.06, 132.42, 132.00 (d, *J* = 6.9 Hz), 131.13, 129.20, 128.76, 127.53, 127.07, 126.87, 123.08, 122.70, 120.36 (d, *J* = 4.4 Hz), 117.16, 115.25, 112.53, 107.35, 105.08, 79.64, 71.64, 70.07, 68.96 (d, *J* = 6.0 Hz), 61.49, 50.77, 46.94, 32.47, 30.89, 29.70, 28.43, 26.66, 21.31, 20.24, 14.13, 11.44.

### d. Preparation of Compound ALPIN-7

Compound **26** (25.7 mg, 0.028 mmol), 1,3-dimethylbarbituric acid (14.3 mg, 0.825 mmol), and Pd(PPh₃)₄ (6.5 mg, 0.006 mmol) were added to a 10 mL Schlenk tube, which was evacuated and purged with N₂ three times, followed by addition of MeCN (0.5 mL) and DCM (0.5 mL). After stirring at room temperature for 1 hour, the reaction mixture was concentrated under reduced pressure using a rotary evaporator, followed by addition of Compound **9** (6.5 mg, 0.027 mmol), CuSO₄ (4.4 mg, 0.027 mmol), ascorbic acid (19.4 mg, 0.11 mmol), DMSO (0.5 mL), and H₂O (0.5 mL). The mixture was stirred at room temperature for 1 hour, followed by purification by preparative HPLC to obtain the target compound (4.5 mg) with a three-step yield of 15%. Spectral data: ¹H NMR (600 MHz, *d*₆-DMSO) δ 8.28 (d, *J* = 14.4 Hz, 1H), 8.11 (s, 1H), 7.94 - 7.86 (m, 2H), 7.83 (d, *J* = 7.2 Hz, 1H), 7.80 - 7.75 (m, 4H), 7.62 (d, *J* = 19.8 Hz, 1H), 7.57 (t, *J* = 7.8 Hz, 1H), 7.50 (t, *J* = 7.8 Hz, 1H), 7.48 (d, *J* = 8.4 Hz, 1H), 7.40 (s, 1H), 7.21 (d, *J* = 8.4 Hz, 2H), 6.79 (d, *J* = 15 Hz, 1H), 5.26 (s, 2H), 5.09 (s, 2H), 4.92 (t, *J* = 7.2 Hz, 2H), 4.44 (t, *J* = 7.2 Hz, 2H), 4.27 (d, *J* = 5.4 Hz, 2H), 3.85 (t, *J* = 6.9 Hz, 2H), 3.07 (s, 6H), 2.77 (s, 2H), 2.70 (s, 2H), 2.45 (t, *J* = 6.9 Hz, 2H), 1.99 - 1.94 (m, 2H), 1.87 - 1.81 (m, 4H), 1.77 (s, 6H), 1.23 (s, 2H), 0.98 (t, *J =* 7.4 Hz, 3H).

### Preparation Example 4: Preparation of probes

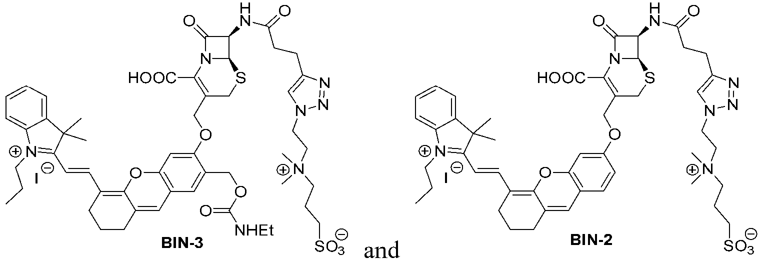

### Example 4.1: Synthesis of probe BIN-3

The synthetic route of the primary probe BIN-3 is as follows:

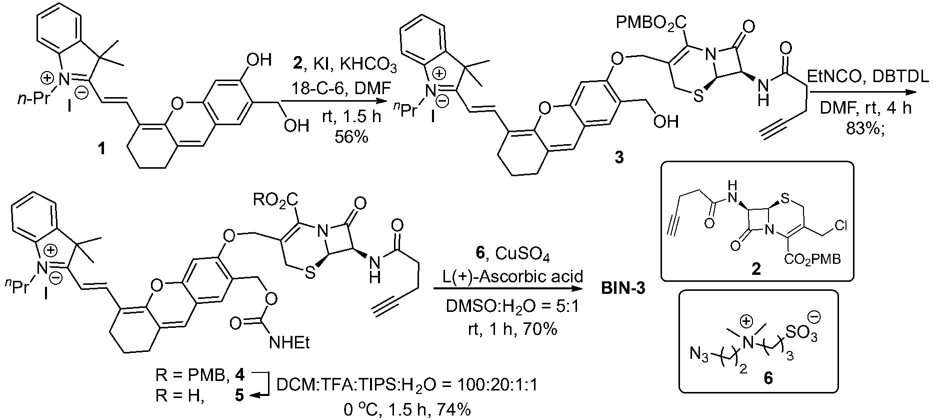

Step 1: Compound **2** (238.7 mg, 0.532 mmol), KI (151.9 mg, 0.915 mmol), and ultra-dry DMF (1.2 mL) were added to a reaction flask, and the mixture was stirred at room temperature for 0.5 hours. KHCO₃ (38.6 mg, 0.386 mmol), 18-crown-6 (24.7 mg, 0.093 mmol), and Compound 1 (103.1 mg, 0.181 mmol) were then added, and the mixture was stirred for 1.5 hours. After completion of the reaction, the reaction mixture was diluted with DCM (20 mL), washed with saturated NaCl solution and water, and extracted three times with DCM (30 mL × 3). The combined DCM extracts were dried over anhydrous Na₂SO₄, concentrated under reduced pressure using a rotary evaporator, and purified by column chromatography to obtain Compound **3** (99.4 mg) as a blue solid, with a yield of 56%. ¹H NMR (600 MHz, CD₃OD) δ 8.75 (d, *J* = 15.0 Hz, 1H), 7.56 (s, 1H), 7.55 - 7.48 (m, 3H), 7.45 (s, 1H), 7.40 (t, *J* = 7.2 Hz, 1H), 7.30 (d, *J* = 8.4 Hz, 2H), 7.23 (s, 1H), 6.79 (d, *J* = 8.4 Hz, 2H), 6.52 (d, *J* = 14.4 Hz, 1H), 5.79 (d, *J* = 4.8 Hz, 1H), 5.36 (d, *J* = 12.0 Hz, 1H), 5.28 - 5.21 (m, 2H), 5.15 (d, *J* = 4.8 Hz, 1H), 4.98 (d, *J* = 12.6 Hz, 1H), 4.68 - 4.60 (m, 2H), 4.33 (t, *J* = 7.2 Hz, 2H), 3.76 - 3.63 (m, 5H), 2.86 - 2.69 (m, 4H), 2.51 - 2.41 (m, 4H), 2.24 (s, 1H), 2.01 - 1.91 (m, 4H), 1.89 - 1.80 (m, 6H), 1.08 (t, *J* = 7.2 Hz, 3H). ¹³C NMR (151 MHz, CD₃OD) δ 177.80, 173.06, 165.13, 162.19, 161.79, 160.03, 158.67, 153.88, 145.41, 142.09, 141.67, 134.02, 130.00, 129.14, 128.78, 127.60, 126.90, 126.80, 126.45, 126.05, 125.39, 122.33, 115.76, 114.23, 113.54, 112.47, 103.18, 99.16, 81.84, 69.02, 67.62, 66.95, 59.42, 58.30, 57.66, 54.32, 50.58, 46.06, 34.03, 28.72, 27.46, 27.07, 25.10, 23.73, 20.85, 20.24, 14.02, 10.19. HRMS (ESI) m/z calcd for C₅₀H₅₂N₃O₈S (M-I)⁺ 854.3470, found 854.3472.

Step 2: Compound **3** (70.9 mg, 0.072 mmol) was added to a 10 mL Schlenk tube, which was purged with nitrogen three times. A solution of Et-NCO (58 µL, 0.723 mmol) and DBTDL (18 µL, 0.029 mmol) in DMF (700 µL) was then added to the reaction tube, and the mixture was stirred at room temperature for 4 hours. After completion of the reaction, the reaction mixture was diluted with DCM (10 mL), washed with saturated NaCl solution and water, and extracted three times with DCM (20 mL × 3). The combined DCM extracts were dried over anhydrous Na₂SO₄, concentrated under reduced pressure using a rotary evaporator, and purified by column chromatography to obtain Compound **4** (63.5 mg) as a blue solid, with a yield of 83%. ¹H NMR (600 MHz, CD₃OD) δ 8.74 (d, *J* = 14.4 Hz, 1H), 7.56 (d, *J* = 8.4 Hz, 1H), 7.54 - 7.47 (m, 3H), 7.42 (t, *J* = 7.2 Hz, 1H), 7.38 (s, 1H), 7.30 (d, *J* = 8.4 Hz, 2H), 7.20 (s, 1H), 6.78 (d, *J* = 8.4 Hz, 2H), 6.55 (d, *J* = 15.0 Hz, 1H), 5.80 (d, *J* = 4.8 Hz, 1H), 5.36 (d, *J* = 12.0 Hz, 1H), 5.28 - 5.20 (m, 2H), 5.17 - 5.06 (m, 3H), 4.96 (d, *J* = 12.6 Hz, 1H), 4.34 (t, *J* = 7.2 Hz, 2H), 3.77 - 3.62 (m, 5H), 3.15 (q, *J* = 7.2 Hz, 2H), 2.85 - 2.71 (m, 4H), 2.50 - 2.41 (m, 4H), 2.24 (s, 1H), 2.01 - 1.92 (m, 4H), 1.90 - 1.79 (m, 6H), 1.12 (t, *J* = 7.2 Hz, 3H), 1.08 (t, *J* = 7.8 Hz, 3H). ¹³C NMR (151 MHz, CD₃OD) δ 178.13, 173.07, 165.14, 162.14, 161.37, 160.02, 158.95, 157.10, 154.23, 145.58, 142.19, 141.62, 133.25, 130.02, 128.83, 127.88, 127.84, 127.10, 126.80, 125.86, 125.51, 124.42, 122.36, 115.65, 114.28, 113.53, 112.65, 103.69, 99.42, 81.85, 69.03, 67.63, 67.08, 60.71, 59.41, 57.65, 54.33, 50.71, 46.17, 35.30, 34.04, 28.75, 27.38, 27.01, 25.21, 23.71, 20.91, 20.20, 14.04, 13.98, 10.19. HRMS (ESI) m/z calcd for C₅₃H₅₇N₄O₉S (M-I)⁺ 925.3841, found 925.3841.

Step 3: Compound **4** (15.2 mg, 0.014 mmol) and DCM (1.0 mL) were sequentially added to a 25 mL reaction flask, which was cooled to 0°C in an ice-water bath. A mixed solution of water (20 µL), triisopropylsilane (20 µL), and TFA (400 µL) in DCM (1.0 mL) was slowly added dropwise to the reaction flask, and the mixture was stirred for 1.5 hours. HPLC analysis indicated complete consumption of the starting material. After completion of the reaction, the reaction mixture was added with 1 M NaHCO₃ solution (7 mL), washed with water, and extracted three times with DCM (10 mL × 3). The combined organic phases were dried over anhydrous Na₂SO₄ and concentrated under reduced pressure using a rotary evaporator. The residue was dissolved in acetonitrile (4.0 mL) and purified using a reverse-phase C18 preparative column. The resulting mixture was concentrated under reduced pressure using a rotary evaporator to remove the acetonitrile, followed by lyophilization to obtain Compound **5** (10.0 mg) as a blue solid, with a yield of 74%. ¹H NMR (600 MHz, CD₃OD) δ 8.78 (d, *J* = 15.0 Hz, 1H), 7.69 (d, *J* = 7.2 Hz, 1H), 7.59 (s, 1H), 7.58 - 7.49 (m, 3H), 7.46 (t, *J* = 7.8 Hz, 1H), 7.36 (s, 1H), 6.54 (d, *J* = 15.0 Hz, 1H), 5.75 (d, *J* = 4.8 Hz, 1H), 5.58 (d, *J* = 13.2 Hz, 1H), 5.22 - 5.04 (m, 4H), 4.34 (t, *J* = 7.2 Hz, 2H), 3.67 (d, *J* = 18.6 Hz, 1H), 3.61 (d, *J* = 18.0 Hz, 1H), 3.15 (q, *J* = 7.2 Hz, 2H), 2.82 - 2.76 (m, 2H), 2.74 - 2.69 (m, 2H), 2.46 - 2.39 (m, 4H), 2.22 (s, 1H), 1.98 - 1.92 (m, 4H), 1.88 - 1.82 (m, 6H), 1.12 (t, *J* = 7.2 Hz, 3H), 1.07 (t, *J* = 7.2 Hz, 3H). HRMS (ESI) m/z calcd for C₄₅H₄₉N₄O₈S (M-I)⁺ 805.3266, found 805.3267.

Step 5: Compound **5** (7.1 mg, 0.008 mmol), Compound **6** (13.2 mg, 0.056 mmol), anhydrous CuSO₄ (15.1 mg, 0.095 mmol), and ascorbic acid (14.9 mg, 0.085 mmol) were dissolved in DMSO (0.5 mL) and H₂O (0.1 mL), and the mixture was stirred at room temperature for 1 hour. After completion of the reaction, the reaction mixture was purified using a reverse-phase C18 preparative column to obtain **BIN-3** (6.2 mg) as a blue solid, with a yield of 70%. HRMS (ESI) m/z calcd for C₅₂H₆₅N₈O₁₁S₂ (M-I)⁺ 1041.4209, found 1041.4219.

### Example 4.2: Synthesis of probe BIN-2

The synthetic route of the control probe **BIN-2** is as follows:

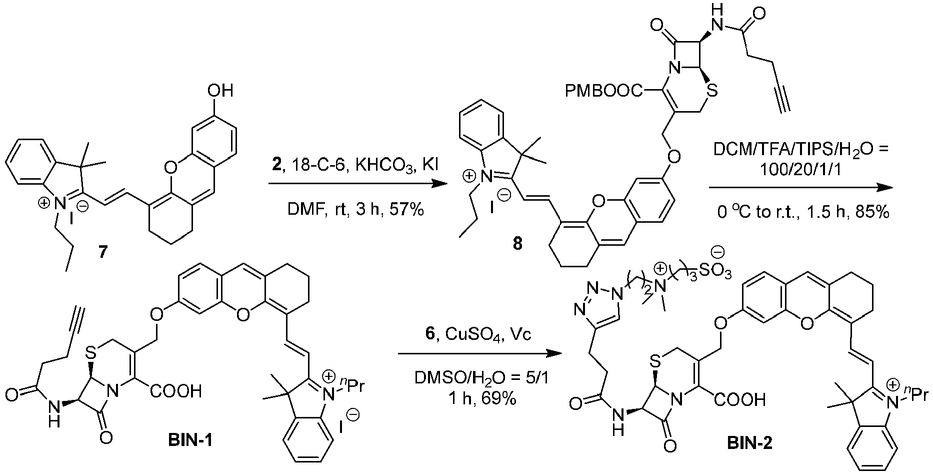

Step 1: Compound **7** (197.2 mg, 0.439 mmol), KI (134.1 mg, 0.878 mmol), and ultra-dry DMF (1.0 mL) were sequentially added to a reaction flask, and the mixture was stirred at room temperature for 0.5 hours. KHCO₃ (31.0 mg, 0.310 mmol), 18-crown-6 (20.0 mg, 0.074 mmol), and Compound **2** (62.4 mg, 0.116 mmol) were then added, and the mixture was stirred for 3 hours. After completion of the reaction, the reaction mixture was diluted with DCM (20 mL), washed with saturated NaCl solution and water, and extracted three times with DCM (30 mL × 3). The combined DCM extracts were dried over anhydrous Na₂SO₄, concentrated under reduced pressure using a rotary evaporator, and purified by column chromatography to obtain Compound **8** (62.2 mg) as a blue solid, with a yield of 57%. ¹H NMR (600 MHz, CD₃OD) δ 8.75 (d, *J* = 15.0 Hz, 1H), 7.60 - 7.49 (m, 3H), 7.47 - 7.40 (m, 2H), 7.38 (s, 1H), 7.29 (d, *J =* 9.0 Hz, 2H), 7.10 (d, *J =* 1.8 Hz, 1H), 6.90 (dd, *J =* 9.0, 2.4 Hz, 1H), 6.79 (d, *J* = 8.4 Hz, 2H), 6.54 (d, *J* = 15.0 Hz, 1H), 5.78 (d, *J* = 4.8 Hz, 1H), 5.30 (d, *J* = 12.0 Hz, 1H), 5.23 (d, *J* = 12.0 Hz, 1H), 5.15 - 5.09 (m, 2H), 4.92 (d, *J* = 12.6 Hz, 1H), 4.34 (t, *J* = 7.8 Hz, 2H), 3.76 - 3.60 (m, 5H), 2.85 - 2.68 (m, 4H), 2.52 - 2.42 (m, 4H), 2.25 (s, 1H), 2.01 - 1.91 (m, 4H), 1.89 - 1.78 (m, 6H), 1.08 (t, *J* = 7.2 Hz, 3H). ¹³C NMR (151 MHz, CD₃OD) δ 178.12, 173.07, 165.07, 162.03, 161.53, 161.47, 160.02, 154.34, 145.71, 142.17, 141.61, 133.41, 130.13, 128.83, 128.68, 127.52, 127.09, 126.88, 126.14, 125.52, 122.38, 116.23, 114.26, 114.05, 113.53, 112.64, 103.61, 100.96, 81.88, 69.06, 67.61, 66.89, 59.41, 57.67, 54.34, 50.69, 46.17, 34.04, 28.71, 27.27, 27.01, 25.39, 23.67, 20.91, 20.24, 14.05, 10.19.

Step 2: Compound **8** (25.2 mg, 0.027 mmol) and DCM (1.0 mL) were sequentially added to a 25 mL reaction flask, which was cooled to 0°C in an ice-water bath. A mixed solution of water (20 µL), triisopropylsilane (20 µL), and TFA (400 µL) in DCM (1.0 mL) was slowly added dropwise to the reaction flask, and the mixture was stirred for 1.5 hours. HPLC analysis indicated complete consumption of the starting material. After completion of the reaction, the reaction mixture was added with 1 M NaHCO₃ solution (7 mL), washed with water, and extracted three times with DCM (10 mL × 3). The combined organic phases were dried over anhydrous Na₂SO₄ and concentrated under reduced pressure using a rotary evaporator. The residue was dissolved in acetonitrile (4.0 mL) and purified using a reverse-phase C18 preparative column. The resulting mixture was concentrated under reduced pressure using a rotary evaporator to remove the acetonitrile, followed by lyophilization to obtain **BIN-1** (18.6 mg) as a blue solid, with a yield of 85%. ¹H NMR (600 MHz, *d*₆-DMSO) δ 8.91 (d, *J* = 8.4 Hz, 1H), 8.61 (d, *J* = 15.0 Hz, 1H), 7.75 (d, *J* = 7.2 Hz, 1H), 7.72 (d, *J* = 7.8 Hz, 1H), 7.59 - 7.52 (m, 2H), 7.52 - 7.45 (m, 2H), 7.29 (d, *J* = 1.8 Hz, 1H), 7.03 (dd, *J* = 8.4, 1.8 Hz, 1H), 6.60 (d, *J* = 15.0 Hz, 1H), 5.74 (dd, *J* = 8.4, 4.8 Hz, 1H), 5.20 - 5.11 (m, 2H), 4.97 (d, *J* = 12.0 Hz, 1H), 4.41 (t, *J* = 7.2 Hz, 2H), 3.71 (d, *J* = 18.6 Hz, 1H), 3.60 (d, *J* = 18.6 Hz, 1H), 2.77 (s, 1H), 2.75 - 2.71 (m, 2H), 2.71 - 2.65 (m, 2H), 2.45 - 2.32 (m, 4H), 1.88 - 1.81 (m, 4H), 1.80 - 1.73 (m, 6H), 0.99 (t, *J* = 7.2 Hz, 3H). ¹³C NMR (151 MHz, *d*₆-DMSO) δ 178.34, 171.66, 165.37, 163.52, 161.46, 160.74, 154.23, 145.41, 142.74, 141.95, 133.39, 129.44, 129.36, 127.67, 127.63, 126.88, 124.56, 123.18, 116.24, 114.40, 114.11, 113.86, 105.14, 102.00, 83.86, 71.95, 67.52, 59.55, 57.94, 50.98, 46.55, 34.06, 28.91, 28.10, 28.01, 25.74, 24.03, 21.43, 20.37, 14.50, 11.48. HRMS (ESI) m/z calcd for C₄₁H₄₂N₃O₆S (M-I)⁺ 704.2789, found 704.2798.

Step 3: Compound **BIN-1** (9.1 mg, 0.011 mmol), Compound **6** (11.3 mg, 0.048 mmol), anhydrous CuSO₄ (11.6 mg, 0.073 mmol), and ascorbic acid (13.2 mg, 0.075 mmol) were dissolved in DMSO (0.5 mL) and H₂O (0.1 mL), and the mixture was stirred at room temperature for 1 hour. After completion of the reaction, the reaction mixture was purified using a reverse-phase C18 preparative column to obtain **BIN-2** (8.0 mg) as a blue solid, with a yield of 69%. ¹H NMR (600 MHz, *d*₆-DMSO) δ 8.92 (d, *J* = 8.4 Hz, 1H), 8.61 (d, *J* = 15.0 Hz, 1H), 7.96 (d, *J* = 6.6 Hz, 1H), 7.75 (d, *J* = 7.2 Hz, 1H), 7.72 (d, *J* = 8.4 Hz, 1H), 7.58 - 7.53 (m, 2H), 7.51 - 7.45 (m, 2H), 7.29 (d, *J* = 2.4 Hz, 1H), 7.03 (dd, *J* = 8.4, 1.8 Hz, 1H), 6.60 (d, *J=* 15.0 Hz, 1H), 5.73 (dd, J = 7.8, 4.8 Hz, 1H), 5.19 - 5.11 (m, 2H), 4.96 (d, *J=* 12.0 Hz, 1H), 4.90 (t, *J=* 6.6 Hz, 2H), 4.41 (t, *J =* 7.2 Hz, 2H), 3.85 (t, *J =* 7.2 Hz, 2H), 3.72 (d, *J =* 18.6 Hz, 1H), 3.61 (d, *J=* 18.0 Hz, 1H), 3.54 - 3.51 (m, 2H), 3.06 (s, 6H), 2.90 - 2.87 (m, 2H), 2.75 - 2.72 (m, 2H), 2.71 - 2.66 (m, 2H), 2.58 - 2.54 (m, 2H), 2.47 (t, *J =* 6.6 Hz, 2H), 2.04 - 1.96 (m, 2H), 1.88 - 1.80 (m, 4H), 1.80 - 1.74 (m, 6H), 0.99 (t, *J* = 7.2 Hz, 3H). HRMS (ESI) m/z calcd for C₄₈H₅₈N₇O₉S₂ (M-I)⁺ 940.3732, found 940.3738.

### Example 5: Preparation of photosensitizers

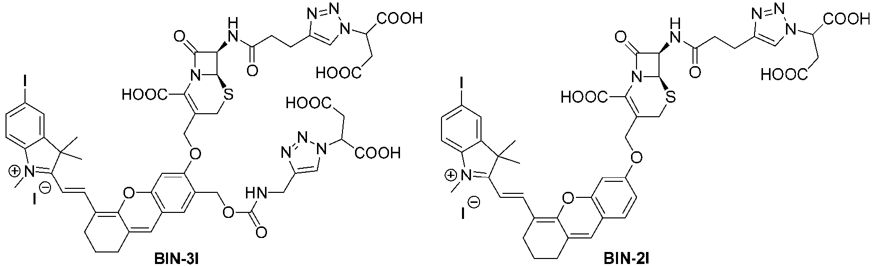

### Example 5.1: Synthesis of photosensitizer BIN-3I

The synthetic method of photosensitizer **BIN-3I** is as follows:

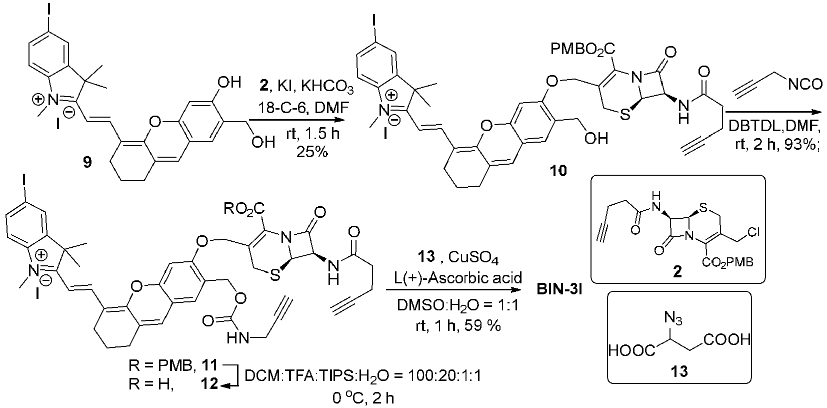

Step 1: Compound **2** (40 mg, 0.060 mmol), KI (108 mg, 0.651 mmol), and ultra-dry DMF (0.4 mL) were added to a reaction flask, and the mixture was stirred at room temperature for 0.5 hours. KHCO₃ (32 mg, 0.032 mmol), 18-crown-6 (16 mg, 0.060 mmol), and Compound **9** (84 mg, 0.187 mmol) were then added, and the mixture was stirred for 1.5 hours. After completion of the reaction, the reaction mixture was diluted with DCM (20 mL), washed with saturated NaCl solution and water, and extracted three times with DCM (30 mL × 3). The combined DCM extracts were dried over anhydrous Na₂SO₄, concentrated under reduced pressure using a rotary evaporator, and purified by column chromatography to obtain Compound **10** (16.4 mg) as a blue solid, with a yield of 25%. ¹H NMR (400 MHz, DMSO-*d*₆) δ = 8.93 (d, *J* = 8.5, 1H), 8.54 (d, *J* = 14.8, 1H), 8.02 (d, *J* = 1.7, 1H), 7.87 (dd, *J* = 8.3, 1.7, 1H), 7.69 (s, 1H), 7.62 (s, 1H), 7.44 (d, *J* = 8.3, 1H), 7.30 (d, *J* = 8.6, 2H), 7.16 (s, 1H), 6.81 (d, *J* = 8.6, 2H), 6.46 (d, *J* = 14.8, 1H), 5.79 (dd, *J* = 8.4, 4.8, 1H), 5.29 - 5.20 (m, 2H), 5.19 (d, *J* = 4.9, 1H), 5.03 (d, *J* = 12.1, 1H), 4.94 (d, *J* = 12.2, 1H), 4.51 (s, 2H), 3.81 (s, 3H), 3.74 - 3.63 (m, 5H), 2.80 - 2.73 (m, 2H), 2.69 (d, *J* = 12.2, 2H), 2.40 - 2.37 (m, 4H), 2.34 - 2.33 (m, 1H), 1.85 (s, 2H), 1.75 (d, *J* = 5.6, 6H).

Step 2: Compound **10** (2 mg, 0.002 mmol) was added to a reaction flask, followed by a solution of 3-isocyanatoprop-1-yne (1.5 mg, 0.018 mmol) and DBTDL (1.3 mg, 0.002 mmol) in DMF (100 µL), and the mixture was stirred at room temperature for 2 hours. After completion of the reaction, the reaction mixture was diluted with DCM (2 mL), washed with saturated NaCl solution and water, and extracted three times with DCM (2 mL × 3). The combined DCM extracts were dried over anhydrous Na₂SO₄, concentrated under reduced pressure using a rotary evaporator, and purified by column chromatography to obtain Compound **11** (2 mg) as a blue solid, with a yield of 93%. ¹H NMR (400 MHz, DMSO-*d*₆) δ = 8.94 (d, *J* = 8.5, 1H), 8.54 (d, *J* = 14.9, 1H), 8.04 (d, *J* = 1.6, 1H), 7.89 (dd, *J* = 8.4, 1.6, 1H), 7.77 (t, *J* = 5.7, 1H), 7.54 (d, *J* = 5.7, 2H), 7.47 (d, *J* = 8.5, 1H), 7.30 (d, *J* = 8.6, 2H), 7.18 (s, 1H), 6.80 (d, *J* = 8.6, 2H), 6.52 (d, *J* = 15.0, 1H), 5.80 (dd, *J* = 8.5, 4.9, 1H), 5.26 (q, *J* = 12.0, 2H), 5.18 (d, *J* = 4.9, 1H), 5.05 (d, *J* = 7.0, 1H), 4.93 (d, *J* = 12.1, 1H), 3.84 (s, 3H), 3.82 - 3.77 (m, 2H), 3.73 - 3.63(m, 5H), 3.12 (d, *J* = 2.7, 1H), 2.77 - 2.73 (m, 2H), 2.72 - 2.66 (m, 2H), 2.40 - 2.37 (m, 4H), 2.18 (t, *J* = 7.3, 1H), 1.83 (d, *J* = 6.3, 2H), 1.76 (d, *J* = 4.8, 6H), 1.58 - 1.45 (m, 2H).

Step 3: Compound **11** (2 mg, 0.002 mmol) was added to a 25 mL reaction flask, which was cooled to 0°C in an ice-water bath. A mixed solution of water (10 µL), triisopropylsilane (10 µL), and TFA (200 µL) in DCM (1.0 mL) was slowly added dropwise to the reaction flask, and the mixture was stirred for 2 hours. HPLC analysis indicated complete consumption of the starting material. After completion of the reaction, the reaction mixture was added with 1 M NaHCO₃ solution (5 mL), washed with water, and extracted three times with DCM (10 mL × 3). The combined organic phases were dried over anhydrous Na₂SO₄ and concentrated under reduced pressure using a rotary evaporator without purification to obtain Compound **12** as a crude product.

Step 4: Compound **12** (2 mg, 0.002 mmol), Compound **13** (1 mg, 0.006 mmol), anhydrous CuSO₄ (0.3 mg, 0.002 mmol), and ascorbic acid (1.4 mg, 0.008 mmol) were dissolved in DMSO (0.1 mL) and H₂O (0.1 mL), and the mixture was stirred at room temperature for 1 hour. After completion of the reaction, the reaction mixture was purified using a reverse-phase C18 preparative column to obtain **BIN-3I** (1 mg) as a blue solid, with a yield of 59%. ¹H NMR (400 MHz, DMSO-*d*₆) δ = 8.92 (d, *J* = 8.4, 1H), 8.55 (d, *J* = 14.8, 1H), 8.11 (d, *J =* 1.7, 1H), 8.05 (s, 1H), 7.92 (s, 1H), 7.91 - 7.85 (m, 2H), 7.55 (s, 1H), 7.53 (s, 1H), 7.49 (d, *J* = 2.6, 1H), 6.50 (d, *J* = 14.9, 1H), 5.73 (dd, *J* = 8.4, 4.8, 1H), 5.68 (t, *J* = 6.9, 1H), 5.59 (dd, *J* = 7.7, 6.1, 1H), 5.36 (d, *J* = 12.9, 1H), 5.15 (d, *J* = 4.9, 1H), 5.10 (d, *J* = 3.9, 1H), 5.02 (d, *J* = 13.0, 1H), 4.28 (d, *J* = 5.9, 2H), 3.83 (s, 3H), 3.63 (d, *J* = 12.3, 2H), 3.20 (s, 1H), 3.16 (s, 1H), 2.86 - 2.80 (m, 2H), 2.72 (d, *J* = 3.9, 2H), 2.68 - 2.66 (m, 2H), 2.54 (s, 4H), 2.33 (p, *J* = 1.8, 2H), 1.82 (s, 2H), 1.78 - 1.71 (m, 6H).

### Example 5.2: Synthesis of photosensitizer BIN-2I

The synthetic route of the control photosensitizer **BIN-2I** is as follows:

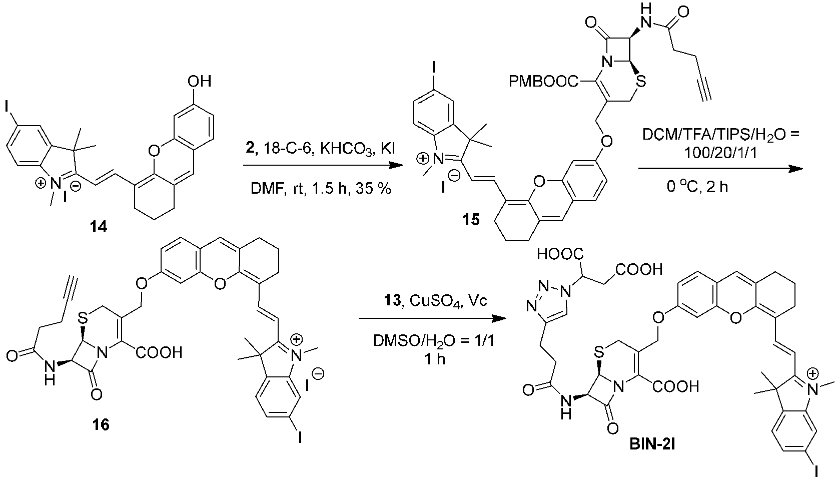

Step 1: Compound **2** (10 mg, 0.016 mmol), KI (35 mg, 0.210 mmol), and ultra-dry DMF (0.4 mL) were added to a reaction flask, and the mixture was stirred at room temperature for 0.5 hours. KHCO₃ (7.7 mg, 0.077 mmol), 18-crown-6 (4 mg, 0.016 mmol), and Compound **14** (24.6 mg, 0.055 mmol) were then added, and the mixture was stirred for 1.5 hours. After completion of the reaction, the reaction mixture was diluted with DCM (20 mL), washed with saturated NaCl solution and water, and extracted three times with DCM (30 mL × 3). The combined DCM extracts were dried over anhydrous Na₂SO₄, concentrated under reduced pressure using a rotary evaporator, and purified by column chromatography to obtain Compound **15** (5.8 mg) as a blue solid, with a yield of 35%.

Step 2: Compound **15** (2 mg, 0.002 mmol) was added to a 25 mL reaction flask, which was cooled to 0°C in an ice-water bath. A mixed solution of water (10 µL), triisopropylsilane (10 µL), and TFA (200 µL) in DCM (1.0 mL) was slowly added dropwise to the reaction flask, and the mixture was stirred for 2 hours. HPLC analysis indicated complete consumption of the starting material. After completion of the reaction, the reaction mixture was added with 1 M NaHCO₃ solution (5 mL), washed with water, and extracted three times with DCM (10 mL × 3). The combined organic phases were dried over anhydrous Na₂SO₄ and concentrated under reduced pressure using a rotary evaporator without purification to obtain Compound **16** as a crude product.

Step 3: Compound **16** (2 mg, 0.002 mmol), Compound **13** (1 mg, 0.006 mmol), anhydrous CuSO₄ (0.3 mg, 0.002 mmol), and ascorbic acid (1.4 mg, 0.008 mmol) were dissolved in DMSO (0.1 mL) and H₂O (0.1 mL), and the mixture was stirred at room temperature for 1 hour. After completion of the reaction, the reaction mixture was purified using a reverse-phase C18 preparative column to obtain **BIN-2I** as a blue solid. Mass spectrometry data for **BIN-2I:** UPLC-MS (EI), calcd for C₄₃H₄₂IN₆O₁₀S⁺ (M-I)⁺ 961.17, found 961.17.

### Test Example 1: Verification of solution-phase enzymatic response of near-infrared fluorescent probe ALPIN-6

### a. Absorption and fluorescence measurement conditions for near-infrared fluorescent probe

The absorption spectra of probes were measured using a UV-Vis spectrophotometer over a wavelength range of 400 to 800 nm. The fluorescence spectra were measured using a fluorescence spectrometer with an excitation wavelength of 687 nm, excitation and emission slit widths of 3 nm, and an emission scanning range of 697 to 850 nm. The temporal changes in fluorescence intensity were measured using a microplate reader (SpectraMax i3). Measurements were performed in a 96-well plate with a default total volume of 100 µL per well, an excitation wavelength of 687 nm, an emission wavelength of 714 nm, and excitation and emission slit widths of 15 nm. The PMT was set to the default value of the microplate reader, with 10 flashes per read. Unless otherwise specified, all fluorescence and absorption measurements were carried out in PBS (1×, pH 7.4) containing 0.1% CHAPS (surfactant; 3-[(3-cholamidopropyl)dimethylammonio]-1-propanesulfonate). The concentration of probe **ALPIN-6** was 10 µM, the final concentration of ALP was 2 U/mL, and the final concentration of β-mercaptoethanol was 140 mM. Samples were incubated in a water bath at 37°C for 30 minutes.

### b. HPLC analysis of changes in near-infrared probe before and after enzymatic hydrolysis

Following incubation of probe **ALPIN-6** (10 µM) with ALP (2 U/mL) in PBS in a water bath at 37°C for 30 minutes, the reaction mixture was centrifuged at 12,000 r/min for 10 minutes using a 10 kDa ultrafiltration membrane to remove proteins. A 100 µL aliquot of the filtrate was analyzed by HPLC at a wavelength of 600 nm.

### c. Gel imaging of covalent protein labeling by near-infrared probe

According to experimental requirements, gel electrophoresis samples with a total volume of 25 µL were prepared as follows: Fluorescent probe **ALPIN-6** or **ALPIN-5** (10 µM) was incubated with different protein samples (ALP, 8 µM; BSA, 5 µM) at 37°C for 2 hours. Subsequently, 2 µL of gel electrophoresis loading buffer (5×) was added and mixed well for later use. An 8% polyacrylamide gel was prepared according to the Beyotime kit instructions, and 20 µL of each sample was loaded into individual wells. SDS-PAGE electrophoresis was carried out at 120 V for 2 to 3 hours. Following electrophoresis, the resulting gel was scanned using an Odyssey CLx Image Studio dual-color near-infrared imaging system (λ_{ex/em} = 685 nm/720 nm). The resulting fluorescence images were analyzed for fluorescent protein bands. Subsequently, the protein gel was stained with Coomassie Brilliant Blue solution for 30 minutes on a shaker to visualize protein bands. Excess dye was then removed using Coomassie Brilliant Blue destaining solution (ethanol/acetic acid/water = 1:2:7), and the gel was photographed using a mobile phone.

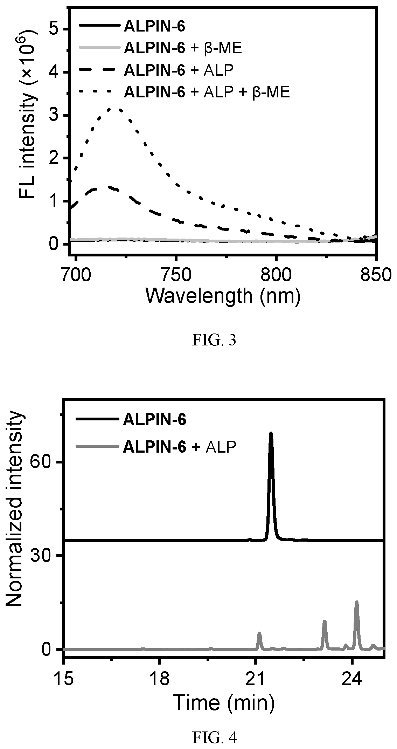

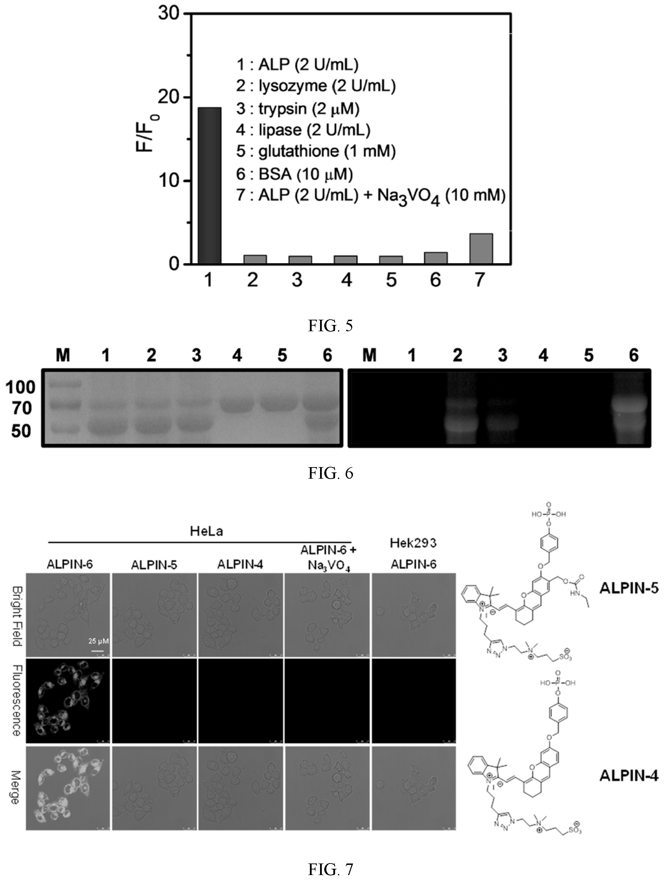

The results are shown in FIGs. 2 to 6. As shown in FIGs. 2 to 6, the fluorescent probe **ALPIN-6** of the present disclosure, which possess a "one-trigger, three-response" capability, exhibited a red-shift in absorption and an enhancement in fluorescence intensity after reaction with ALP. Furthermore, the changes in absorption and fluorescence were more pronounced in the presence of a sufficient amount of nucleophile β-ME (FIGs 2 and 3). HPLC analysis further confirmed that **ALPIN-6** underwent enzymatic conversion in the presence of ALP (FIG. 5). Specificity studies showed that significant fluorescence enhancement was observed only in the probe + ALP group (FIG. 5). Protein-labeling experiments revealed that **ALPIN-6** exhibited the strongest protein-labeling performance following enzymatic activation (FIG. 6). (In FIG. 6, M: protein marker; 1: ALP; 2: ALP + **ALPIN-6;** 3: ALP + **ALPIN-5;** 4: BSA; 5: BSA + **ALPIN-6;** 6: ALP + BSA + **ALPIN-6).**

Experimental conclusion: The fluorescent probe **ALPIN-6** can specifically respond to ALP and exhibits excellent protein-labeling performance following enzymatic activation.

### Test Example 2: Biological validation of near-infrared probe ALPIN-6

### Test Example 2.1: Comparative imaging of HeLa cells overexpressing alkaline phosphatase (ALP) and HEK293 cells weakly expressing ALP using near-infrared fluorescent probes ALPIN-6, ALPIN-5, and ALPIN-4 (FIG. 7)

Experimental conditions: 1 µM of the fluorescent probes **ALPIN-6, ALPIN-5, and ALPIN-4** were incubated separately with HeLa cells overexpressing ALP on the cell surface and normal HEK293 cells with low ALP expression in a culture dish at 37°C for 30 minutes. Alternatively, HeLa cells were pre-treated with the alkaline phosphatase inhibitor Na₃VO₄ for 1 hour, followed by incubation with the probes for 30 minutes. After incubation, the probe-containing culture medium was discarded, and the cells were washed once with PBS (pH = 7.4). Subsequently, 1 mL of phenol red-free DMEM was added, and fluorescence imaging was performed using a confocal fluorescence microscope (Cy 5.5 channel).

The experimental results are shown in FIG. 7. As shown in FIG. 7, compared with the conventional self-anchoring probe **ALPIN-5** (structure shown in FIG. 7) and the conventional fluorescent probe **ALPIN-4** (structure shown in FIG. 7), the fluorescent probe **ALPIN-6** of the present disclosure, which possess a "one-trigger, three-response" capability, not only enabled more pronounced fluorescence imaging of ALP-overexpressing cells, but also exhibited more significant fluorescence signals in ALP-overexpressing cells than in HEK293 cells with low ALP expression. Furthermore, in the ALP inhibitor group, fluorescence labeling of HeLa cells by **ALPIN-6** was reduced, indicating that probe activation depends on the specific hydrolysis of phosphate ester bonds by alkaline phosphatase.

Experimental conclusion: Following reaction with intracellular ALP, the fluorescent probe **ALPIN-6** exhibits enhanced cellular accumulation, reduces probe diffusion during cellular imaging, lowers background fluorescence, and greatly improves detection sensitivity.

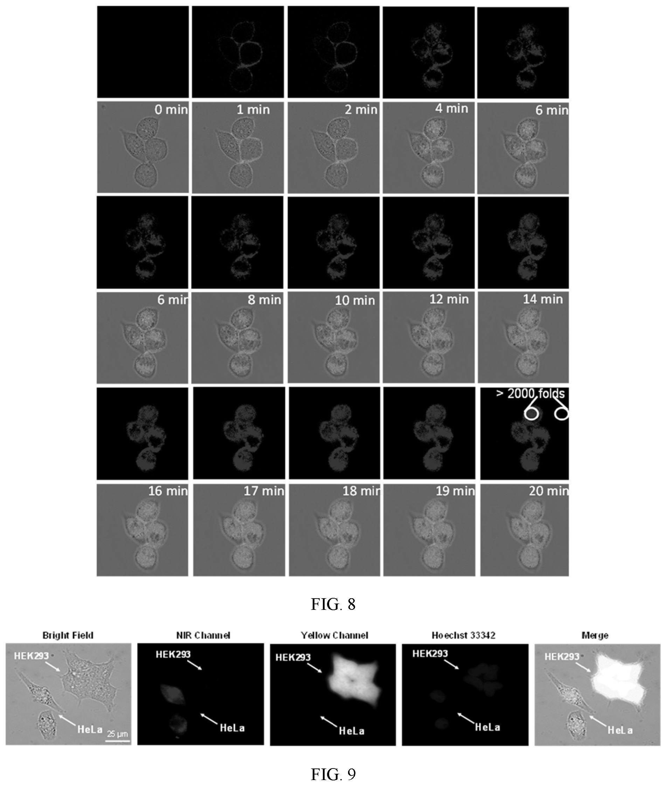

### Test Example 2.2: Real-time imaging of HeLa cells using near-infrared fluorescent probe ALPIN-6 (FIG. 8)

Experimental conditions: Cultured HeLa cells were seeded in a confocal dish, and 0.1 µM of the fluorescent probe **ALPIN-6** was added to the HeLa cells. Time-dependent fluorescence imaging was performed using a confocal fluorescence microscope (Cy 5.5 channel).

The experimental results are shown in FIG. 8. As shown in FIG. 8, during real-time imaging of HeLa cells, the fluorescent probe **ALPIN-6,** which possess a "one-trigger, three-response" capability, initially generated fluorescence signals rapidly on the cell membrane, consistent with the membrane localization of ALP in HeLa cells. As time progressed, fluorescence gradually appeared within the cells, and intracellular fluorescence intensity continuously increased over time. At 20 minutes, the fluorescence intensity associated with the cells was more than 2000-fold higher than the background fluorescence.

Experimental conclusion: The fluorescent probe **ALPIN-6** enables real-time visualization of ALP activity in HeLa cells. Upon enzymatic activation, the probe undergoes a hydrophilic-to-lipophilic transformation that enhances cellular uptake, while the quinone methide-mediated self-anchoring process limits the permeation and diffusion of the activated fluorescence signals, thereby greatly enhancing the accumulation of fluorescence signals within tumor cells and improving the capability of the probe for real-time visualization of enzyme activity.

### Test Example 2.3: Specific imaging of mixed HeLa and HEK293 cells using near-infrared fluorescent probe ALPIN-6 (FIG. 9)

Experimental conditions: Cultured HeLa and HEK293 cells (stably expressing green fluorescent protein) were seeded in a confocal dish, and 1 µM of the fluorescent probe **ALPIN-**6 was added to the mixed cells, followed by incubation at 37°C for 20 minutes. Fluorescence imaging was then performed using a fluorescence microscope.

The experimental results are shown in FIG. 9. As shown in FIG. 9, HEK293 cells stably expressing yellow fluorescent protein exhibited only yellow fluorescence and no red fluorescence, while red fluorescence was observed only in HeLa cells without green fluorescence.

Experimental conclusion: After mixing HeLa cells overexpressing ALP and HEK293 cells with low ALP expression, the fluorescent probe **ALPIN-6** enables specific imaging of the ALP-overexpressing HeLa cells. The specificity results from the "one-trigger, three-response" design strategy, which enables substantial accumulation of the activated probe at the detection site.

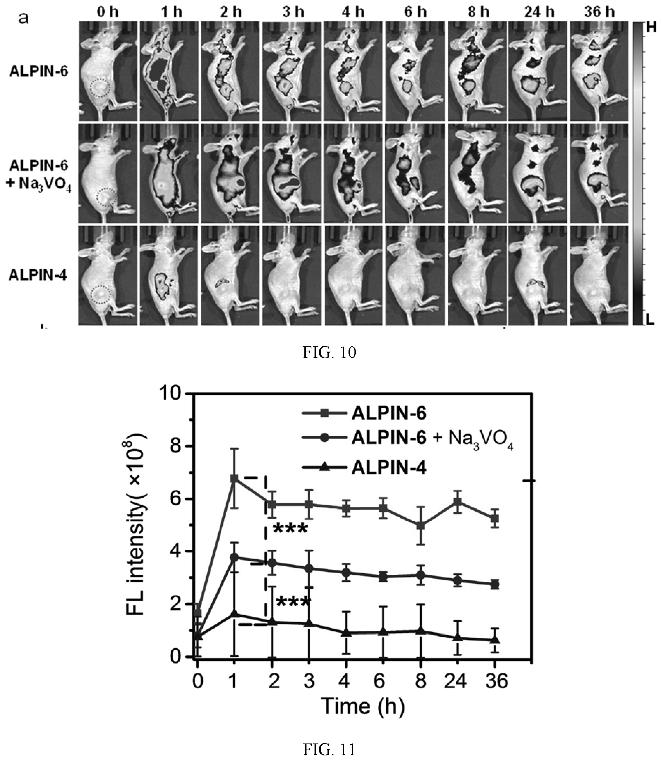

### Test Example 2.4: Real-time imaging of HeLa tumor-bearing mice using near-infrared fluorescent probe ALPIN-6 (FIGs. 10 to 13)

### Experimental conditions:

Establishment of HeLa tumor xenograft model in nude mice:
(1) Preparation of HeLa cells: Cells were cultured and passaged as described above. The cultured cells were centrifuged following digestion, and washed twice with PBS (1 mL per wash). After centrifugation and removal of the supernatant, the cells were resuspended in FBS-free DMEM, counted, mixed with Matrigel, and placed in an ice bath for later use.
(2) Inoculation of HeLa cells into tumor-bearing nude mice: Female BALB/c nude mice aged 4 to 6 weeks were subcutaneously inoculated in the right hind limb with 200 µL of a mixture containing HeLa cells (4 × 10⁶) in DMEM (without FBS) and Matrigel (1:4), using a 1 mL sterile medical syringe. The mice were maintained at 25°C for 2 to 3 weeks with free access to water and food. When the tumor volume reached approximately 0.1 cm³, animal imaging experiments were performed.
(3) *In vivo* imaging of HeLa tumor-bearing nude mice using probes via tail vein injection: The experimental mice were first divided into three groups with three mice per group. Whole-body background fluorescence images were acquired from six nude mice using an IVIS Lumina XRMS Series **III** *in vivo* imaging system (λₑₓ = 660 nm, λₑₘ = 710 nm). The fluorescent probe **ALPIN-6** or **ALPIN-4** was then prepared as a 40 µM solution in PBS. Each tumor-bearing mouse received 200 µL of the prepared probe solution via tail vein injection using an insulin syringe. Subsequently, whole-body fluorescence data from the experimental mice were collected using the *in vivo* imaging system at multiple time points (1 h, 2 h, 3 h, 4 h, 6 h, 8 h, 24 h, and 36 h). For the inhibitor group, 50 µL of 10 mM Na₃VO₄ solution was first injected intratumorally, and 30 minutes later, **ALPIN-6** was administered at the same concentration, followed by imaging at the same time points.
(4) *Ex vivo* imaging of tissues from HeLa tumor-bearing nude mice: In the tail vein injection group, at 36 hours post-injection, the nude mice were sacrificed by cervical dislocation. The tumors, heart, lungs, liver, spleen, and kidneys were harvested and subjected to fluorescence imaging.

The experimental results are shown in FIGs. 10 to 13 and Table 2. As shown in FIGs. 10 to 13, following intravenous administration, the mice injected with **ALPIN-6** exhibited strong fluorescence signals at the tumor site at 1 hour post-injection, and significant tumor-associated fluorescence remained detectable up to 36 hours. In contrast, the mice pre-injected with the inhibitor exhibited significantly reduced fluorescence signals at the tumor site. The control probe **ALPIN-4** also produced detectable fluorescence at 1 hour, but the fluorescence was significantly weaker than that observed with **ALPIN-6** and disappeared from the tumor site after 2 hours. The results were also consistent with the fluorescence signals observed at the tumor site after dissection of the tumor-bearing mice.

**Table 2**

| | Fluorescence intensity at different time points (× 10⁸) | | | | | | | | |
|---|---|---|---|---|---|---|---|---|---|
| | 0 (h) | 1 (h) | 2 (h) | 3 (h) | 4 (h) | 6 (h) | 8 (h) | 24 (h) | 36 (h) |
| ALPIN-6 | 1.65 ± 0.37 | 6.78 ± 1.13 | 5.78 ± 0.50 | 5.79 ± 0.55 | 5.64 ± 0.31 | 5.65 ± 0.39 | 4.98 ± 0.72 | 5.89 ± 0.42 | 5.25 ± 0.34 |
| ALPIN-6 + Na₃VO₄ | 0.80 ± 0.44 | 3.76 ± 0.57 | 3.56 ± 0.46 | 3.34 ± 0.69 | 3.19 ± 0.33 | 3.03 ± 0.17 | 3.09 ± 0.36 | 2.90 ± 0.23 | 2.75 ± 0.17 |
| ALPIN-4 | 0.77 ± 0.75 | 1.61 ± 1.59 | 1.32 ± 1.34 | 1.25 ± 1.36 | 0.91 ± 0.80 | 0.94 ± 0.97 | 0.98 ± 1.01 | 0.72 ± 0.64 | 0.63 ± 0.45 |

Experimental conclusion: In a highly dynamic *in vivo* system, **ALPIN-6** demonstrates superior tumor-targeting accumulation and signal retention, enhancing tumor-associated fluorescence intensity *in vivo,* which facilitates the detection of tumor tissues *in vivo* and plays a crucial role in surgical navigation for tumor resection.

### Test Example 3: Biological validation of near-infrared photosensitizer ALPIN-6I

### Test Example 3.1: Quantification of enrichment of near-infrared photosensitizer ALPIN-6I in HeLa cells

Experimental conditions: 1.5 mL of the photosensitizer **ALPIN-6I** (4 µM) was incubated with HeLa cells (1 × 10⁵ cells) overexpressing ALP on the cell surface at 37°C for 1 hour. After incubation, the incubation medium was collected. The cells were washed twice with phenol red-free DMEM, and the wash buffer was collected. Subsequently, 200 µL of RIPA lysis buffer was added to fully lyse the cells, and the lysate was collected. ALP and BSA were added to the collected incubation medium and wash buffer to ensure complete activation of residual probes. Fluorescence intensity was measured using a fluorometer, with three parallel replicates. Probe concentration was calculated based on the standard calibration curve. Photosensitizer concentration in the cell lysate was determined using the same method. The concentration within the cells was then calculated based on the cell volume (2.6 × 10³ µm³ = 2.6 × 10⁻¹² L per cell).

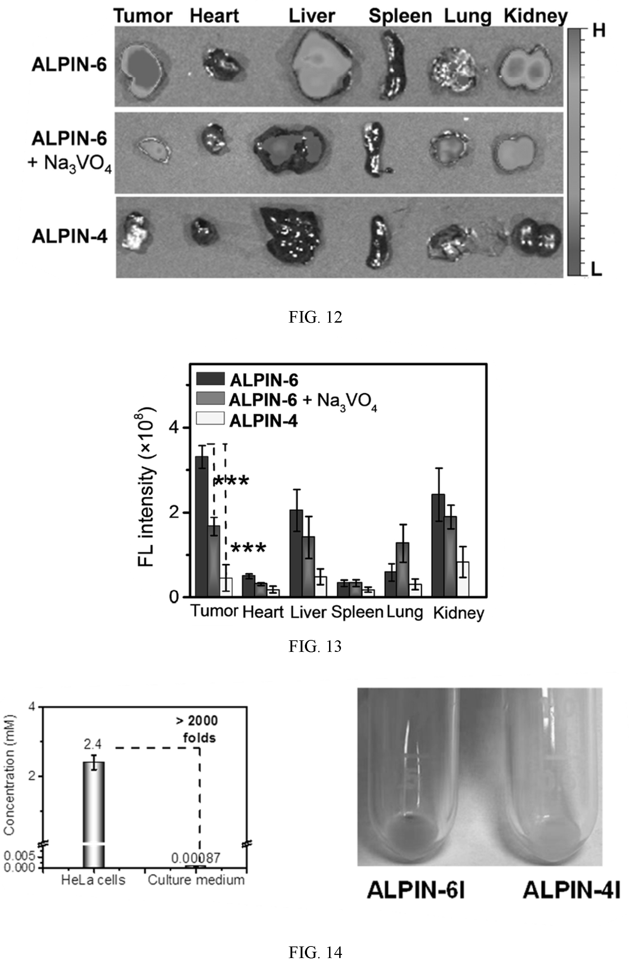

The experimental results are shown in FIG. 14. As shown in FIG. 14, the photosensitizer concentration in HeLa cells was as high as 2.4 mM, while the total concentration in the incubation medium and wash buffer was only 0.00087 mM. The fluorescence intensity within the cells was more than 2000-fold higher than that in the incubation medium. Furthermore, after incubation of the cells with the photosensitizer followed by centrifugation, the cell pellet exhibited distinct blue fluorescence for **ALPIN-6I,** whereas no blue fluorescence was observed for the control photosensitizer **ALPIN-4I.**

Experimental conclusion: The photosensitizer **ALPIN-6I** exhibits extremely high enrichment in ALP-overexpressing HeLa cells, which is highly beneficial for improving the photodynamic therapeutic efficacy of photosensitizers.

### Test Example 3.2: ROS production by photosensitizer ALPIN-6I in HeLa cells

Experimental conditions: HeLa cells or HEK293 cells were incubated with the photosensitizer **ALPIN-6I** (0, 1, 2, 4 µM) or **ALPIN-4I** (4 µM) and the ROS probe **H₂DCFDA** (10 µM) at 37°C for 30 minutes. After washing twice with PBS, the cells were irradiated with a 660 nm light source at 36 mW for 2 minutes, and fluorescence imaging was performed using a fluorescence microscope.

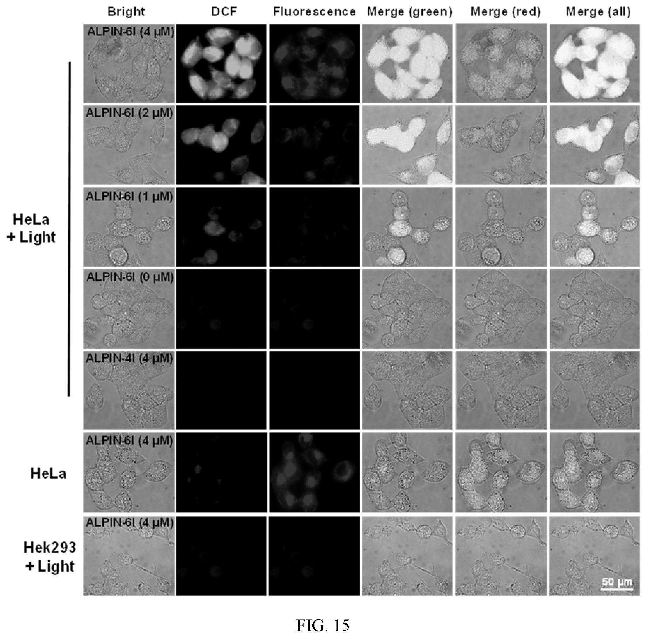

The experimental results are shown in FIG. 15. As shown in FIG. 15, the green fluorescence of DCF increased with increasing concentration of **ALPIN-6I,** indicating elevated ROS generation. In the group treated with **ALPIN-6I,** no green fluorescence was observed in the absence of light irradiation. Similarly, no green fluorescence was observed in HEK293 cells (negative control). Furthermore, under the same conditions, the control photosensitizer **ALPIN-4I** generated significantly weaker green fluorescence than **ALPIN-6I.**

Experimental conclusion: The photosensitizer **ALPIN-6I** can be specifically activated in ALP-overexpressing HeLa cells, but cannot be activated in negative cells, and can generate ROS only under light irradiation. Moreover, the efficiency of ROS generation is significantly higher than that of the control photosensitizer **ALPIN-4I,** owing to the enrichment of ALPIN-6I in tumor cells.

### Test Example 3.3: Killing of tumor cells by photosensitizer ALPIN-6I

Experimental conditions: HeLa cells or HEK293 cells were incubated with the photosensitizer **ALPIN-6I** or **ALPIN-4I** (4 µM) at 37°C for 1 hour. The incubation medium was then removed, and fresh medium containing FBS was added. Subsequently, the cells were irradiated with a 660 nm light source at 36 mW for 10 minutes, followed by incubation at 37°C for 24 hours. Staining was performed using a live/dead cell staining kit (Beyotime), and fluorescence imaging was performed using a fluorescence microscope.

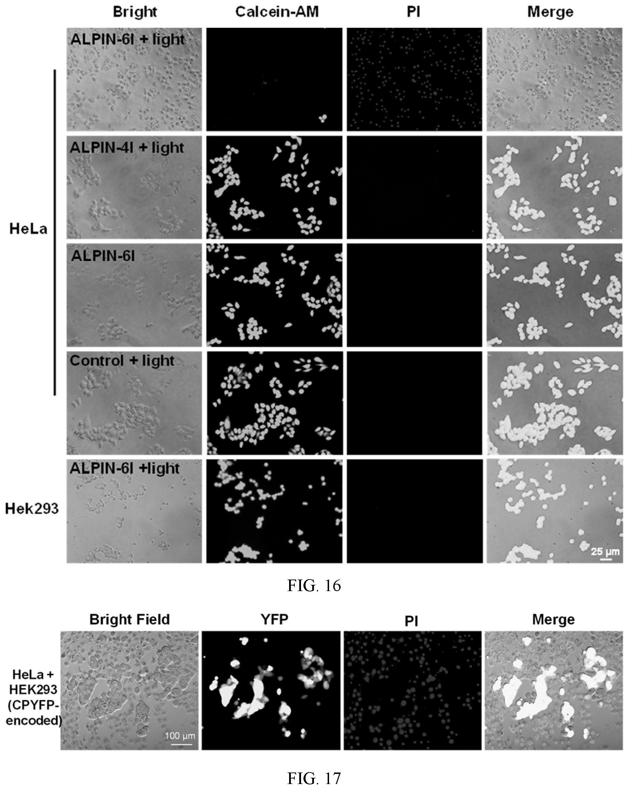

The experimental results are shown in FIG. 16. As shown in FIG. 16, only the **ALPIN-6I** plus light irradiation group showed PI staining for HeLa cells (red indicates dead cells). In contrast, both the control probe **ALPIN-4I** plus light irradiation group and the PBS plus light irradiation group showed only AM staining (green indicates live cells). The same results were observed for the HEK293 cells plus light irradiation group and the **ALPIN-6I** without light irradiation group.

Experimental conclusion: Only in the presence of both ALP and light irradiation can **ALPIN-6I** be activated to kill tumors. Furthermore, under the same conditions, **ALPIN-6I** shows superior photodynamic therapeutic efficacy than the control photosensitizer **ALPIN-4I.**

### Test Example 3.4: Selective killing of tumor cells by photosensitizer ALPIN-6I in mixed cell populations (FIG. 17)

Experimental conditions: A mixed population of HeLa cells and HEK293 cells (stably expressing yellow fluorescent protein) was incubated with the photosensitizer **ALPIN-6I** (4 µM) at 37°C for 30 minutes. The incubation medium was then removed, and fresh medium containing FBS was added. Subsequently, the cells were irradiated with a 660 nm light source at 36 mW for 10 minutes, followed by incubation at 37°C for 24 hours. PI staining was performed to identify dead cells using a live/dead cell staining kit (Beyotime), and fluorescence imaging was performed using a fluorescence microscope.

The experimental results are shown in FIG. 17. As shown in FIG. 17, only HeLa cells were stained red by PI, while HEK293 cells stably expressing yellow fluorescent protein showed no PI staining, indicating selective death of HeLa cells and survival of HEK293 cells.

Experimental conclusion: In a mixed cell population, **ALPIN-6I** can selectively kill ALP-overexpressing HeLa cells without affecting normal cells, which is attributed to the enzyme-activated retention of **ALPIN-6I** within tumor cells, preventing diffusion and uptake by normal cells.

### Test Example 3.5: Real-time imaging of HeLa tumor-bearing mice using near-infrared photosensitizer ALPIN-6I

Experimental conditions: Tumor-bearing mice were first divided into three groups with three mice per group. Whole-body background fluorescence images were acquired from six nude mice using an IVIS Lumina XRMS Series III *in vivo* imaging system (λₑₓ = 660 nm, λₑₘ = 710 nm). The photosensitizer **ALPIN-6I** or **ALPIN-4I** was then prepared as a 40 µM solution in PBS. Each tumor-bearing mouse received 200 µL of the prepared probe solution via tail vein injection using an insulin syringe. Subsequently, whole-body fluorescence data from the experimental mice were collected using the *in vivo* imaging system at multiple time points (1 h, 2 h, 3 h, 4 h, 6 h, 8 h, and 24 h). For the inhibitor group, 50 µL of 10 mM Na₃VO₄ solution was first injected intratumorally, and 30 minutes later, **ALPIN-6I** was administered at the same concentration, followed by imaging at the same time points.

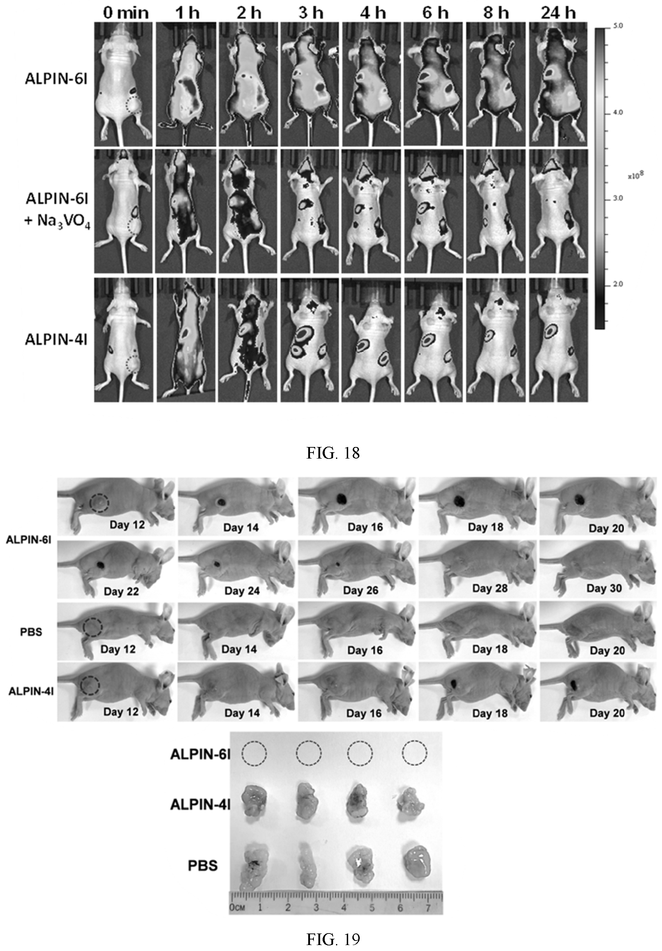

The experimental results are shown in FIG. 18. As shown in FIG. 18, following intravenous administration, the mice injected with **ALPIN-6I** exhibited strong fluorescence signals at the tumor site at 1 hour post-injection, and significant tumor-associated fluorescence remained detectable up to 24 hours. In contrast, the mice pre-injected with the inhibitor exhibited significantly reduced fluorescence signals at the tumor site. The control probe **ALPIN-4I** also produced detectable fluorescence at 1 hour, but the fluorescence was significantly weaker than that observed with **ALPIN-6I** and disappeared from the tumor site after 2 hours.

Experimental conclusion: In a highly dynamic *in vivo* system, **ALPIN-6I** demonstrates superior tumor-targeting accumulation and signal retention.

### Test Example 3.6: Photodynamic therapy of HeLa tumor-bearing mice using near-infrared photosensitizer ALPIN-6I

Experimental conditions: Tumor-bearing mice were first divided into three groups with four mice per group. The photosensitizer was administered via tail vein injection at 1.1 mg/kg. After 1 hour, the mice were irradiated with a 660 nm light source at 0.5 W for 10 minutes, and a second irradiation was performed 24 hours later. After 48 hours, the photosensitizer was re-injected, followed by light irradiation 1 hour later, and a second irradiation was performed 72 hours later. Subsequently, the tumor-bearing mice were allowed to recover spontaneously for 15 days.

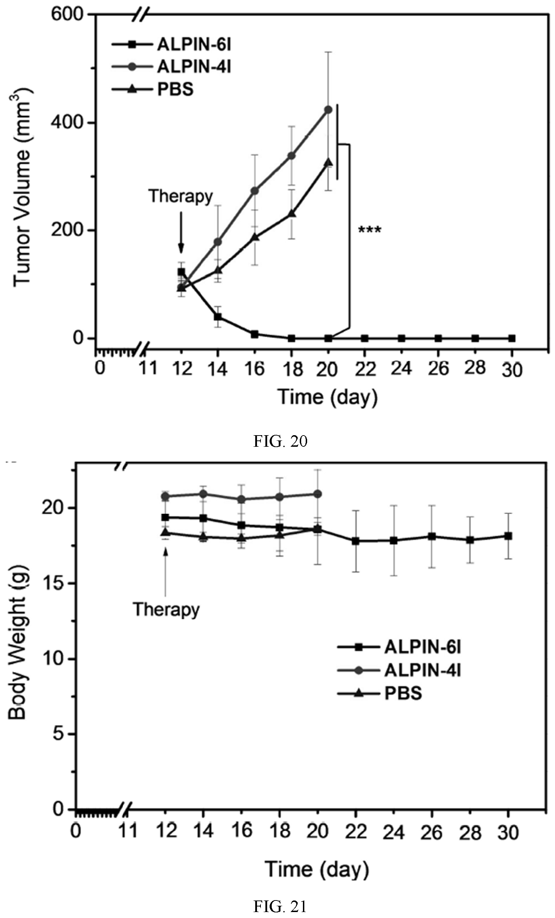

The experimental results are shown in FIGs. 19 to 21 and Table 3. As shown in FIGs. 19 to 21, following photodynamic therapy, the tumors of the tumor-bearing mice in the **ALPIN-6I** group completely disappeared after 4 days of treatment, and the mice recovered after 18 days of treatment with no recurrence observed. In contrast, the tumors in the control group did not disappear and gradually increased in size.

**Table 3**

| | Tumor volume (mm³) | | | | |
|---|---|---|---|---|---|
| | Day 12 | Day 14 | Day 16 | Day 18 | Day 20 |
| ALPIN-6I | 122.30 ± 17.56 | 39.73 ± 18.93 | 7.70 ± 7.01 | 0.00 | 0.00 |
| ALPIN-4I | 94.33 | 178.33 ± 67.68 | 273.33 ± 67.01 | 338.67 ± 54.45 | 424.00 ± 106.53 |
| PBS | 92 | 124.33 ± 20.55 | 186.33 ± 51.60 | 229.67 ± 45.46 | 325.33 ± 50.85 |

Experimental conclusion: In a highly dynamic *in vivo* system, **ALPIN-6I** demonstrates tumor-targeting ability and enables enrichment within tumor tissues, thereby improving the photodynamic therapeutic efficacy and enabling complete elimination of tumor tissues without recurrence.

### Test Example 4: Biological validation of near-infrared fluorescent and photoacoustic probe ALPIN-7

**Test Example 4.1:** Comparative imaging of HeLa cells overexpressing alkaline phosphatase (ALP) and HEK293 cells weakly expressing ALP using near-infrared fluorescent and photoacoustic probes ALPIN-7 and ALPIN-8

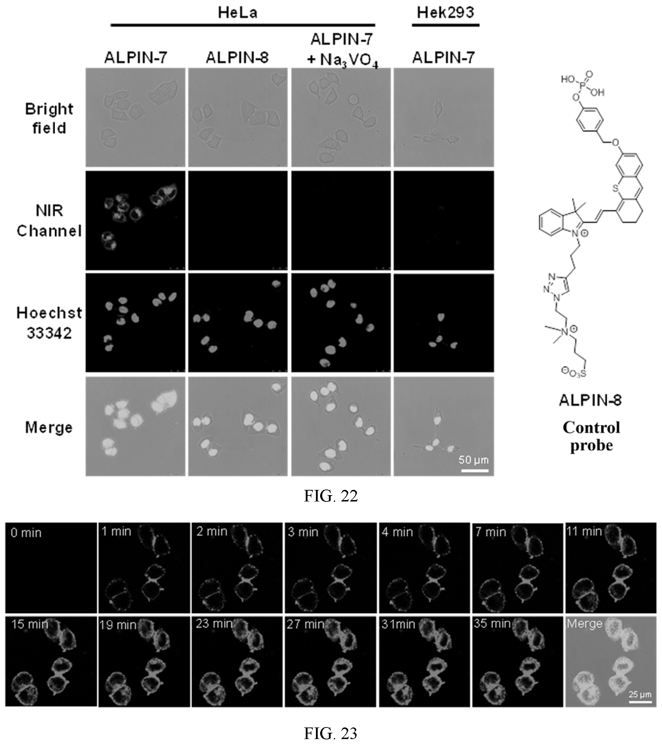

Experimental conditions: 5 µM of the fluorescent probes ALPIN-7 and ALPIN-8 (structure shown in FIG. 22) were incubated separately with HeLa cells overexpressing ALP on the cell surface and normal HEK293 cells with low ALP expression in a culture dish at 37°C for 30 minutes. Alternatively, HeLa cells were pre-treated with the alkaline phosphatase inhibitor Na₃VO₄ for 1 hour, followed by incubation with the probes for 30 minutes. After incubation, the probe-containing culture medium was discarded, and the cells were washed once with PBS (pH = 7.4). Subsequently, 1 mL of phenol red-free DMEM was added, and fluorescence imaging was performed using a confocal fluorescence microscope (Cy 5.5 channel).

The experimental results are shown in FIG. 22. As shown in FIG. 22, compared with the control probe ALPIN-8, ALPIN-7 not only enabled more pronounced fluorescence imaging of ALP-overexpressing cells, but also exhibited more significant fluorescence signals in ALP-overexpressing cells than in HEK293 cells with low ALP expression. Furthermore, in the ALP inhibitor group, fluorescence labeling of HeLa cells by ALPIN-7 was reduced, indicating that probe activation depends on the specific hydrolysis of phosphate ester bonds by alkaline phosphatase.

Experimental conclusion: Following reaction with intracellular ALP, the fluorescent probe ALPIN-7 exhibits enhanced cellular accumulation, reduces probe diffusion during cellular imaging, lowers background fluorescence, and greatly improves detection sensitivity.

### Test Example 4.2: Real-time imaging of HeLa cells using near-infrared fluorescent and photoacoustic probe ALPIN-7

Experimental conditions: Cultured HeLa cells were seeded in a confocal dish, and 1 µM of the fluorescent probe ALPIN-7 was added to the HeLa cells. Time-dependent fluorescence imaging was performed using a confocal fluorescence microscope (Cy 5.5 channel).

The experimental results are shown in FIG. 23. As shown in FIG. 23, during real-time imaging of HeLa cells, ALPIN-7 initially generated fluorescence signals rapidly on the cell membrane, consistent with the membrane localization of ALP in HeLa cells. As time progressed, fluorescence gradually appeared within the cells, and intracellular fluorescence intensity continuously increased over time.

Experimental conclusion: The fluorescent probe ALPIN-7 enables real-time visualization of ALP activity in HeLa cells. Upon enzymatic activation, the probe undergoes a hydrophilic-to-lipophilic transformation that enhances cellular uptake, while the quinone methide-mediated self-anchoring process limits the permeation and diffusion of the activated fluorescence signals, thereby greatly enhancing the accumulation of fluorescence signals within tumor cells and improving the capability of the probe for real-time visualization of enzyme activity.

### Test Example 4.3: Real-time imaging of HeLa tumor-bearing mice using near-infrared fluorescent and photoacoustic probe ALPIN-7

### Experimental conditions:

Establishment of HeLa tumor xenograft model in nude mice:
(1) Preparation of HeLa cells: Cells were cultured and passaged as described above. The cultured cells were centrifuged following digestion, and washed twice with PBS (1 mL per wash). After centrifugation and removal of the supernatant, the cells were resuspended in FBS-free DMEM, counted, mixed with Matrigel, and placed in an ice bath for later use.
(2) Inoculation of HeLa cells into tumor-bearing nude mice: Female BALB/c nude mice aged 4 to 6 weeks were subcutaneously inoculated in the right hind limb with 200 µL of a mixture containing HeLa cells (4 × 10⁶) in DMEM (without FBS) and Matrigel (1:4), using a 1 mL sterile medical syringe. The mice were maintained at 25°C for 2 to 3 weeks with free access to water and food. When the tumor volume reached approximately 0.1 cm³, animal imaging experiments were performed.
(3) *In vivo* imaging of HeLa tumor-bearing nude mice using probes via tail vein injection: The experimental mice were first divided into three groups with three mice per group. Whole-body background fluorescence images were acquired from six nude mice using an IVIS Lumina XRMS Series III *in vivo* imaging system (λₑₓ = 660 nm, λₑₘ = 710 nm). The fluorescent probe ALPIN-7 or ALPIN-8 was then prepared as a 40 µM solution in PBS. Each tumor-bearing mouse received 200 µL of the prepared probe solution via tail vein injection using an insulin syringe. Subsequently, whole-body fluorescence data from the experimental mice were collected using the *in vivo* imaging system at multiple time points (1 h, 2 h, 3 h, 4 h, 6 h, 8 h, and 48 h). For the inhibitor group, 50 µL of 10 mM Na₃VO₄ solution was first injected intratumorally, and 30 minutes later, ALPIN-7 was administered at the same concentration, followed by imaging at the same time points.
(4) *Ex vivo* imaging of tissues from HeLa tumor-bearing nude mice: In the tail vein injection group, at 36 hours post-injection, the nude mice were sacrificed by cervical dislocation. The tumors, heart, lungs, liver, spleen, and kidneys were harvested and subjected to fluorescence imaging.

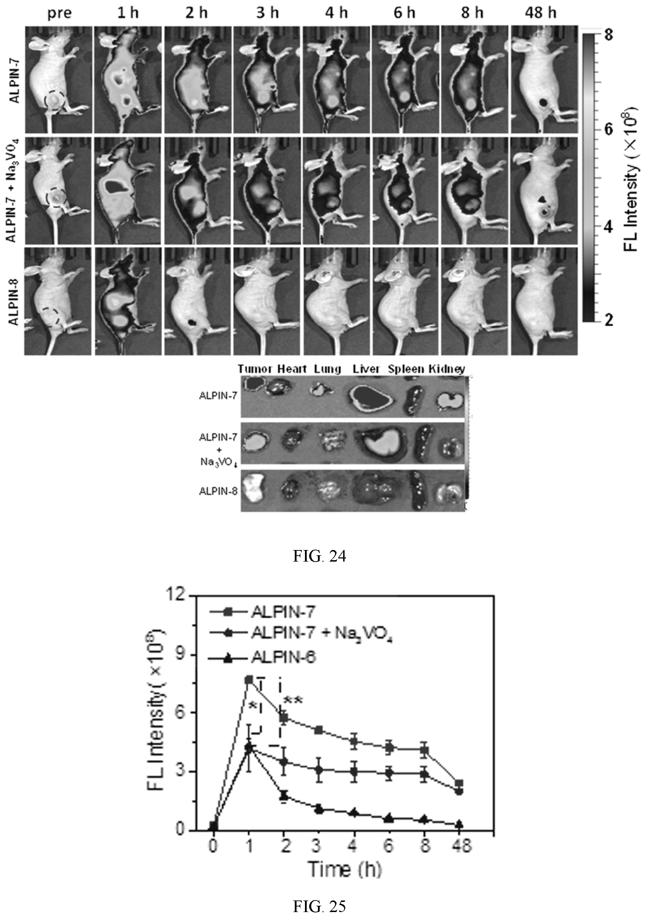

The experimental results are shown in FIGs. 24 to 26. As shown in FIGs. 24 to 26, following intravenous administration, the mice injected with ALPIN-7 exhibited strong fluorescence signals at the tumor site at 1 hour post-injection, and significant tumor-associated fluorescence remained detectable up to 36 hours. In contrast, the mice pre-injected with the inhibitor exhibited significantly reduced fluorescence signals at the tumor site. The control probe ALPIN-8 also produced detectable fluorescence at 1 hour, but the fluorescence was significantly weaker than that observed with ALPIN-7 and disappeared from the tumor site after 2 hours. The results were also consistent with the fluorescence signals observed at the tumor site after dissection of the tumor-bearing mice.

Experimental conclusion: In a highly dynamic *in vivo* system, ALPIN-7 demonstrates superior tumor-targeting accumulation and signal retention, enhancing tumor-associated fluorescence intensity *in vivo,* which facilitates the detection of tumor tissues *in vivo* and plays a crucial role in surgical navigation for tumor resection.

### Test Example 5: UV absorption and fluorescence spectral analysis of enzymatic hydrolysis

Experimental conditions: The probes **BIN-3** and **BIN-2** were prepared as 5 µM working solutions in PBS (pH = 7.4). The final concentration of β-lactamase TEM-1 was 100 nM, and the final concentration of nucleophile β-mercaptoethanol was 10 mM. The total reaction volume was 100 µL, and the reaction mixture was incubated at 37°C for 15 minutes. The photosensitizers **BIN-3I** and **BIN-2I** were prepared as 10 µM working solutions in PBS (pH = 7.4). The final concentration of β-lactamase TEM-1 was 100 nM, and the final concentration of nucleophile β-mercaptoethanol was 100 µM. The total reaction volume was 100 µL, and the reaction mixture was incubated at 37°C for 15 minutes. Subsequently, all UV absorption spectra were measured at room temperature using a Shimadzu UV-Vis spectrophotometer over a wavelength range of 400 to 800 nm. All fluorescence spectra were measured using a fluorescence spectrometer with an excitation wavelength of 680 nm and an emission scanning range of 690 to 850 nm.

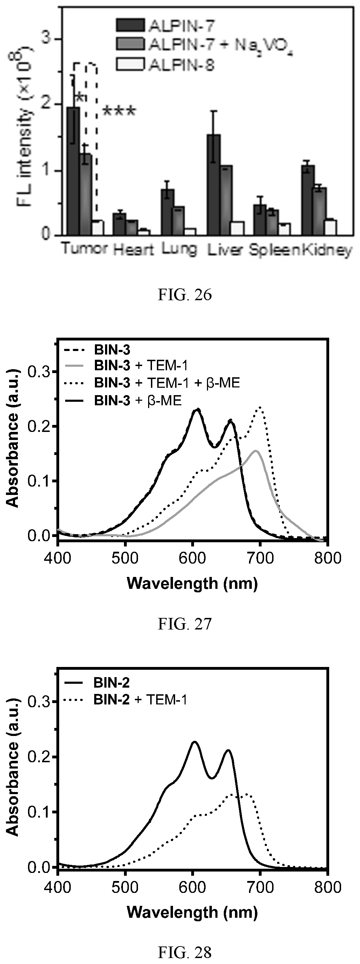

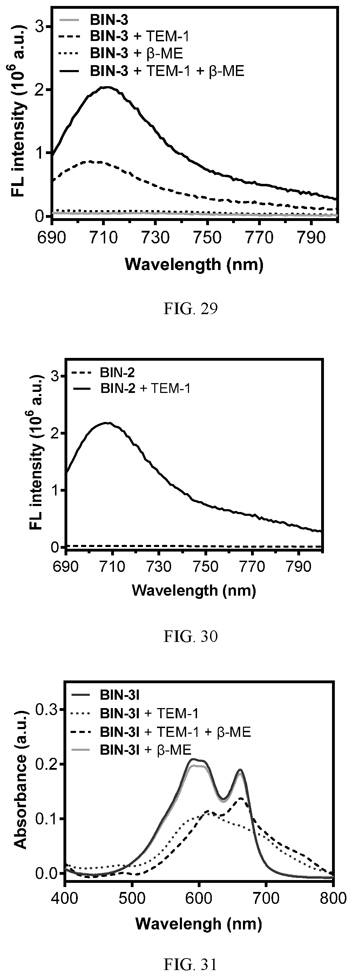

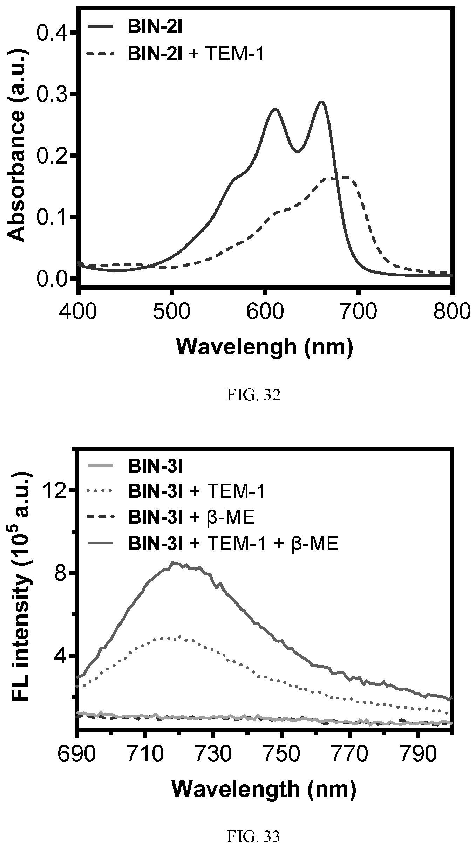

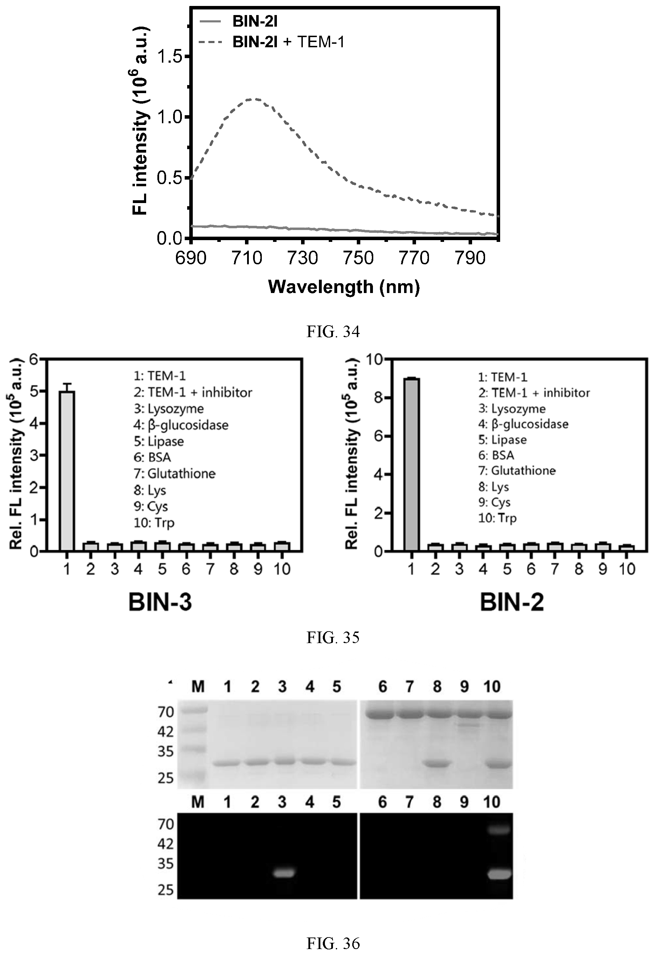

Experimental results: The UV absorption spectra of the near-infrared probes are shown in FIGs. 27 and 28. Both the primary probe **BIN-3** and the control probe **BIN-2** exhibited a red-shift in the absorption spectra following enzymatic hydrolysis. **BIN-3** also exhibited a red-shift in the absorption spectra after enzymatic hydrolysis followed by nucleophile attack, while no changes were observed in the absorption spectrum after incubation of **BIN-3** with the nucleophile. The fluorescence spectra shown in FIGs. 29 and 30 demonstrate that fluorescence was activated after enzymatic hydrolysis for both the primary probe **BIN-3** and the control probe **BIN-2.** Moreover, fluorescence from **BIN-3** was further enhanced after enzymatic hydrolysis followed by nucleophile attack. The UV absorption spectra of the photosensitizers are shown in FIGs. 31 and 32. Both the primary photosensitizer **BIN-3I** and the control photosensitizer **BIN-2I** exhibited changes in the absorption spectra following enzymatic hydrolysis, while no changes were observed in the absorption spectrum after incubation of **BIN-3I** with the nucleophile. The fluorescence spectra shown in FIGs. 33 and 34 demonstrate that fluorescence was activated after enzymatic hydrolysis for both the primary photosensitizer **BIN-3I** and the control photosensitizer **BIN-2I.** Moreover, fluorescence from **BIN-3I** was further enhanced after enzymatic hydrolysis followed by nucleophile attack. These results indicate that, under conditions simulating the *in vivo* dynamic process through the addition of a large amount of nucleophile β-mercaptoethanol, enzymatic hydrolysis of the fluorescent probe **BIN-3** or photosensitizer **BIN-3I** first generates a quinone methide intermediate, which is subsequently attacked by a large amount of the nucleophile, resulting in secondary activation of fluorescence.

### Test Example 6: HPLC analysis of enzymatic hydrolysis

Experimental conditions: The probes were prepared as 5 µM working solutions in PBS (pH = 7.4), while the photosensitizers were prepared as 10 µM working solutions in PBS (pH = 7.4). The final concentration of β-lactamase TEM-1 was 100 nM, and the final concentration of nucleophile β-mercaptoethanol was 10 mM. The total reaction volume was 100 µL, and the reaction mixture was incubated at 37°C for 15 minutes. Hydrolysis was monitored by HPLC at a detection wavelength of 660 nm.

The experimental results showed that the primary probe **BIN-3** can be hydrolyzed by β-lactamase TEM-1. Following enzymatic hydrolysis, the HPLC chromatogram of **BIN-3** exhibited a series of peaks, indicating the formation of an unstable quinone methide intermediate. In the presence of a sufficient amount of nucleophile β-mercaptoethanol after enzymatic hydrolysis, a single stable chromatographic peak was obtained, which was verified by mass spectrometry to be a stable structure formed by covalent conjugation of the thiol group with the quinone methide intermediate

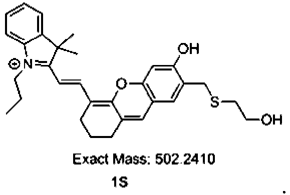

The experimental results further showed that the control probe **BIN-2** can also be hydrolyzed by β-lactamase TEM-1, yielding Compound 7 as the hydrolysis product 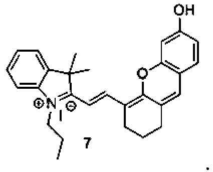 The results for the photosensitizers were similar to those for the probes. Following enzymatic hydrolysis, the primary photosensitizer **BIN-3I** produced a series of irregular HPLC peaks, indicating the formation of an unstable quinone methide intermediate. In the presence of a sufficient amount of β-mercaptoethanol after enzymatic hydrolysis, a single chromatographic peak was formed. By contrast, hydrolysis of the control photosensitizer **BIN-2I** by TEM-1 produced Compound **14** 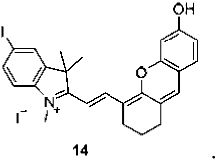 These results indicate that, unlike the hydrolysis of the comparative/control probes and photosensitizers, which directly yields a single hydrolysis product, the probes and photosensitizers of the present disclosure undergo a dual-activation process: enzymatic hydrolysis followed by nucleophile attack, ultimately resulting in a single stable product.

### Test Example 7: Enzyme selectivity assay

Experimental conditions: The probes were prepared as 5 µM working solutions in PBS (pH = 7.4), followed by addition of various enzymes and proteins. The total reaction volume was 100 µL, and the reaction mixture was incubated at 37°C for 30 minutes. Subsequently, the fluorescence intensity of all groups was measured using a microplate reader, with excitation and emission wavelengths set at 680 nm and 720 nm, respectively.

As shown in FIG. 35, both the primary probe **BIN-3** and the control probe **BIN-2** specifically responded to β-lactamase TEM-1, but showed no response to other enzymes and proteins, indicating that the probes exhibit good selectivity for β-lactamase and can avoid interference from other endogenous enzymes and proteins.

### Test Example 8: SDS-PAGE and dual-color near-infrared fluorescence scanning analysis

Experimental conditions: A 12% SDS-PAGE resolving gel and a 5% stacking gel were prepared. The probes and photosensitizers were prepared as 10 µM working solutions in PBS (pH = 7.4) and incubated with different proteins at 37°C for 2 hours. The concentrations of β-lactamase TEM-1 and BSA were 5 µM and 10 µM, respectively. For the inhibitor group, the inhibitor avibactam (1 mM) was pre-incubated with TEM-1 for 30 minutes before co-incubation with the probe. Subsequently, SDS-PAGE loading buffer was added to the samples and mixed thoroughly. Each sample (15 µL) was loaded onto the gel and subjected to electrophoresis. Upon completion, fluorescent bands were obtained using dual-color near-infrared fluorescence scanning. Finally, the gel was stained with Coomassie Brilliant Blue and photographed using a gel imaging system.

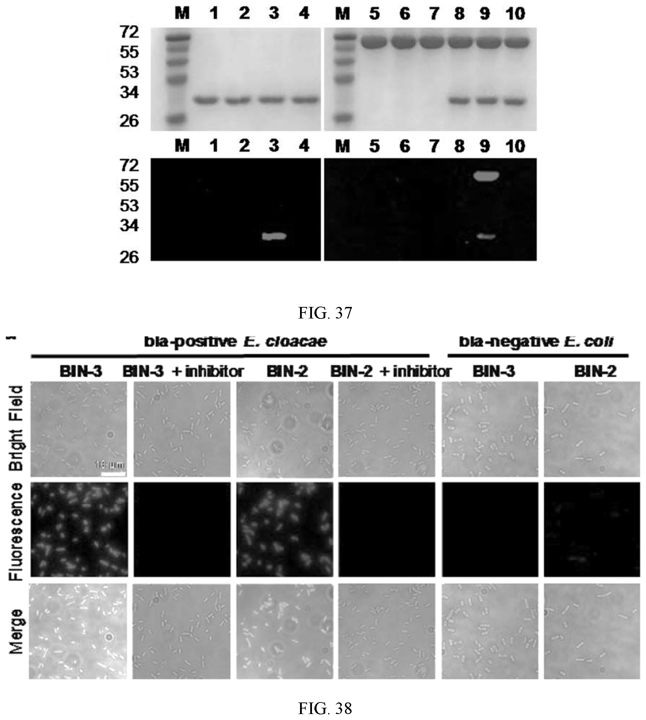

As shown in FIGs. 36 and 37, red fluorescent bands were observed for the primary probe **BIN-3** or primary photosensitizer **BIN-3I** after incubation with TEM-1. In contrast, no fluorescence signals were observed for the control probe **BIN-2** or control photosensitizer **BIN-2I** after incubation with TEM-1 in the presence of the inhibitor. In groups containing additional BSA protein, **BIN-3** and **BIN-3I,** after activation by TEM-1, were able to label the BSA protein bands, while **BIN-2** and **BIN-2I** did not exhibit any protein-labeling capability. These results indicate that, after **BIN-3 or BIN-3I** is activated by TEM-1, the recognition moiety is released to generate a quinone methide intermediate, which are capable of forming covalent bonds with the target protein or with neighboring binding proteins, thereby enabling protein labeling.

### Test Example 9: Biocompatibility evaluation in cells and bacteria

Experimental conditions: The *in vitro* biocompatibility of probes was evaluated using bacterial growth curve assay, CCK-8 cell viability assay, and red blood cell hemolysis assay.

In the bacterial growth curve assay, the experiment was conducted in a biosafety cabinet. *E. coli* strains producing or not producing β-lactamase were first revived on LB agar plates. A single colony was then inoculated into 1 mL of culture medium and incubated overnight at 37°C with shaking. The following day, the bacterial culture was diluted 10⁶-fold, and the time point was designated as 0 h. Subsequently, the probe was added to a final concentration of 50 µM, and the culture was incubated with shaking. At predetermined intervals, aliquots of the bacterial culture were serially diluted and spotted onto agar plates. After overnight incubation, bacterial colonies were counted to evaluate bacterial survival and determine the effect of the probe on bacterial growth.

The results showed that the growth profiles of *E. coli* producing or not producing β-lactamase were essentially identical in the presence and absence of the probe, indicating that the probe exhibited good biocompatibility with bacteria.

In the CCK-8 cell viability assay, the experiment was conducted in a biosafety cabinet. Well-adhered human embryonic kidney cells (HEK293 cells) were digested with trypsin, collected into a centrifuge tube, and centrifuged at 1,000 rpm for 3 minutes. After centrifugation, the supernatant was discarded, and the cells were diluted to a density of 1 × 10⁵ cells/mL to prepare a working suspension. 100 µL of the cell suspension was added to each well of a transparent 96-well plate and incubated for 24 hours to allow the cells to adhere. After gently discarding the culture medium with a pipette, fresh medium containing the probe at different concentrations (10, 20, 40, and 100 µM) were added. DMEM alone and untreated cells were used as blank and positive controls, respectively. The plate was then incubated for another 24 hours. Subsequently, according to the instructions for the CCK-8 kit, 10 µL of CCK-8 reagent was added to each well, followed by incubation at 37°C for 3 hours. Absorbance at 450 nm was then measured using a microplate reader.

The results showed that no reduction in cell viability was observed in probe-treated groups compared with untreated controls, indicating that the probe exhibited high safety for cells.

In the red blood cell hemolysis assay, fresh red blood cells (RBCs) collected from Balb/C mice were washed with PBS and diluted to prepare a 5% (v/v) RBC working solution. The compound was serially diluted in PBS, starting at an initial concentration of 200 µM and using a two-fold dilution series down to 3.125 µM. Subsequently, 50 µL of each compound solution was pipetted into a transparent 96-well plate, followed by 50 µL of the RBC working solution to make a final volume of 100 µL per well. PBS-treated and 0.1% Triton X-100-treated samples were used as blank and positive controls, respectively. After incubation at 37°C for 1 hour, the plate was centrifuged at 3,500 rpm for 5 minutes. An 80 µL aliquot of the supernatant from each well was transferred to a new transparent plate, and absorbance at 405 nm was measured using a microplate reader to calculate the percentage of hemolysis.

The experimental results showed that at a probe concentration of 100 µM, the hemolysis rate was comparable to that of the blank control group, indicating that the probe exhibited no hemolytic toxicity to mouse red blood cells with excellent biocompatibility.

### Test Example 10: Imaging experiment on bacteria using probes

Experimental conditions: The bacterial experiment was conducted in a biosafety cabinet. Bacteria producing or not producing β-lactamase were first revived on LB agar plates. A single colony was then inoculated into 1 mL of culture medium and incubated at 37°C with shaking for 3 to 5 hours until reaching the logarithmic growth phase. The bacteria were collected into a sterile EP tube, centrifuged at 5,000 rpm, washed twice with PBS, and resuspended in PBS. A bacterial suspension at a concentration of 1 × 10⁷ CFU was incubated with the primary probe **BIN-3** and the control probe **BIN-2** at 37°C for 2 hours. An additional "probe-washing" group was included. After incubation, the bacterial suspension was washed twice with PBS. Subsequently, the bacteria were spotted onto a glass slide and imaged under a fluorescence microscope using the Cy5.5 channel, with the Cy5.5 fluorescence signal pseudo-colored red.

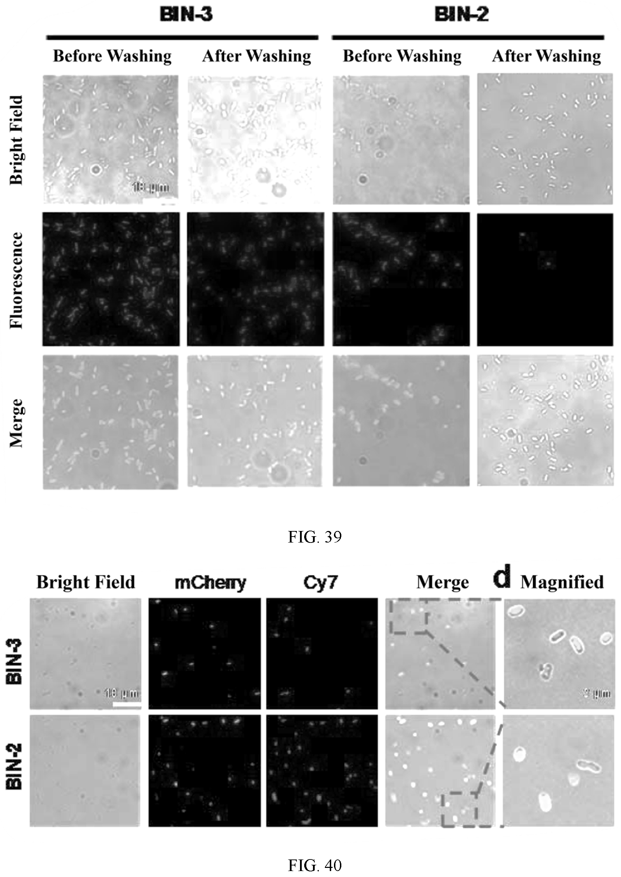

Experimental results: As shown in FIG. 38, red fluorescence was observed on the bacteria after normal incubation with both the primary probe **BIN-3** and the control probe **BIN-2.** However, no fluorescence was observed on the bacteria after pre-treatment with an inhibitor. After washing with PBS, as shown in FIG. 39, the **BIN-3** group still exhibited fluorescence labeling, whereas the **BIN-2** group showed almost no fluorescence. These results indicate that the primary probe **BIN-3** can label drug-resistant bacteria under wash-free conditions and is not prone to diffusion.

For selective imaging experiments involving mixed bacteria populations expressing β-lactamase and mCherry fluorescent protein, the procedures for bacterial culture were identical to those described above, except that prior to imaging, the two bacterial populations were mixed thoroughly and incubated with the probe at 37°C for 2 hours. Subsequently, the bacteria were spotted onto a glass slide and imaged under a fluorescence microscope using both the mCherry and Cy5.5 channels, with the mCherry fluorescence signal pseudo-colored green and the Cy5.5 fluorescence signal pseudo-colored red.

Experimental results: As shown in FIG. 40, in the mixed-bacteria imaging experiment, the primary probe **BIN-3** accurately labeled the drug-resistant bacteria expressing β-lactamase, while no fluorescence signal was observed on the mCherry-expressing bacteria. However, the control probe **BIN-2** generated fluorescence signals not only on the drug-resistant bacteria but also on the mCherry-expressing bacteria. These results indicate that the primary probe **BIN-3** can eliminate interference from bacteria that do not express β-lactamase and can covalently label bacteria expressing β-lactamase. In contrast, the control probe, which lacks an anchoring group, is affected by the diffusing fluorophore and generates fluorescence signals on bacteria that do not express β-lactamase.

### Test Example 11: Evaluation of probe enrichment

Experimental conditions: The bacterial experiment was conducted in a biosafety cabinet. *Enterobacter cloacae* producing β-lactamase was first revived on LB agar plates. A single colony was then inoculated into 1 mL of culture medium and incubated at 37°C with shaking. The bacteria were collected into a sterile EP tube, centrifuged at 5,000 rpm, washed twice with PBS, and resuspended in PBS. A bacterial suspension at a concentration of 1 × 10⁹ CFU was incubated with the primary probe **BIN-3** and the control probe **BIN-2** at 37°C for 2 hours. Subsequently, the culture medium was collected to measure the fluorescence intensity. The bacteria were resuspended in lysis buffer, and the fluorescence intensity of the bacterial lysate was measured to calculate the probe concentration in the bacteria.

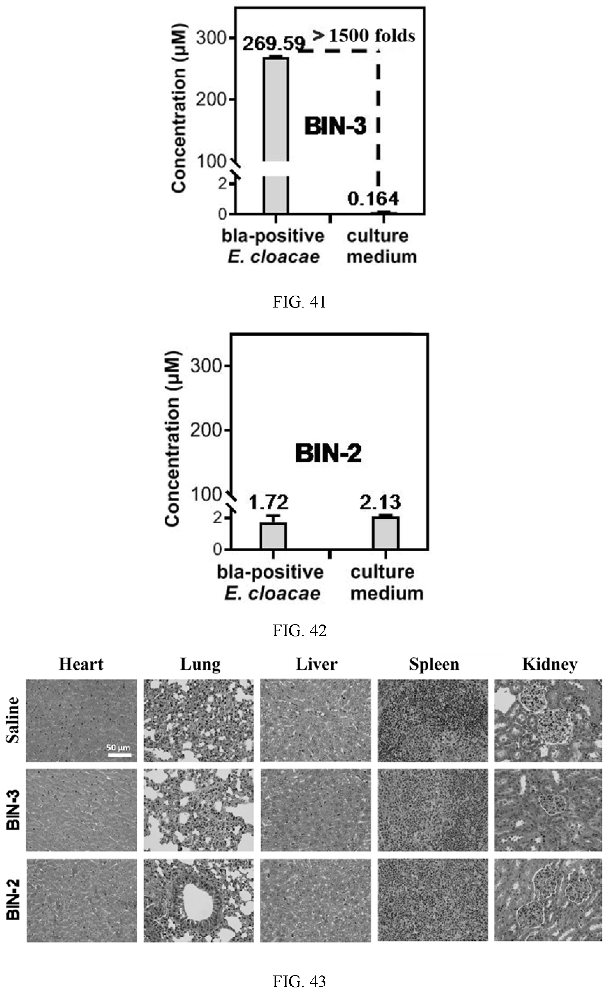

The experimental results are shown in FIGs. 41 to 42. After incubation with the primary probe **BIN-3** followed by PBS washing, β-lactamase-expressing *Enterobacter cloacae* exhibited a visible green coloration, while the bacteria incubated with the control probe **BIN-2** showed no visible coloration. Following fluorescence measurements of both the culture supernatant and bacterial lysate, the probe concentrations in the bacteria and the culture medium were calculated. As shown in FIGs. 41 to 42, in the **BIN-3** group, the probe concentration in the bacteria was more than 1500-fold higher than that in the culture medium, far exceeding the probe concentration in the culture medium. Although **BIN-2** and **BIN-3** have highly similar structures, the concentration of **BIN-2** was 150-fold lower than that of **BIN-3** in the bacteria. Furthermore, analysis of the probe concentration in the culture medium showed that most of **BIN-2** remained in the culture medium, rather than being enriched on drug-resistant bacteria as was the case with **BIN-3.** These findings demonstrate that the primary probe can achieve enrichment in bacteria, a property particularly useful for detection of expressing bacteria in highly dynamic environments such as living animals.

### Test Example 12: In vivo safety

Experimental conditions: The *in vivo* safety of probes was evaluated by analyzing blood routine parameters, blood biochemical indicators, and histological staining. Female BALB/c mice aged 4 to 6 weeks were obtained from the Shanghai Laboratory Animal Research Center. The mice were housed at 25°C with free access to food and water. For histological analysis, the mice were divided into three groups with three mice per group, and administered normal saline, the primary probe **BIN-3,** and the control probe **BIN-2** (1 µmol/kg) via tail vein injection. Twenty-four hours after injection of normal saline or probes, the mice were euthanized and dissected. Tissue sections were prepared from the organs (heart, lungs, liver, spleen, and kidneys) and subjected to hematoxylin and eosin (H&E) staining. For hematological analysis, the mice were divided into three groups with three mice per group, and administered normal saline, **BIN-3,** and **BIN-2** (1 µmol/kg) via tail vein injection. Twenty-four hours after injection, blood samples were collected from the mice in each group and submitted for blood routine analysis.

Experimental results: As shown in FIG. 43, 24 hours after intravenous injection of **BIN-3 or BIN-2,** a series of blood biochemical indicators were evaluated, including white blood cells, red blood cells, hemoglobin, hematocrit, mean corpuscular volume, mean corpuscular hemoglobin, mean corpuscular hemoglobin concentration, platelet count, procalcitonin, total protein, albumin, and urea levels, as well as H&E staining of major organs. According to the results, no significant differences were observed among the mice injected with **BIN-2, BIN-3,** and normal saline, indicating that these probes exhibit excellent biocompatibility *in vivo.*

### Test Example 13: Real-time in vivo imaging of bacteria

Experimental conditions: *In vivo* imaging experiments were conducted by injecting the probes into murine infection models followed by real-time fluorescence imaging using an IVIS system. Female BALB/c mice aged 4 to 6 weeks were obtained from the Shanghai Laboratory Animal Research Center. The mice were housed at 25°C with free access to food and water. For real-time *in vivo* imaging, the mice were divided into groups with five mice per group: the primary probe **BIN-3, BIN-3** combined with an inhibitor, and the control probe **BIN-2.** The probe concentration was 1 µmol/kg, and the concentration of the inhibitor avibactam was 100 mM. *E. coli* expressing bla (5 × 10⁸ CFU) was injected into the right hind limb of the mice to establish a mouse infection model. One hour after bacterial infection, **BIN-3** or **BIN-2** (1 µmol/kg) was administered via tail vein injection. For the inhibitor group, avibactam (100 mM) was incubated with the bacteria at 37°C for 2 hours and injected into the right hind limb of the mice, followed by tail vein injection of **BIN-3.** Whole-body fluorescence images were acquired at 1, 2, 3, 4, 6, 8, and 10 hours post-injection using an IVIS Lumina XRMS Series III imaging system. Fluorescence intensity was quantified using region of interest (ROI) analysis in Living Image software.

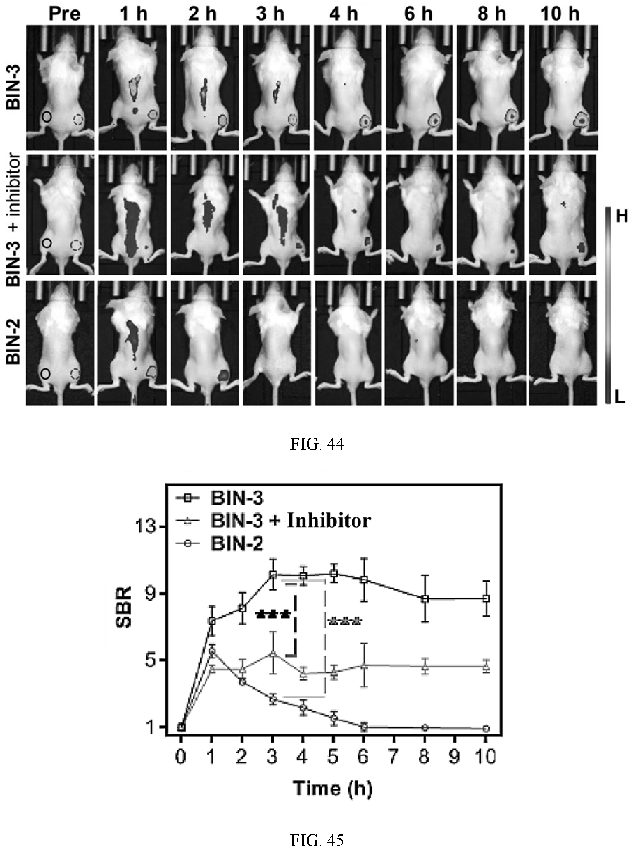

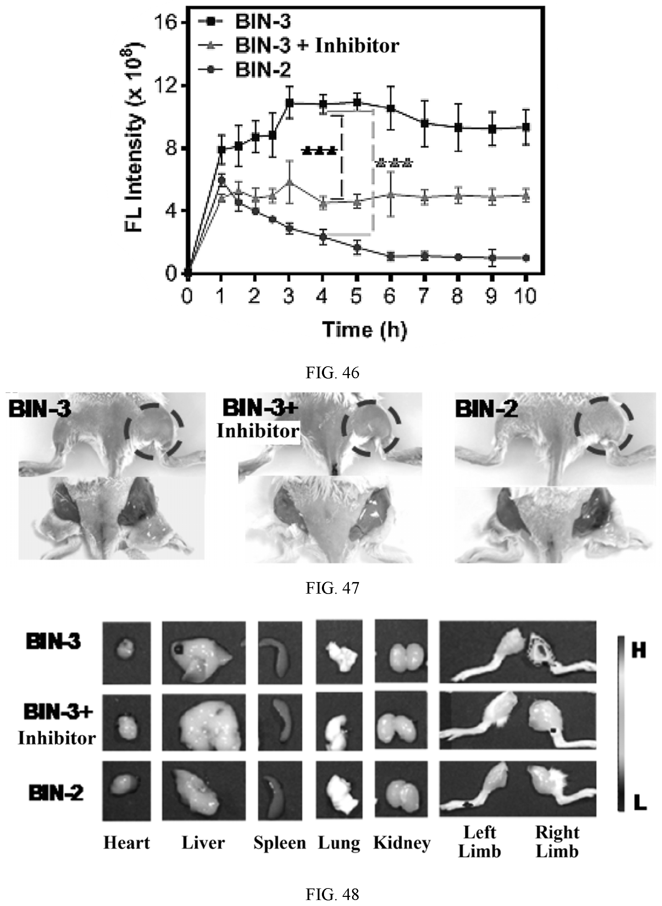

Experimental results: As shown in FIGs. 44 to 49 and Table 4, a strong fluorescence signal was observed at the site of bacterial infection in the mice 1 hour after injection of the primary probe **BIN-3.** In the following hours, the fluorescence intensity continued to increase. At 3 hours after injection of the primary probe **BIN-3,** the signal-to-background ratio (SBR) exceeded 10. At 10 hours post-injection, a detectable fluorescence signal remained at the infection site, with an SBR of approximately 9. The effective retention of the active fluorophore at the infection site extended the imaging window and improved both signal-to-background ratio and detection sensitivity. Furthermore, pre-treatment of the bacteria with the inhibitor avibactam effectively reduced the fluorescence intensity, thereby demonstrating that the observed fluorescence signal resulted from bla-mediated hydrolysis of **BIN-3.** As a control, the imaging performance of the control probe **BIN-2** was also evaluated *in vivo.* Fluorescence was observed at the infection site 1 hour after tail vein injection of the probe. However, both fluorescence intensity and SBR were lower compared with **BIN-3.** As time progressed, fluorescence from **BIN-2** at the infection site decreased rapidly, with the SBR dropping to approximately 1 at 5 hours post-injection, almost indistinguishable from the background signal. In contrast, the self-anchoring probe **BIN-3** produced significantly stronger fluorescence and higher SBR at the infection site. *Ex vivo* fluorescence imaging showed that the self-anchoring primary probe **BIN-3** produced much stronger fluorescence signals at the infected right hind limb compared with the control probe **BIN-2,** while the fluorescence signals in other major organs (such as the heart, liver, spleen, lungs, and kidneys) remained very weak, indicating that **BIN-3** exhibits high specificity *in vivo* (FIG. 46). When the skin over the infected hind limb of the mice injected with **BIN-3** was removed, a distinct blue-green coloration, characteristic of hemicyanine dyes, was clearly visible at the infection site. In contrast, no such blue-green coloration was observed in the mice injected with **BIN-2** or the mice pre-treated with the inhibitor (FIG. 47). Quantitative analysis of the fluorescence intensity in isolated organs further demonstrated that the infected right hind limb in the **BIN-3** group exhibited the highest fluorescence intensity, while both the inhibitor group and **BIN-2** group showed significantly weaker signals than the **BIN-3** group. These findings demonstrate that **BIN-3** exhibits significant retention efficiency at the activation site in highly dynamic animals, which may be attributed to the novel hydrophilicity-switchable anchoring design based on quinone methide.

**Table 4**

| | Relative fluorescence intensity at different time points (× 10⁸) | | | | | | | | | | |
|---|---|---|---|---|---|---|---|---|---|---|---|
| | 0 (h) | 1 (h) | 2 (h) | 3 (h) | 4 (h) | 5 (h) | 6 (h) | 7 (h) | 8 (h) | 9 (h) | 10 (h) |
| BIN-2 | 0 | 5.97 ± 0.41 | 3.99 ± 0.18 | 2.88 ± 0.34 | 2.33 ± 0.48 | 1.66 ± 0.44 | 1.1 ± 0.28 | 1.12 ± 0.28 | 1.05 ± 0.2 | 1 ± 0.54 | 1 ± 0.19 |
| BIN-3 | 0.1 | 7.91 ± 0.92 | 8.73 ± 1.01 | 10.9 ± 1 | 10.82 ± 0.6 | 10.97 ± 0.58 | 10.55 ± 1.36 | 9.6 ± 1.48 | 9.33 ± 1.51 | 9.24 ± 1.07 | 9.36 ± 1.12 |
| BIN-3 + inhibitor | 0.1 | 4.83 ± 0.26 | 4.83 ± 0.61 | 5.85 ± 1.34 | 4.53 ± 0.4 | 4.62 ± 0.44 | 5.06 ± 1.42 | 4.88 ± 0.46 | 5 ± 0.5 | 4.9 ± 0.5 | 5 ± 0.4 |

### Test Example 14: In vivo sensitivity imaging of bacteria

Experimental conditions: *In vivo* sensitivity imaging experiments were conducted by injecting the probes into murine myositis infection models inoculated with different bacterial loads, followed by fluorescence imaging using an IVIS system. Female BALB/c mice aged 4 to 6 weeks were obtained from the Shanghai Laboratory Animal Research Center. The mice were housed at 25°C with free access to food and water. For *in vivo* sensitivity imaging, the mice were divided into groups with three mice per group. The primary probe **BIN-3** was administered at a dose of 1 µmol/kg. Four different sites were established on each mouse. The left upper limb served as the normal saline control. The right upper limb, left lower limb, and right lower limb were inoculated with 5 × 10⁶ CFU, 5 × 10⁷ CFU, and 5 × 10⁸ CFU, respectively. One hour after bacterial injection, the probe was administered via tail vein injection. Five hours later, fluorescence imaging was performed using an IVIS Lumina XRMS Series III imaging system. Fluorescence intensity was quantified using ROI analysis in Living Image software.

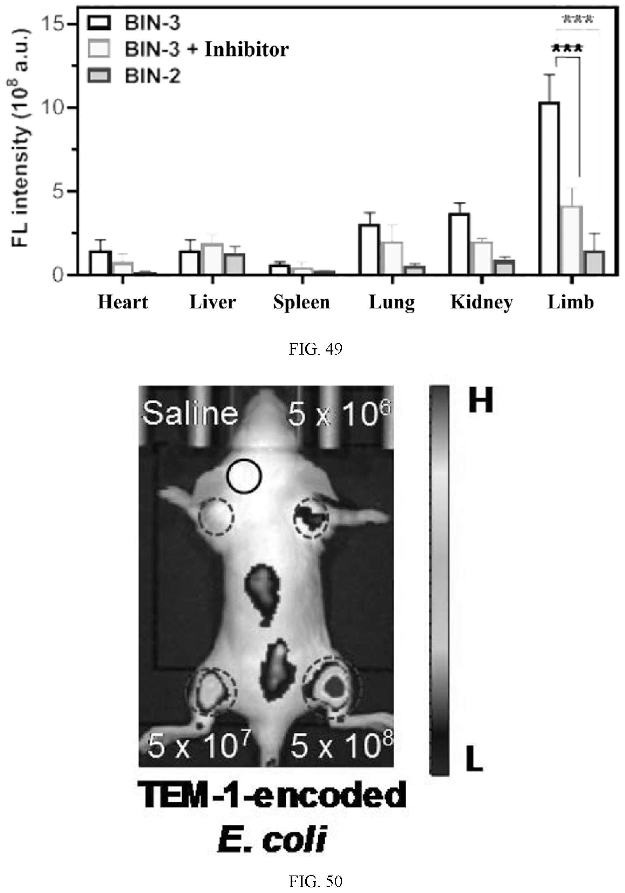

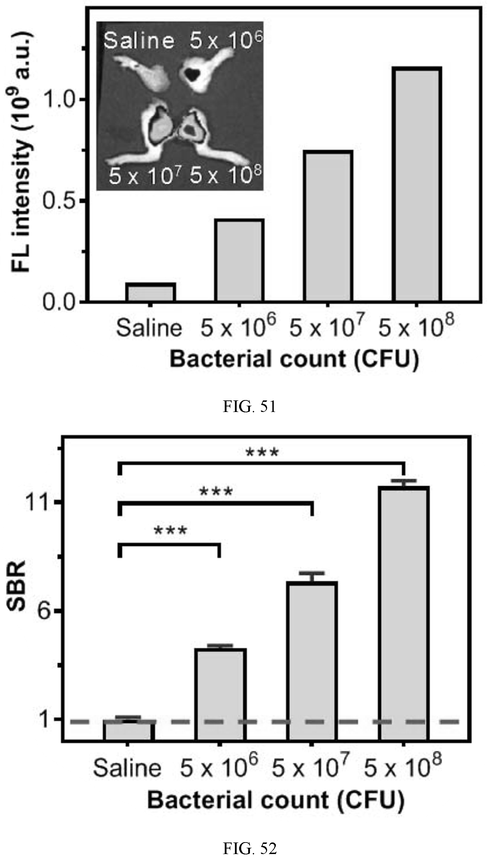

Experimental results: As shown in FIGs. 50 to 52 and Tables 5 to 6, one hour after injection of the primary probe **BIN-3,** fluorescence signals of varying intensities were observed at sites in the mice inoculated with different bacterial loads. The fluorescence intensity was positively correlated with the bacterial load. Quantitative analysis of the fluorescence intensity at different infection sites demonstrated that a higher bacterial load corresponded to a higher fluorescence intensity, which was further confirmed by the signal-to-background ratio between the infected site and the whole-body background.

**Table 5**

| Bacterial load (× 10⁶ CFU) | Fluorescence intensity (× 10⁶) |
|---|---|
| 0 (saline) | 0.095 |
| 5 | 0.411 |
| 50 | 0.753 |
| 500 | 1.16 |

**Table 6**

| Bacterial load (× 10⁶ CFU) | Fluorescence intensity (× 10⁶) |
|---|---|
| 0 (saline) | 1.01 ± 0.1 |
| 5 | 4.31 ± 0.1 |
| 50 | 7.37 ± 0.38 |
| 500 | 11.74 ± 0.26 |

### Test Example 15: In vivo selective imaging of drug-resistant bacteria

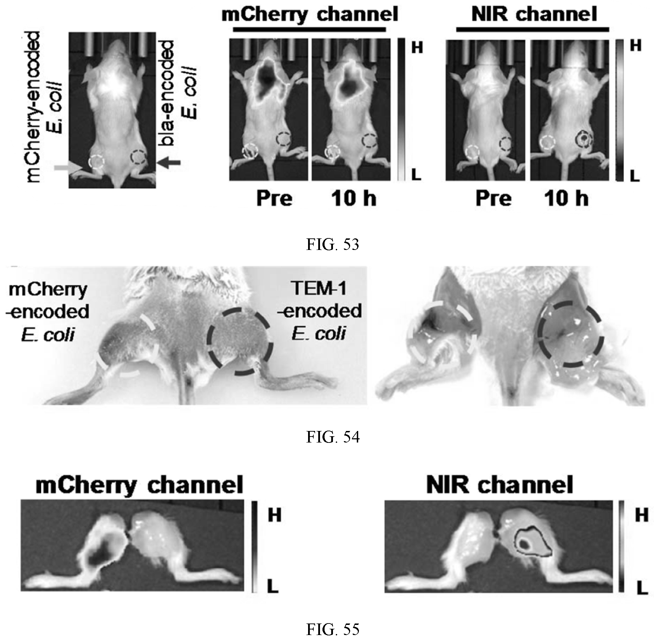

Experimental conditions: *In vivo* selective imaging experiments were conducted by injecting the probes into mice infected with different bacterial strains, followed by real-time fluorescence imaging using an IVIS system. Female BALB/c mice aged 4 to 6 weeks were obtained from the Shanghai Laboratory Animal Research Center. The mice were housed at 25°C with free access to food and water. For *in vivo* sensitivity imaging, the mice were divided into groups with three mice per group. The primary probe **BIN-3** was administered at a dose of 1 µmol/kg. As shown in FIG. 53, a sensitive strain of *E. coli* (expressing mCherry fluorescent protein) was injected into the left thigh, and bla-positive *E. coli* was injected into the right thigh. One hour after bacterial injection, the primary probe **BIN-3** was administered. Fluorescence imaging was performed using an IVIS Lumina XRMS Series III imaging system. Images were acquired using the near-infrared channel (λ_{ex/em} = 660/710 nm) and the mCherry channel (λ_{ex/em} = 580/620 nm).

Experimental results: As shown in FIGs. 54 to 55, the primary probe **BIN-3** selectively labeled the bla-expressing bacteria in the right thigh, while fluorescence in the left thigh was detected only in the mCherry channel. After removal of the skin over the thighs, a characteristic blue-green coloration associated with hemicyanine dyes, was clearly visible in the right thigh (infected with bla-expressing *E. coli*), while no such coloration was observed in the left thigh (infected with mCherry-expressing *E. coli*)*.* These results indicate that the primary probe exhibits good selectivity for bla-expressing bacteria *in vivo.*

All references cited in the present disclosure are incorporated herein by reference to the same extent as if each reference is individually incorporated by reference. Furthermore, it should be understood that, after reading the above teachings of the present disclosure, those skilled in the art may make various changes or modifications to the present disclosure, and these equivalent forms also fall within the scope defined by the appended claims of the present disclosure.

## Claims

1. A compound or a salt thereof, wherein the compound is represented by formula I; 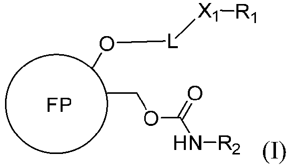 in formula I,
R₁ is a group capable of being specifically hydrolyzed by a biological macromolecular enzyme or a group capable of being specifically activated by a disease-related microenvironmental factor; and upon specific hydrolysis or activation, the bond between R₁ and X₁ is cleaved and R₁ is released;
X₁ is a linking bond, O, or NH;
L is a linking group capable of self-immolation after the release of R₁;
R₂ is C₁₋₆ alkyl or a water-solubilizing group;
the FP moiety is a fluorophore or a photosensitizing group.

2. The compound according to claim 1 or a salt thereof, wherein the compound satisfies one or more of the following conditions:
(1) the group capable of being specifically hydrolyzed by a biological macromolecular enzyme means that when R₁ is a group capable of being specifically hydrolyzed by a biological macromolecular enzyme, the biological macromolecular enzyme is capable of recognizing the group, and under the action of the biological macromolecular enzyme, X₁-R₁ is hydrolyzed, thereby cleaving the bond between X₁ and R₁;
(2) the biological macromolecular enzyme is alkaline phosphatase, γ-glutamyl transpeptidase, aminopeptidase, Fapα, Hepsin, or β-lactamase;
(3) the group capable of being specifically hydrolyzed by a biological macromolecular enzyme is: an enzyme-recognition group capable of being specifically hydrolyzed by alkaline phosphatase, an enzyme-recognition group capable of being specifically hydrolyzed by γ-glutamyl transpeptidase, an enzyme-recognition group capable of being specifically hydrolyzed by aminopeptidase, an enzyme-recognition group capable of being specifically hydrolyzed by Fapα, an enzyme-recognition group capable of being specifically hydrolyzed by Hepsin, or an enzyme-recognition group capable of being specifically hydrolyzed by β-lactamase;
(4) the group capable of being specifically activated by a disease-related microenvironmental factor means that when R₁ is a group capable of being specifically activated by a disease-related microenvironmental factor, the bond between X₁ and R₁ can be cleaved under the action of the disease-related microenvironmental factor;
(5) the disease-related microenvironmental factor is a β-lactamase-expressing drug-resistant bacterial infection or reactive oxygen species;
(6) the group capable of being specifically activated by a disease-related microenvironmental factor is a group capable of being specifically activated by a peroxide or a group capable of being specifically activated by a superoxide anion;
(7) the water-solubilizing group is -L₁-W₁-L₂-water-solubilizing moiety; wherein L₁ and L₂ are each independently absent or a divalent linking group, and W₁ is absent or a linking group formed via a click chemistry reaction;
preferably, the water-solubilizing group is 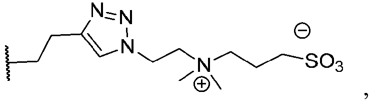 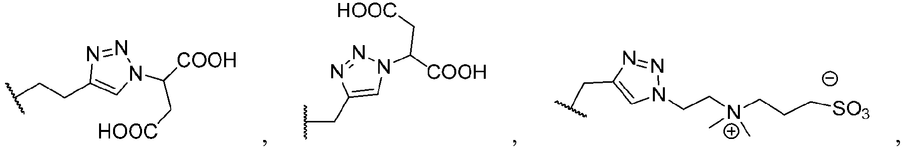 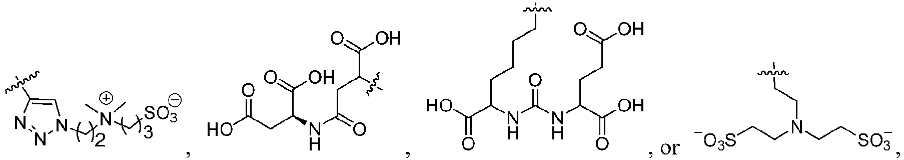 preferably 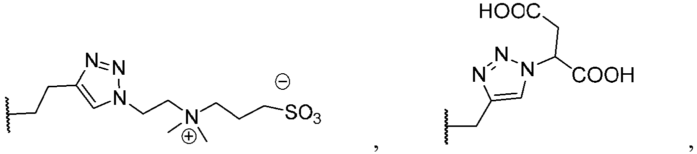 or 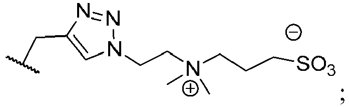
(8) the water-solubilizing group is a water-solubilizing group capable of being linked to a carbamate structure, such that upon activation of the compound, the carbamate structure is cleaved and the water-solubilizing group is released.

3. The compound according to claim 2 or a salt thereof, wherein the compound satisfies one or more of the following conditions:
(1) the biological macromolecular enzyme is alkaline phosphatase or β-lactamase;
(2) the enzyme-recognition group capable of being specifically hydrolyzed by alkaline phosphatase is 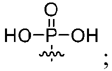
(3) the enzyme-recognition group capable of being specifically hydrolyzed by γ-glutamyl transpeptidase is 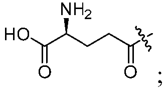
(4) the enzyme-recognition group capable of being specifically hydrolyzed by aminopeptidase is 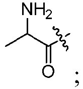
(5) the enzyme-recognition group capable of being specifically hydrolyzed by Fapα is 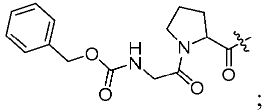
(6) the enzyme-recognition group capable of being specifically hydrolyzed by Hepsin is 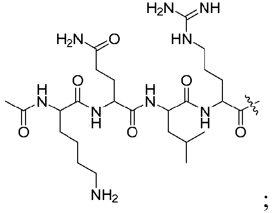
(7) the enzyme-recognition group capable of being specifically hydrolyzed by β-lactamase is 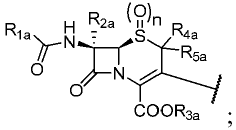 wherein R₁ₐ is selected from the group consisting of C₁₋₆ alkyl, phenyl, 5- or 6-membered heteroaryl, C₁₋₄ alkylene-phenyl, C₁₋₄ alkylene-(5- or 6-membered heteroaryl), and a water-solubilizing group, wherein the C₁₋₆ alkyl, phenyl, 5- or 6-membered heteroaryl, C₁₋₄ alkylene-phenyl, or C₁₋₄ alkylene-(5- or 6-membered heteroaryl) is optionally substituted by one or more halogens, C₁₋₆ alkyl, or C₁₋₆ haloalkyl;
R₂ₐ is selected from the group consisting of H and -OC₁₋₄ alkyl;
R₃ₐ is selected from the group consisting of H, C₁₋₆ alkyl, and a monovalent cation (e.g., Na⁺), wherein the C₁₋₆ alkyl is optionally substituted by one or more halogens, C₁₋₆ alkyl, or C₁₋₆ haloalkyl;
R₄ₐ and R₅ₐ are each independently selected from the group consisting of H and C₁₋₄ alkyl *(e.g.,* methyl or ethyl); alternatively, R₄ₐ and R₅ₐ, together with the carbon atom to which they are attached, form a C₃₋₄ cycloalkyl group;
n is 0, 1, or 2;
(8) the reactive oxygen species is preferably a peroxide or a superoxide anion;
(9) the group capable of being specifically activated by a peroxide is 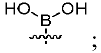
(10) the group capable of being specifically activated by a superoxide anion is 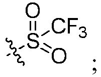
(11) L₁ and L₂ are each independently absent or a stable, non-cleavable divalent linking group under intracellular or *in vivo* conditions;
(12) W₁ is absent or 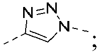
(13) the water-solubilizing moiety is selected from the group consisting of a group containing a cation-anion pair, a group containing one or more carboxyl groups, and a group containing one or more sulfonic acid groups.

4. The compound according to claim 3 or a salt thereof, wherein the compound satisfies one or more of the following conditions:
(1) R₁ₐ is a water-solubilizing group;
(2) R₂ₐ is selected from the group consisting of H and -OMe;
preferably, R₂ₐ is H;
(3) R₃ₐ is selected from the group consisting of H, C₁₋₆ alkyl, and a monovalent cation;
preferably, R₃ₐ is H;
(4) R₄ₐ and R₅ₐ are each independently selected from the group consisting of H and C₁₋₄ alkyl (e.g., methyl or ethyl);
preferably, both R₄ₐ and R₅ₐ are H;
(5) n is 0;
(6) L₁ and L₂ are each independently absent or C₁-C₄ alkylene;
(7) the group containing one or more carboxyl groups is a group containing 2 or 3 carboxyl groups, such as 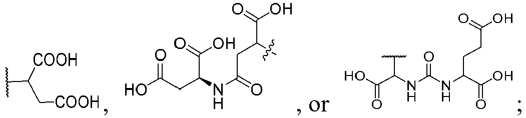
(8) the group containing one or more sulfonic acid groups is a group containing 2 or 3 sulfonic acid groups, such as 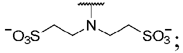
(9) the group containing a cation-anion pair is 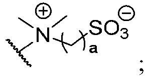 wherein a is 1, 2, 3, or 4, preferably 2 or 3.

5. The compound according to claim 1 or a salt thereof, wherein the compound satisfies one or more of the following conditions:
(1) R₁ is selected from the group consisting of: 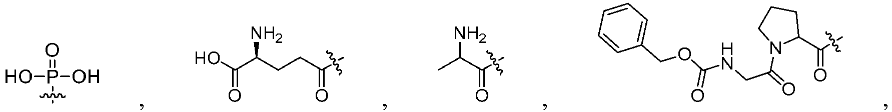 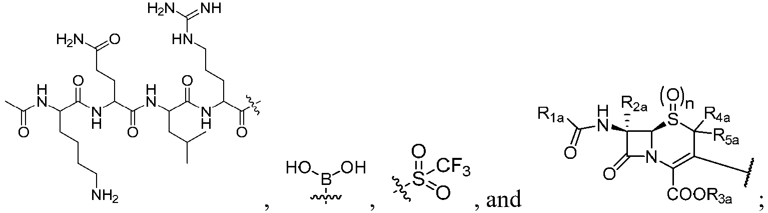 wherein R₁ₐ, R₂ₐ, R₃ₐ, R₄ₐ, R₅ₐ, and n are as defined in claim 3 or 4; (2) X₁ is a linking bond or O;
(3) L is selected from the group consisting of 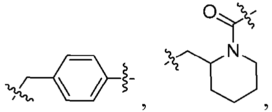 and C₁-C₄ alkylene; (4) R₂ is C₁₋₆ alkyl or a water-solubilizing group;
(4) the FP moiety has a structure represented by formula II-1 or II-2: 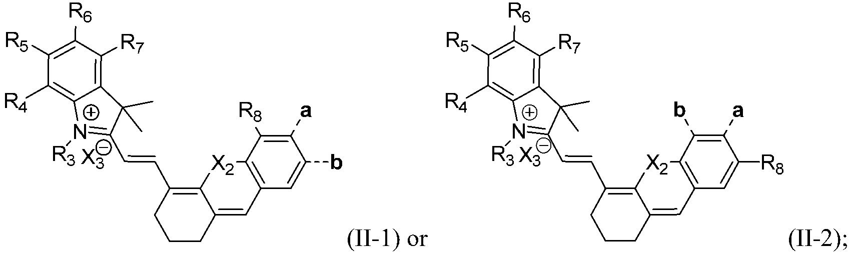 wherein
a represents the position of attachment to 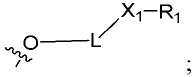
b represents the position of attachment to 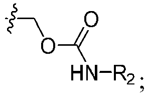
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 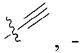 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br;
X₂ is O or S;
X₃ is I, Br, or Cl;
alternatively,
the FP moiety has a structure represented by formula III-1 or III-2: 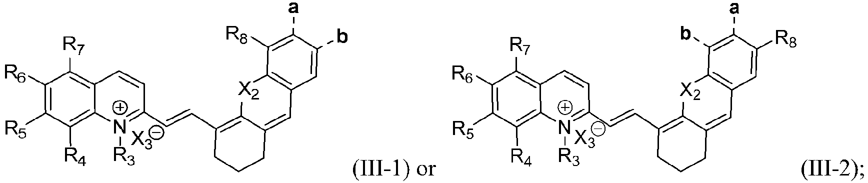
wherein
a represents the position of attachment to 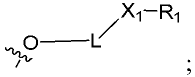
b represents the position of attachment to 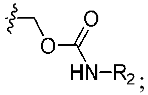
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 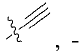 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br;
X₂ is O or S;
X₃ is I, Br, or Cl;
alternatively, the FP moiety has a structure represented by formula IV-1 or IV-2: 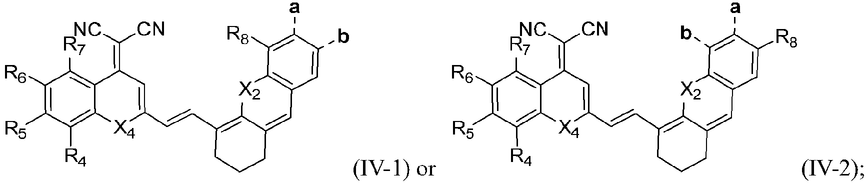
wherein
a represents the position of attachment to 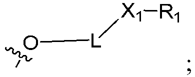
b represents the position of attachment to 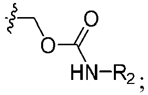
X₂ is O or S;
X₄ is O or NR₃;
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 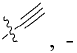 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br;
alternatively,
the FP moiety has a structure represented by formula V-1 or V-2: 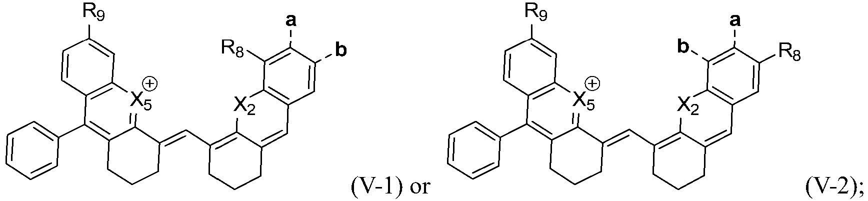
wherein
a represents the position of attachment to 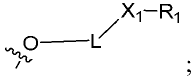
b represents the position of attachment to 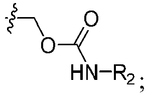
X₂ is O or S;
X₅ is O or S;
R₉ is NEt₂, NPh₂, or NCH₃Ph;
R₈ is H, F, Cl, or Br;
alternatively,
the FP moiety has a structure represented by formula VI-1 or VI-2: 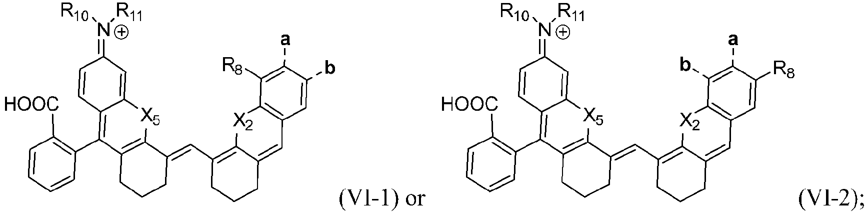
wherein
a represents the position of attachment to 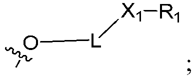
b represents the position of attachment to 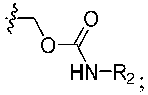
X₂ is O or S;
X₅ is O or S;
R₁₀ and R₁₁ are each independently C₁₋₆ alkyl *(e.g.,* Et) or Ph;
R₈ is H, F, Cl, or Br.

6. The compound according to claim 5 or a salt thereof, wherein the compound satisfies one or more of the following conditions:
(1) R₁ is 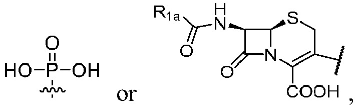 wherein R₁ₐ is a water-solubilizing group; preferably, R₁ is 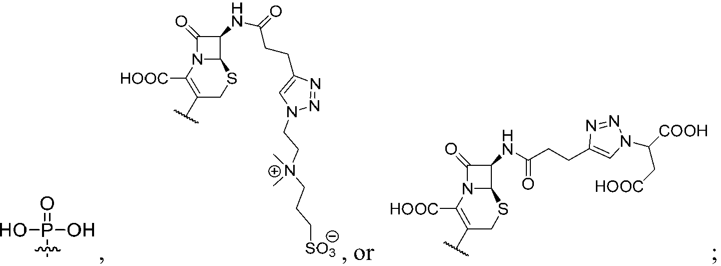
(2) X₁ is O;
(3) L is selected from the group consisting of 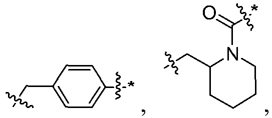 and C₁-C₄ alkylene; wherein * represents the position of attachment to X₁;
(4) R₂ is ethyl, 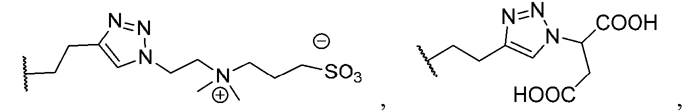 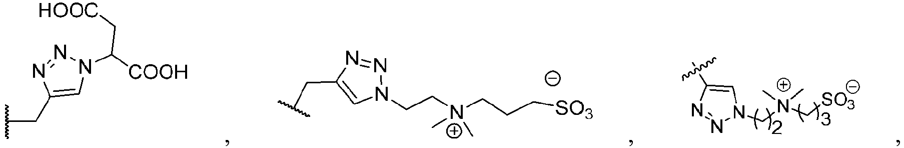 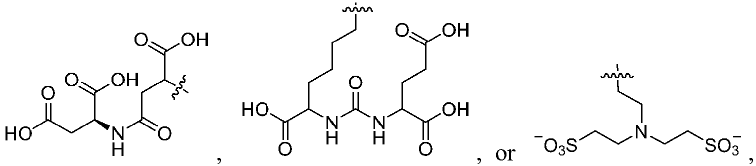 preferably ethyl, 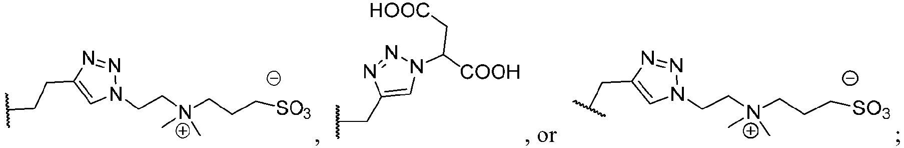
(5) the FP moiety has a structure represented by formula II-1 or II-2: 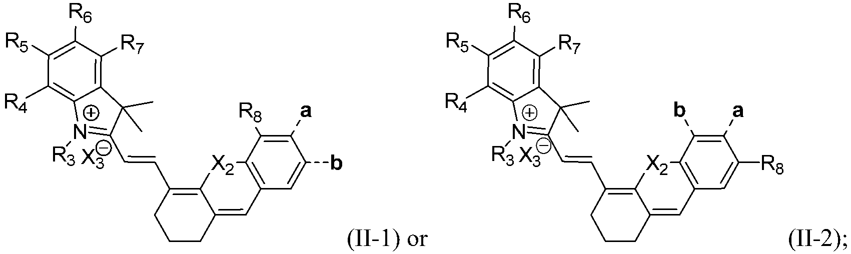 wherein
a represents the position of attachment to 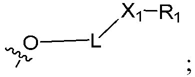
b represents the position of attachment to 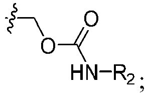
R₃ is -C₁₋₆ alkyl or -C₁₋₆ alkyl-R^{Fu}; wherein R^{Fu} is selected from the group consisting of 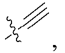 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl;
preferably, the FP moiety is

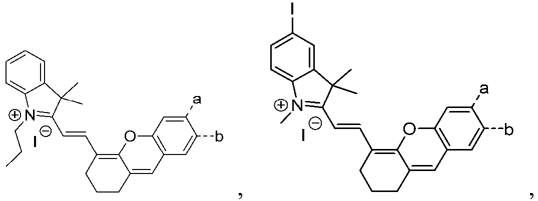

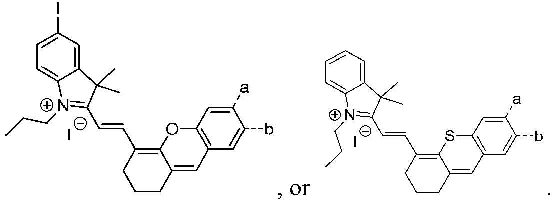

7. The compound according to claim 1 or a salt thereof, wherein X₁ is O or NH;
R₂ is a water-solubilizing group.

8. The compound according to claim 7 or a salt thereof, wherein the compound satisfies one or more of the following conditions:
(1) R₁ is selected from the group consisting of: 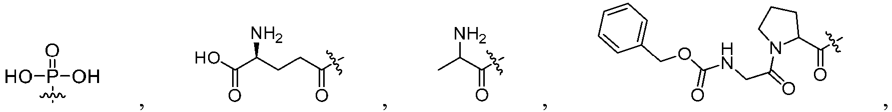 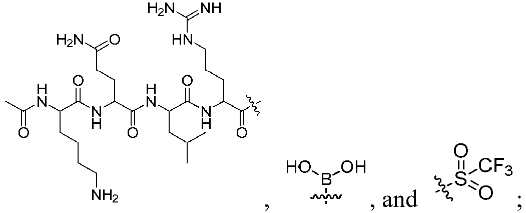
(2) L is selected from the group consisting of: 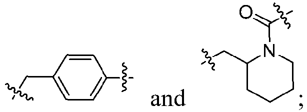
(3) R₂ is selected from the group consisting of: 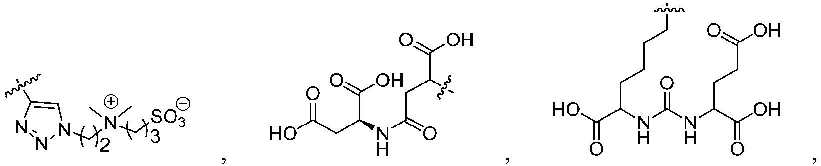 and 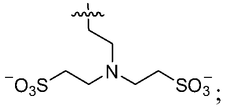
(4) the FP moiety has a structure represented by formula II-1 or II-2: 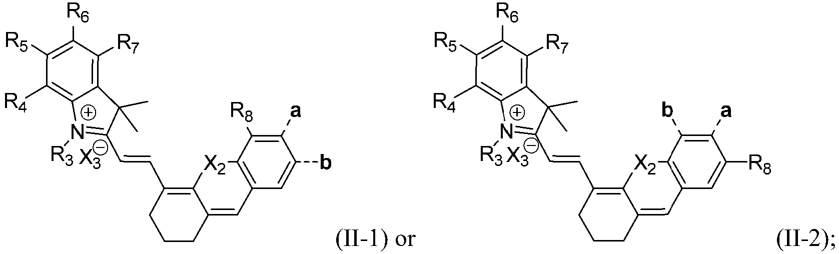 wherein
a represents the position of attachment to 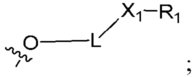
b represents the position of attachment to 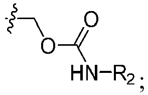
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 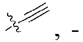 COOH, -N₃, -NH₂, and -N⁺(C₁₋₆ alkyl)₃);
R₄, R₅, R₆, and R₇ are each independently H or a heavy atom I or Br;
R₈ is H, F, Cl, or Br;
X₂ is O or S;
X₃ is I, Br, or Cl;
alternatively,
the FP moiety has a structure represented by formula III-1 or III-2: 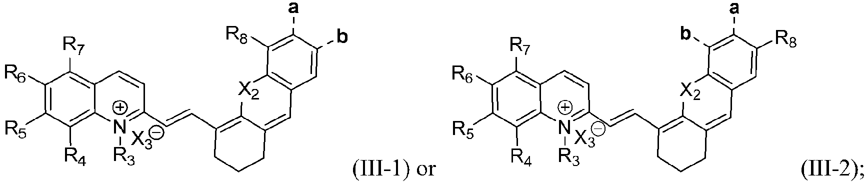
wherein
a represents the position of attachment to 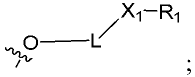
b represents the position of attachment to 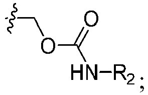
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 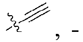 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br;
X₂ is O or S;
X₃ is I, Br, or Cl;
alternatively, the FP moiety has a structure represented by formula IV-1 or IV-2: 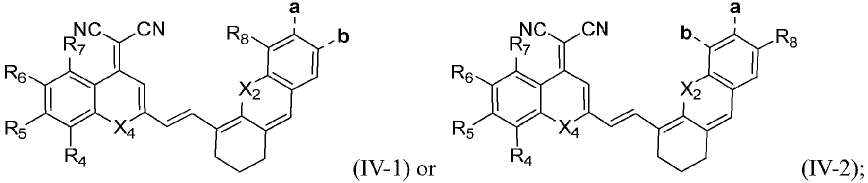
wherein
a represents the position of attachment to 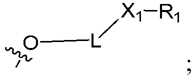
b represents the position of attachment to 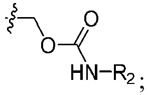
X₂ is O or S;
X₄ is O or NR₃;
R₃ is -C₁₋₆ alkyl or -C₁₋₆ alkyl-R^{Fu}; wherein R^{Fu} is a modifiable functional group or a positively charged group (preferably, R^{Fu} is selected from the group consisting of 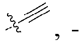 COOH, -N₃, -NH₂, and -N⁺(C₁₋₆ alkyl)₃);
R₄, R₅, R₆, and R₇ are each independently H or a heavy atom (e.g., I or Br);
R₈ is H, F, Cl, or Br;
alternatively,
the FP moiety has a structure represented by formula V-1 or V-2: 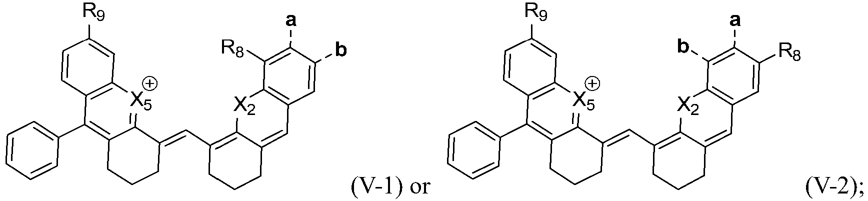
wherein
a represents the position of attachment to 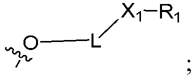
b represents the position of attachment to 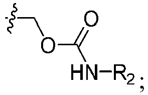
X₂ is O or S;
X₅ is O or S;
R₉ is NEt₂, NPh₂, or NCH₃Ph;
R₈ is H, F, Cl, or Br;
alternatively,
the FP moiety has a structure represented by formula VI-1 or VI-2: 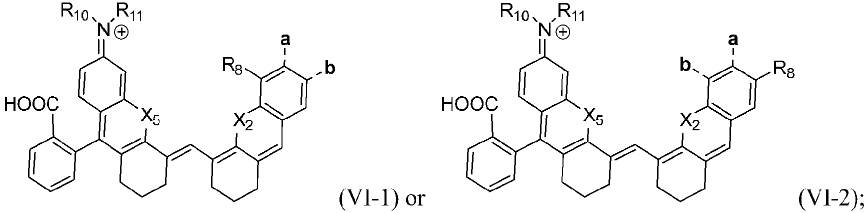
wherein
a represents the position of attachment to 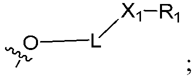
b represents the position of attachment to 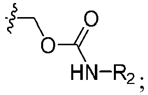
X₂ is O or S;
X₅ is O or S;
R₁₀ and R₁₁ are each independently C₁₋₆ alkyl *(e.g.,* Et) or Ph;
R₈ is H, F, Cl, or Br.

9. The compound according to claim 7 or a salt thereof, wherein the compound is selected from Table 1:
**Table 1**
| | |
|---|---|
| **ALPIN-6** | 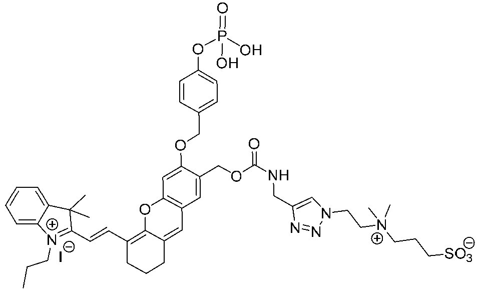 |
| **ALPIN-6I** | 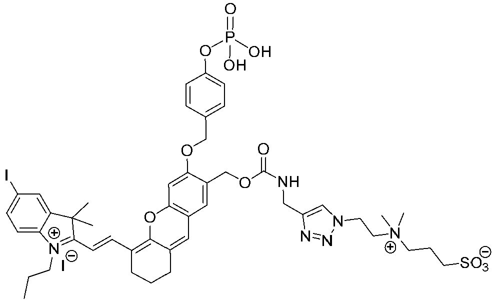 |
| **ALPIN-7** | 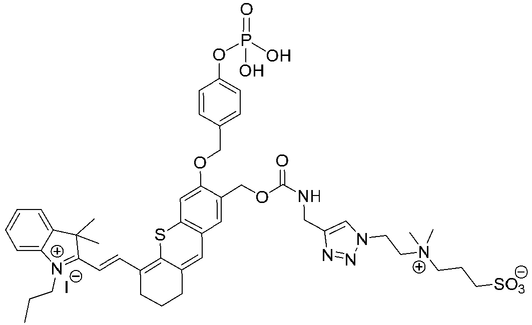 |

10. The compound according to any one of claims 1 to 7 or a salt thereof, wherein the compound is represented by formula I-A; 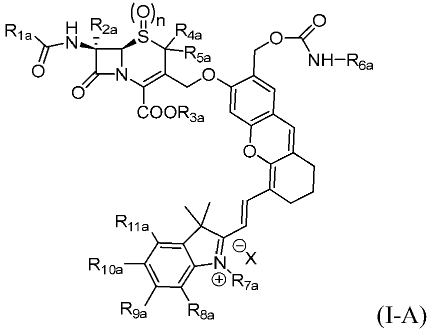 wherein
n is 0, 1, or 2;
R₁ₐ is selected from the group consisting of C₁₋₆ alkyl, phenyl, 5- or 6-membered heteroaryl, C₁₋₄ alkylene-phenyl, C₁₋₄ alkylene-(5- or 6-membered heteroaryl), and a water-solubilizing group, wherein the C₁₋₆ alkyl, phenyl, 5- or 6-membered heteroaryl, C₁₋₄ alkylene-phenyl, and C₁₋₄ alkylene-(5- or 6-membered heteroaryl) are optionally substituted by one or more halogens, C₁₋₆ alkyl, or C₁₋₆ haloalkyl;
R₂ₐ is selected from the group consisting of H and -OC₁₋₄ alkyl;
R₃ₐ is selected from the group consisting of H, C₁₋₆ alkyl, and a monovalent cation (e.g., Na⁺), wherein the C₁₋₆ alkyl is optionally substituted by one or more halogens, C₁₋₆ alkyl, or C₁₋₆ haloalkyl;
R₄ₐ and R₅ₐ are each independently selected from the group consisting of H and C₁₋₄ alkyl *(e.g.,* methyl or ethyl); alternatively, R₄ₐ and R₅ₐ, together with the carbon atom to which they are attached, form a C₃₋₄ cycloalkyl group;
R₆ₐ is selected from the group consisting of C₁₋₆ alkyl and a group containing a water-solubilizing moiety, wherein the C₁₋₆ alkyl is optionally substituted by one or more halogens, C₁₋₆ alkyl, or C₁₋₆ haloalkyl;
R₇ₐ is C₁₋₆ alkyl, wherein the C₁₋₆ alkyl is optionally substituted by one or more halogens, C₁₋₆ alkyl, or C₁₋₆ haloalkyl;
R₈ₐ, R₉ₐ, R₁₀ₐ, and R₁₁ₐ are each independently selected from the group consisting of H and a heavy atom;
x^{Θ} is a monovalent anion.

11. The compound according to claim 10 or a salt thereof, wherein the compound satisfies one or more of the following conditions:
(1) the group containing a water-solubilizing moiety is -L₁-W₁-L₂-water-solubilizing moiety; wherein L₁ and L₂ are each independently absent or a divalent linking group, and W₁ is absent or a linking group formed via a click chemistry reaction;
preferably, the water-solubilizing moiety is selected from the group consisting of a group containing a cation-anion pair, a group containing one or more carboxyl groups, and a group containing one or more sulfonic acid groups;
more preferably, the moiety containing a cation-anion pair comprises 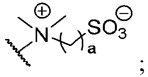 wherein a is 1, 2, 3, or 4;
the group containing one or more carboxyl groups comprises 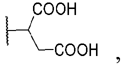 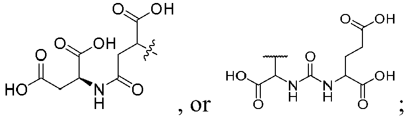
the group containing one or more sulfonic acid groups comprises 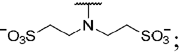
(2) at least one of R₁ₐ or R₆ₐ is a group containing a water-solubilizing moiety.

12. The compound according to claim 10 or a salt thereof, wherein the compound is selected from the group consisting of:

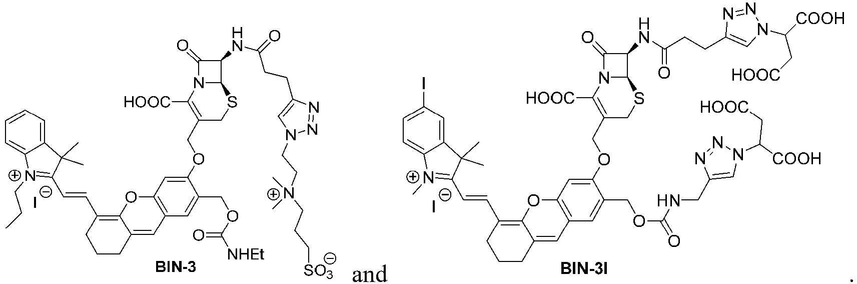

13. The compound according to any one of claims 1 to 12 or a salt thereof, wherein the compound satisfies one of the following schemes:
scheme 1:
the compound is represented by formula I; 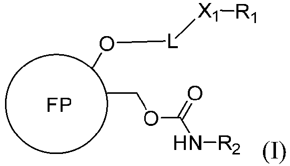
in formula I,
R₁ is a group capable of being specifically hydrolyzed by a biological macromolecular enzyme; and upon specific hydrolysis, the bond between R₁ and X₁ is cleaved and R₁ is released; the biological macromolecular enzyme is alkaline phosphatase or β-lactamase;
X₁ is a linking bond, O, or NH;
L is a linking group capable of self-immolation after the release of R₁;
R₂ is C₁₋₆ alkyl or a water-solubilizing group; the water-solubilizing group is -L₁-W₁-L₂-water-solubilizing moiety;
L₁ and L₂ are each independently C₁-C₄ alkylene;
W₁ is 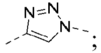
the water-solubilizing moiety is selected from the group consisting of 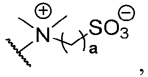 a group containing 2 or 3 carboxyl groups, and a group containing 2 or 3 sulfonic acid groups, wherein a is 1, 2, 3, or 4;
the FP moiety has a structure represented by formula II-1 or II-2: 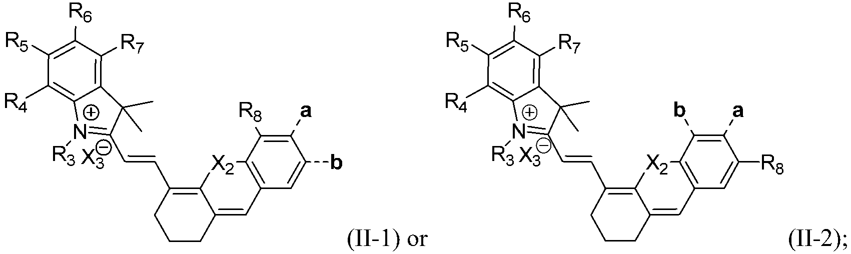
wherein
a represents the position of attachment to 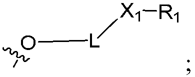
b represents the position of attachment to 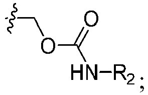
R₃ is -C₁₋₆ alkyl or -C₁₋₆ alkyl-R^{Fu}; wherein R^{Fu} is selected from the group consisting of 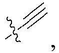 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl;
scheme 2:
the compound is represented by formula I; 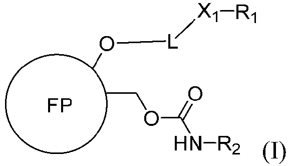
in formula I,
R₁ is 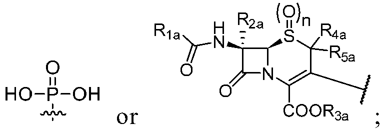 wherein R₁ₐ, R₂ₐ, R₃ₐ, R₄ₐ, R₅ₐ, and n are as defined in claim 3 or 4;
X₁ is a linking bond or O;
L is 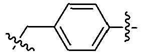 or C₁-C₄ alkylene;
R₂ is C₁₋₆ alkyl or a water-solubilizing group; the water-solubilizing group is -L₁-W₁-L₂-water-solubilizing moiety;
L₁ and L₂ are each independently C₁-C₄ alkylene;
W₁ is 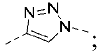
the water-solubilizing moiety is selected from the group consisting of 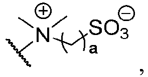 a group containing 2 or 3 carboxyl groups, and a group containing 2 or 3 sulfonic acid groups, wherein a is 1, 2, 3, or 4;
the FP moiety has a structure represented by formula II-1 or II-2: 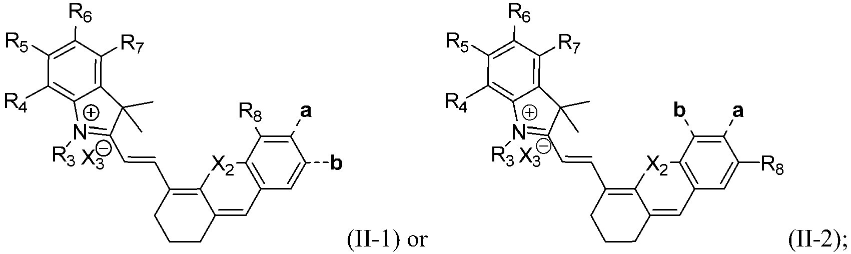
wherein
a represents the position of attachment to 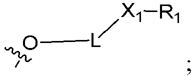
b represents the position of attachment to 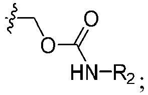
R₃ is -C₁₋₆ alkyl or -C₁₋₆ alkyl-R^{Fu}; wherein R^{Fu} is selected from the group consisting of 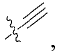 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl;
scheme 3:
the compound is represented by formula I; 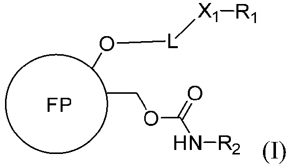
in formula I,
R₁ is a group capable of being specifically hydrolyzed by a biological macromolecular enzyme; and upon specific hydrolysis, the bond between R₁ and X₁ is cleaved and R₁ is released; the biological macromolecular enzyme is alkaline phosphatase;
X₁ is O or NH;
L is a linking group capable of self-immolation after the release of R₁;
R₂ is a water-solubilizing group; the water-solubilizing group is -L₁-W₁-L₂-water-solubilizing moiety;
L₁ and L₂ are each independently C₁-C₄ alkylene;
W₁ is 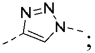
the water-solubilizing moiety is selected from the group consisting of 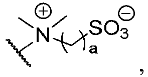 a group containing 2 or 3 carboxyl groups, and a group containing 2 or 3 sulfonic acid groups, wherein a is 1, 2, 3, or 4;
the FP moiety has a structure represented by formula II-1 or II-2: 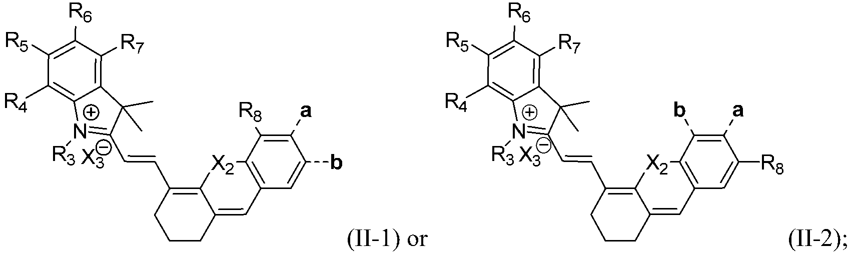
wherein
a represents the position of attachment to 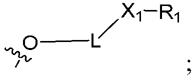
b represents the position of attachment to 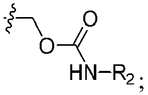
R₃ is -C₁₋₆ alkyl or -C₁₋₆ alkyl-R^{Fu}; wherein R^{Fu} is selected from the group consisting of 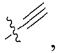 -COOH, -N₃, -NH₂, and -N⁺(C₁₋₆ alkyl)₃;
R₄, R₅, R₆, and R₇ are each independently H, Br, or I;
R₈ is H, F, Cl, or Br;
X₂ is O or S;
X₃ is I, Br, or Cl;
scheme 4:
the compound is represented by formula I; 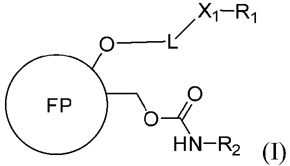
in formula I,
R₁ is 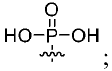
X₁ is O;
L is a linking group capable of self-immolation after the release of R₁;
R₂ is a water-solubilizing group; the water-solubilizing group is -L₁-W₁-L₂-water-solubilizing moiety;
L₁ and L₂ are each independently C₁-C₄ alkylene;
W₁ is 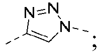
the water-solubilizing moiety is 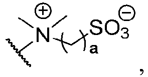 wherein a is 2 or 3;
the FP moiety has a structure represented by formula II-1: 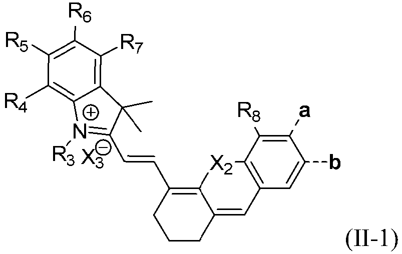
wherein
a represents the position of attachment to 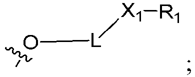
b represents the position of attachment to 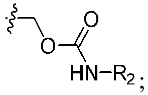
R₃ is -C₁₋₆ alkyl;
R₄, R₅, R₆, and R₇ are each independently H, Br, or I;
R₈ is H, F, Cl, or Br;
X₂ is O or S;
X₃ is I, Br, or Cl;
scheme 5:
the compound is represented by formula I; 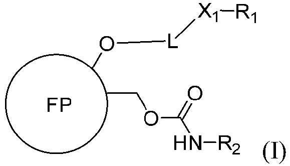
in formula I,
R₁ is 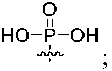
X₁ is O;
L is 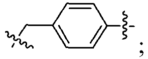
R₂ is a water-solubilizing group; the water-solubilizing group is -L₁-W₁-L₂-water-solubilizing moiety;
L₁ and L₂ are each independently C₁-C₄ alkylene;
W₁ is 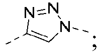
the water-solubilizing moiety is 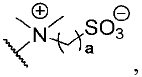 wherein a is 2 or 3;
the FP moiety is 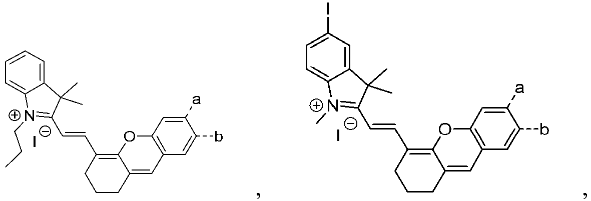 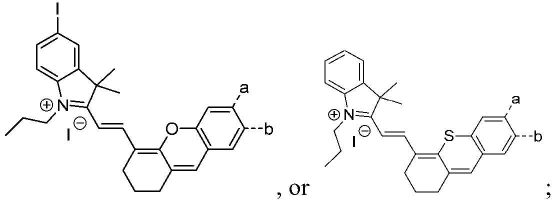
scheme 6:
the compound is represented by formula I-A; 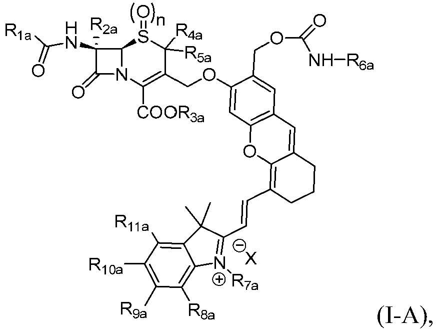
wherein
n is 0, 1, or 2;
R₁ₐ is selected from the group consisting of C₁₋₆ alkyl, phenyl, 5- or 6-membered heteroaryl, C₁₋₄ alkylene-phenyl, C₁₋₄ alkylene-(5- or 6-membered heteroaryl), and a water-solubilizing group;
R₂ₐ is selected from the group consisting of H and -OC₁₋₄ alkyl;
R₃ₐ is selected from the group consisting of H, C₁₋₆ alkyl, and a monovalent cation;
R₄ₐ and R₅ₐ are each independently selected from the group consisting of H and C₁₋₄ alkyl;
R₆ₐ is selected from the group consisting of C₁₋₆ alkyl and a water-solubilizing group;
R₇ₐ is C₁₋₆ alkyl;
R₈ₐ, R₉ₐ, R₁₀ₐ, and R₁₁ₐ are each independently selected from the group consisting of H, Br, and I;
x^{⊝} is a monovalent anion;
the water-solubilizing group is -L₁-W₁-L₂-water-solubilizing moiety;
L₁ and L₂ are each independently C₁-C₄ alkylene;
W₁ is 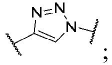
the water-solubilizing moiety is 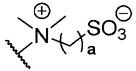 or a group containing 2 or 3 carboxyl groups, wherein a is 2 or 3;
scheme 7:
the compound is represented by formula I-A; 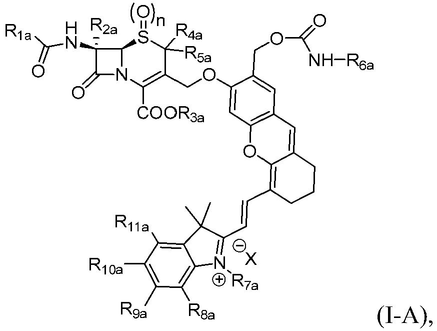
wherein
n is 0;
R₁ₐ is a water-solubilizing group;
R₂ₐ is selected from the group consisting of H and -OC₁₋₄ alkyl;
R₃ₐ is selected from the group consisting of H, C₁₋₆ alkyl, and a monovalent cation;
R₄ₐ and R₅ₐ are each independently selected from the group consisting of H and C₁₋₄ alkyl;
R₆ₐ is selected from the group consisting of C₁₋₆ alkyl and a water-solubilizing group;
R₇ₐ is C₁₋₆ alkyl;
R₈ₐ, R₉ₐ, R₁₀ₐ, and R₁₁ₐ are each independently selected from the group consisting of H, Br, and I;
x^{⊝} is a monovalent anion;
the water-solubilizing group is -L₁-W₁-L₂-water-solubilizing moiety;
L₁ and L₂ are each independently C₁-C₄ alkylene;
W₁ is 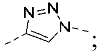 the water-solubilizing moiety is 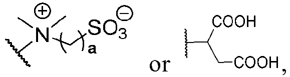 wherein a is 2 or 3;
scheme 8:
the compound is represented by formula I-A; 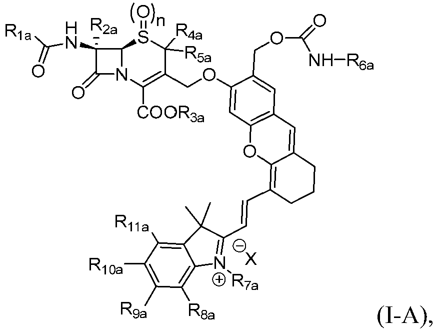
wherein
n is 0;
R₁ₐ is a water-solubilizing group;
R₂ₐ is H;
R₃ₐ is H;
both R₄ₐ and R₅ₐ are H;
R₆ₐ is selected from the group consisting of C₁₋₆ alkyl and a water-solubilizing group;
R₇ₐ is C₁₋₆ alkyl;
R₈ₐ, R₉ₐ, R₁₀ₐ, and R₁₁ₐ are each independently selected from the group consisting of H, Br, and I;
x^{⊝} is a monovalent anion;
the water-solubilizing group is -L₁-W₁-L₂-water-solubilizing moiety;
L₁ and L₂ are each independently C₁-C₄ alkylene;
W₁ is 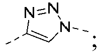
the water-solubilizing moiety is 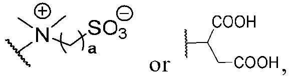 wherein a is 2 or 3.

14. A composition, wherein the composition comprises:
(1) the compound according to any one of claims 1 to 13 or a salt thereof; and
(2) a pharmaceutically or diagnostically acceptable carrier.

15. A detection kit, wherein the kit comprises the compound according to any one of claims 1 to 13 or a salt thereof, or the composition according to claim 14.

16. Use of the compound according to any one of claims 1 to 13 or a salt thereof, or the composition according to claim 14, wherein the use is selected from the group consisting of the following or any combination thereof:
(a) for the detection of an enzyme;
(b) for the detection of a disease-related microenvironmental factor *(e.g.,* ROS);
(c) for the manufacture of a detection reagent for the detection of an enzyme or a disease-related microenvironmental factor *(e.g.,* ROS);
(d) for the imaging of cells;
(e) for the manufacture of a detection reagent for imaging analysis of cells.

17. Use of the compound according to any one of claims 7 to 9 or a salt thereof, wherein the use is selected from the group consisting of the following or any combination thereof:
(f) for the imaging of tumor tissues or cells;
(g) for the manufacture of a detection reagent for tumor tissues or cells;
(h) for the photodynamic therapy of tumors;
(i) for the manufacture of a photosensitizing therapeutic agent for the photodynamic therapy of tumors.

18. Use of the compound according to any one of claims 10 to 12 or a salt thereof, wherein the use is selected from the group consisting of the following or any combination thereof:
(a) for the detection of β-lactamase;
(b) for the detection or imaging of drug-resistant bacteria;
(c) for the manufacture of a detection reagent for the detection or imaging of drug-resistant bacteria;
alternatively,
the use is selected from the group consisting of the following or any combination thereof:
(a) for the killing of drug-resistant bacteria;
(b) for the manufacture of a medicament for the killing of drug-resistant bacteria or for the treatment or prevention of diseases caused by infections with drug-resistant bacteria.

19. A method of imaging drug-resistant bacteria, wherein the method comprises the steps of: incubating the compound according to any one of claims 10 to 12 or a salt thereof with a subject, and obtaining an image of the drug-resistant bacteria using an instrument.

20. A detection kit, wherein the kit comprises the compound according to any one of claims 1 to 13 or a salt thereof, or the composition according to claim 14.
